(19) Europäisches Patentamt
European Patent Office
Office européen des brevets

(11) Veröffentlichungsnummer: **0 082 109**
**A2**

(12) # EUROPÄISCHE PATENTANMELDUNG

(21) Anmeldenummer: **82810440.6**

(22) Anmeldetag: **22.10.82**

(51) Int. Cl.³: **C 07 D 295/14,** C 07 D 307/83, A 61 K 31/535, C 07 D 307/60, C 07 D 405/04, C 07 D 295/08, C 07 D 295/10, C 07 D 207/327, C 07 D 209/14, A 61 K 7/44, A 61 K 31/165

(30) Priorität: **28.10.81 CH 6883/81**

(43) Veröffentlichungstag der Anmeldung: **22.06.83 Patentblatt 83/25**

(84) Benannte Vertragsstaaten: **AT BE CH DE FR IT LI LU NL SE**

(71) Anmelder: **CIBA-GEIGY AG, Patentabteilung Postfach, CH-4002 Basel (CH)**

(72) Erfinder: **Wenk, Paul, Dr., Quellenweg 7, CH-4123 Allschwil (CH)**
Erfinder: **Breitenstein, Werner, Dr., St. Galler-Ring 179, CH-4054 Basel (CH)**
Erfinder: **Baumann, Marcus, Dr., Rührbergerstrasse 6, CH-4058 Basel (CH)**

(54) **Substituierte 2-Hydroxy-phenylessigsäuren und Derivate davon.**

(57) Die Erfindung betrifft neue Phenol-Derivate, insbesondere solche der allgemeinen Formel

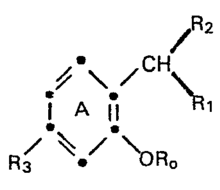

(I) ,

worin $R_0$ Wasserstoff oder einen Acylrest darstellt, $R_1$ Carboxy, verestertes Carboxy oder amidiertes bedeutet, $R_2$ für Wasserstoff oder einen aliphatischen Rest steht, $R_3$ eine durch zwei einwertige Kohlenwasserstoffreste oder durch einen zweiwertigen Kohlenwasserstoffrest disubstituierte Aminogruppe darstellt und der aromatische Ring A zusätzlich substituiert sein kann, und ihre Salze und Isomeren, Verfahren zur Herstellung von Verbindungen der Formel (I) und ihrer Salze und Isomeren, solche Verbindungen enthaltende pharmazeutische Präparate sowie deren Verwendung als Arzneimittelwirkstoffe und/oder zur Herstellung pharmazeutischer Präparate. Die Verbindungen der Formel (I) zeichnen sich durch ausgeprägte antiinflammatorische und analgetische Eigenschaften aus.

EP 0 082 109 A2

CIBA-GEIGY AG 4-13616/+

Basel (Schweiz)

Phenol-Derivate

Die Erfindung betrifft neue Phenol-Derivate, insbesondere solche der allgemeinen Formel

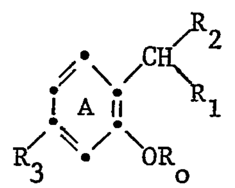

(I),

worin $R_o$ Wasserstoff oder einen Acylrest darstellt, $R_1$ Carboxy, verestertes Carboxy oder amidiertes Carboxy bedeutet, $R_2$ für Wasserstoff oder einen aliphatischen Rest steht, $R_3$ eine durch zwei einwertige Kohlenwasserstoffreste oder durch einen zweiwertigen Kohlenwasserstoffrest disubstituierte Aminogruppe darstellt und der aromatische Ring A zusätzlich substituiert sein kann, und ihre Salze und Isomeren, Verfahren zur Herstellung von Verbindungen der Formel (I) und ihrer Salze und Isomeren, solche Verbindungen enthaltende pharmazeutische Präparate sowie deren Verwendung als Arzneimittelwirkstoffe und/oder zur Herstellung pharmazeutischer Präparate.

Ein aliphatischer Rest $R_2$ ist insbesondere gesättigt und unsubstituiert und stellt in erster Linie einen Niederalkylrest dar.

Ein Acylrest ist beispielsweise ein Niederalkanoylrest oder ein Arylniederalkanoylrest, wie ein im Phenylteil ein- oder mehrfach substituierter bzw. unsubstituierter Phenylniederalkanoylrest, wobei Phenyl z.B. durch einen aliphatischen Rest, wie Niederalkyl, Niederalkenyl, gegebenenfalls verzweigtes, insbesondere zwei C-Atome überbrückendes, 3- bis 4-gliedriges Alkylen mit 3 bis 8 C-Atomen, Hydroxyniederalkyl oder Halogenniederalkyl, Niederalkoxy, Niederalkylthio, Niederalkansulfinyl, Niederalkansulfonyl, Hydroxy, Niederalkanoyloxy, Niederalkanoyl, Halogen und/oder Nitro ein- oder mehrfach substituiert oder unsubstituiert sein kann. Ein Arylniederalkanoylrest leitet sich in erster Linie von einer Phenylalkancarbonsäure der Formel (I) ab, wobei die Reste $R_2$ und $R_3$ sowie die Substituenten des Rings A die für die Verbindungen der Formel (I) angegebenen, vorzugsweise die gleichen, Bedeutungen haben und $R_o$ insbesondere Wasserstoff, ferner Niederalkanoyl bedeutet.

Verestertes Carboxy ist beispielsweise mit einem aliphatischen oder aromatischen Alkohol verestertes Carboxy. Als aliphatischer Alkohol kommt beispielsweise ein Niederalkanol bzw. ein z.B. durch Hydroxy, durch Niederalkoxy, durch Niederalkanoyloxy oder durch Aryl, wie substituiertes bzw. unsubstituiertes Phenyl, substituiertes Niederalkanol in Frage, wobei Phenyl z.B. durch einen aliphatischen Rest, wie Niederalkyl, Niederalkenyl, gegebenenfalls verzweigtes, insbesondere zwei C-Atome überbrückendes, 3- bis 4-gliedriges Alkylen mit 3 bis 8 C-Atomen, Hydroxyniederalkyl oder Halogenniederalkyl, Niederalkoxy, Niederalkylthio, Niederalkansulfinyl, Niederalkansulfonyl, Hydroxy, Niederalkanoyloxy, Niederalkanoyl, Halogen und/oder Nitro ein- oder mehrfach substituiert oder unsubstituiert sein kann.

Als aromatischer Alkohol kommt beispielsweise substituiertes bzw. unsubstituiertes Phenol in Betracht, wobei Phenol z.B. durch einen aliphatischen Rest, wie Niederalkyl, Niederalkenyl, gegebenenfalls verzweigtes, insbesondere zwei C-Atome überbrückendes, 3- bis 4-gliedriges Alkylen mit 3 bis 8 C-Atomen, Hydroxyniederalkyl oder Halogenniederalkyl, Niederalkoxy, Niederalkylthio, Niederalkansulfinyl, Niederalkansulfonyl, Hydroxy, Niederalkanoyloxy, Niederalkanoyl, Halogen und/oder Nitro ein- oder mehrfach substituiert oder unsubstituiert sein kann.

Entsprechend verestertes Carboxy ist z.B. Niederalkoxycarbonyl, Hydroxyniederalkoxycarbonyl, Niederalkoxyniederalkoxycarbonyl, Niederalkanoyloxyniederalkoxycarbonyl, Phenylniederalkoxycarbonyl oder Phenoxycarbonyl.

Amidiertes Carboxy weist als Aminogruppe beispielsweise eine freie, mono- oder disubstituierte Aminogruppe auf. Die monosubstituierte Aminogruppe ist beispielsweise durch Niederalkyl, durch im Phenylteil

substituiertes bzw. unsubstituiertes Phenylniederalkyl oder durch substituiertes bzw. unsubstituiertes Phenyl monosubstituiert. Disubstituiertes Amino ist beispielsweise durch Niederalkyl, durch im Phenylteil substituiertes bzw. unsubstituiertes Phenylniederalkyl, durch substituiertes bzw. unsubstituiertes Phenyl oder durch Niederalkylen bzw. Niederalkenyl oder auch durch Monoaza, N-alkyliertes Monoaza, durch Monooxa, Monothia unterbrochenes Niederalkylen bzw. Niederalkenylen disubstituiert, wobei Niederalkylen bzw. Niederalkenylen unverzweigt oder verzweigt und/oder ein oder zwei ortho-annelierte Benzosysteme aufweisen können. Phenyl ist z.B. durch einen aliphatischen Rest, wie Niederalkyl, Niederalkenyl, gegebenenfalls verzweigtes, insbesondere zwei C-Atome überbrückendes 3- bis 4-gliedriges Alkylen mit 3 bis 8 C-Atomen, Hydroxyniederalkyl oder Halogenniederalkyl, Niederalkoxy, Niederalkylthio, Niederalkansulfinyl, Niederalkansulfonyl, Hydroxy, Niederalkanoyloxy, Niederalkanoyl, Halogen und/oder Nitro ein- oder mehrfach substituiert oder unsubstituiert. Entsprechend amidiertes Carboxy ist z.B. Carbamoyl, N-Mono- oder N,N-Diniederalkylcarbamoyl, N-Mono- und N,N-Di-phenylniederalkylcarbamoyl, N-Mono- oder N,N-Diphenyl-carbamoyl, Niederalkylencarbamoyl bzw. durch Monoaza, N'-Niederalkylmonoaza, Monooxa oder Monothia unterbrochenes Niederalkylencarbamoyl, ferner N-Niederalkyl-N-phenylniederalkyl-, N-Phenylniederalkyl-N-phenyl-, Niederalkenylcarbamoyl.

Der aromatische Ring A kann zusätzlich durch einen aliphatischen Rest, wie Niederalkyl, Hydroxyniederalkyl, Halogenniederalkyl, Niederalkenyl oder gegebenenfalls verzweigtes, insbesondere zwei benachbarte C-Atome überbrückendes, 3- bis 4-gliedriges Alkylen, Niederalkoxy, Hydroxy, Niederalkylthio, Niederalkansulfinyl, Niederalkansulfonyl, Halogen, Niederalkanoyloxy, Niederalkanoyl und/oder Nitro ein- oder mehrfach substituiert oder, bis auf $R_3$, unsubstituiert sein.

Eine durch zwei einwertige Kohlenwasserstoffreste disubstituierte Aminogruppe weist als solche einwertige aliphatische Reste, wie Niederalkylreste auf, die unsubstituiert oder durch 3- bis 7-gliedriges Cycloalkyl oder durch Aryl, wie unsubstituiertes bzw. z.B. einen aliphatischen Rest, wie Niederalkyl, Niederalkenyl, Niederalkylen, Hydroxyniederalkyl, Halogenniederalkyl, Niederalkoxy, Niederalkylthio, Niederalkansulfinyl, Niederalkansulfonyl, Hydroxy, Halogen, Niederalkanoyloxy, Niederalkanoyl und/oder Nitro aufweisendes Phenyl, substituiert sein kann. Eine durch einen zweiwertigen Kohlenwasserstoffrest disubstituierte Aminogruppe weist als solchen einen zweiwertigen aliphatischen Rest auf, der auch durch Aza, N-Niederalkylaza, Oxa oder Thia unterbrochen sein kann, wie Niederalkylen, Niederalkenylen, jeweils durch Aza, N-Niederalkylaza, Oxa, Thia unterbrochenes Niederalkylen bzw. Niederalkenylen auf, wobei Niederalkylen bzw. Niederalkenylen jeweils auch verzweigt sein können. Ferner können derartige cyclische Amine $R_3$ auch ein oder zwei ortho-anellierte Benzosysteme aufweisen.

$R_3$ bedeutet vorzugsweise Di-niederalkyl-amino, Cycloalkylniederalkylniederalkyl-amino, Di-cycloalkylniederalkyl-amino, Niederalkyl-phenylniederalkyl-amino, Di-phenylniederalkyl-amino, ferner Phenylniederalkyl-cycloniederalkylniederalkyl-amino, sowie jeweils 5- bis 8-gliedriges Niederalkylenamino, Niederalkenylenamino, Aza-niederalkylenamino, N'-Niederalkylaza-niederalkylen-amino, Oxa-niederalkylen-amino, Thia-niederalkylen-amino, Aza-niederalkenylenamino, N'-Niederalkylaza-niederalkenylen-amino, Oxa-niederalkenylen-amino oder Thia-niederalkenylen-amino, wobei Niederalkylen- bzw. Niederalkenylen-amino auch verzweigt sein und entsprechend 4 bis 14, vorzugsweise 4 bis 7 C-Atome aufweisen kann.

Als Beispiele für derartige Reste $R_3$ seien genannt: Pyrrolidin-1-yl, 2- oder 3-Pyrrolin-1-yl, Pyrrol-1-yl, Piperidin-1-yl, Azepin-1-yl, Imidazolidin-1-yl, 2-, 3- oder 4-Imidazolin-1-yl, Oxazolidin-3-yl, 4-Oxazolin-3-yl, Thiazolidin-3-yl, 4-Thiazolin-3-yl, Piperazin-1-yl, Morpholin-4-yl, Thiomorpholin-4-yl, 3-Methyl-imidazolidin-1-yl, 4-Methylpiperazin-1-yl.

Ferner bedeutet $R_3$ Niederalkylen- bzw. Niederalkenylen-amino, welches ein oder zwei ortho-anellierte Benzosysteme aufweist, wie Indol-1-yl, Indolin-1-yl, Isoindol-2-yl, Isoindolin-2-yl, Carbazol-9-yl oder β-Carbolin-9-yl.

Vor- und nachstehend sind unter mit "nieder" bezeichneten organischen Resten und Verbindungen vorzugsweise solche zu verstehen, die bis und mit 7, vor allem bis und mit 4 Kohlenstoffatome enthalten.

Die im Rahmen des vorliegenden Textes verwendeten Allgemeindefinitionen haben in erster Linie die folgenden Bedeutungen:

Niederalkyl ist z.B. Methyl, Ethyl, n-Propyl, Isopropyl, n-Butyl, Isobutyl, sek.-Butyl, tert.-Butyl und umfasst ferner entsprechende Pentyl-, Hexyl- oder Heptylreste.

Hydroxyniederalkyl ist z.B. Hydroxymethyl, 2-Hydroxyethyl oder 2- oder 3-Hydroxypropyl.

Halogenniederalkyl ist z.B. Chlormethyl oder Trifluormethyl.

Niederalkenyl ist z.B. Vinyl, 1- bzw. 2-Propenyl, 1-, 2- oder 3-Butenyl oder Butadien-1,3-yl.

3- oder 4-gliedriges Alkylen ist z.B. geradkettig, wie Tri- oder Tetramethylen, oder verzweigt, wie 2,4-Butylen, 2,4-Pentylen oder 2-Methyl-1,3-propylen.

Niederalkoxy ist z.B. Methoxy, Ethoxy, n-Propyloxy, Isopropyloxy, n-Butyloxy, Isobutyloxy, sek.-Butyloxy, tert.-Butyloxy, ferner entsprechende Pentyloxy-, Hexyloxy- oder Heptyloxyreste.

Niederalkylthio ist z.B. Methyl-, Ethyl-, n-Propyl-, Isopropyl-, n-Butyl-, Isobutyl-, sek.-Butyl- oder tert.-Butylthio.

Niederalkansulfinyl bzw. -sulfonyl ist z.B. Methan-, Ethan-, n-Propan- oder Isopropan-sulfinyl bzw. -sulfonyl.

Halogen ist z.B. Halogen der Atomnummer bis und mit 35, wie Fluor, Chlor oder Brom, und umfasst ferner Iod.

Niederalkanoyloxy ist z.B. Acetyloxy, Propionyloxy, Butyryloxy, Isobutyryloxy, sek.-Butyryloxy oder tert.-Butyryloxy.

Niederalkanoyl ist z.B. Acetyl, Propionyl, Butyryl, Isobutyryl oder tert.-Butyryl.

3- bis 7-gliedriges Cycloalkyl ist z.B. Cyclopropyl, Cyclobutyl, Cyclopentyl, Cyclohexyl oder Cycloheptyl.

Niederalkylen ist z.B. geradkettig, wie 4- bis 7-gliedriges Niederalkylen, wie Tetra-, Penta- oder Hexamethylen, ferner Heptamethylen oder verzweigt, wie 2,3-Dimethyl- oder 2,3-Diethyl-1,4-butylen.

Durch Aza bzw. N-Niederalkylaza unterbrochenes Niederalkylen ist z.B. 4- bis 7-gliedriges Monoaza- bzw. N'-Niederalkylmonoaza-niederalkylen, wie 2-Aza-tetramethylen, 3-Aza-pentamethylen oder 3-Methylazapentamethylen.

Durch Oxa bzw. Thia unterbrochenes Niederalkylen ist z.B. Monooxa- bzw. Monothia-niederalkylen, wie 3-Oxa- bzw. 3-Thia-pentamethylen.

Niederalkenylen weist eine oder mehrere Doppelbindungen auf und ist z.B. 4- bis 7-gliedriges Niederalkenylen, wie But-2-en-1,4-ylen, Buta-1,3-dien-1,4-ylen, Pent-2-en-1,5-ylen oder Penta-1,3-dien-1,5-ylen, Penta-1,4-dien-1,5-ylen, Hex-3-en-2,5-ylen oder Hexa-2,4-dien-2,4-ylen.

Durch Aza bzw. N-Niederalkylaza unterbrochenes Niederalkenylen mit einer oder mehreren Doppelbindungen ist z.B. 2-Aza-buten-1-ylen, 2-

Aza-buten-2-ylen, 2-Aza-buten-3-ylen, 2-Methylaza-buten-3-ylen oder 2-Aza-butadien-1,3-ylen.

Salze von erfindungsgemässen Verbindungen der Formel (I) sind vorzugsweise pharmazeutisch verwendbare Salze, wie pharmazeutisch verwendbare Säureadditionssalze. Diese werden beispielsweise mit starken anorganischen Säuren, wie Mineralsäuren, z.B. Schwefelsäure, Phosphorsäure oder Halogenwasserstoffsäuren, mit starken organischen Carbonsäuren, wie Niederalkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder mit Sulfonsäuren, wie Niederalkan- oder gegebenenfalls substituierte Benzolsulfonsäure, z.B. Methan- oder p-Toluolsulfonsäure,gebildet. Liegt $R_1$ beispielsweise als Carboxy vor, können entsprechende Verbindungen Salze mit Basen bilden. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Mono-, Di- bzw. Triniederalkylamine, wie Hydroxyniederalkylamine, z.B. Mono-, Di- bzw. Trihydroxyniederalkylamine, Hydroxyniederalkyl-niederalkyl-amine oder wie Polyhydroxyniederalkylamine. Cyclische Amine sind z.B. Morpholin, Thiomorpholin, Piperidin oder Pyrrolidin. Als Mononiederalkylamine kommen beispielsweise Ethyl- oder tert.-Butylamin, als Diniederalkylamine beispielsweise Diethyl- oder Diisoporpylamin, als Triniederalkylamine beispielsweise Trimethyl- oder Triethylamin in Betracht. Entsprechende Hydroxyniederalkylamine sind z.B. Mono-, Di- bzw. Triethanolamine, und Hydroxyniederalkyl-niederalkylamine sind z.B. N,N-Dimethylamino- oder N,N-Diethylamino-ethanol, ferner Glucosamin als Polyhydroxyniederalkylamin.

Isomere Verbindungen der Formel (I) liegen insbesondere als Strukturisomere vor. Weisen beispielsweise Verbindungen der Formel (I) chirale

Kohlenstoffatome auf, können sie als Diastereomere, Diastereomerengemische, Racemate oder in Form eines reinen Enantiomeren vorliegen.

Die Verbindungen der Formel (I) weisen wertvolle pharmakologische Eigenschaften auf. Insbesondere besitzen sie eine ausgeprägte antiinflammatorische Wirkung, die sie z.B. durch Hemmung des durch Carrageenin erzeugter Pfotenoedems bei der Ratte ab einer Dosis von etwa 0,1 mg/kg p.o. analog der von Pasquale et al., Ag. and Actions, 5, 256 (1975), beschriebenen Methode sowie im Adjuvans-Arthritis-Modell an der Ratte ab einer Dosis von etwa 1,0 mg/kg p.o. analog L. Riesterer et al., Pharmacology, 2, 288 (1969), nachweisen lässt. Ausserdem hemmen Verbindungen der Formel (I) in vitro ab einer Konzentration von etwa 10 µmol/l die Prostaglandinsynthese aus Arachidonsäure analog der von H.L. White et al., Prostaglandins, 7, 123 (1974), beschriebenen Methode.

Weiterhin weisen die Verbindungen der Formel (I) eine deutliche antinociceptive Wirkungskomponente auf, die sich z.B. aus der von L.C. Hendershot et al., J. Pharmacol. exp. Therap., 125, 237 (1959) beschriebenen Reduktion des durch Phenyl-p-Benzochinon induzierten Writhing-Syndroms an der Maus ab einer Dosis von etwa 0,1 mg/kg p.o. ableiten lässt.

Ferner zeigen die Verbindungen der Formel (I) die Fähigkeit, aus dem Bereich des UV-Spektrums die auf der Epidermis Erythreme erzeugenden Strahlen (zwischen 290 und 320 nm) zu absorbieren, während die Substanzen für die bräunend wirkenden Strahlen von etwa 320 bis 400 nm durchlässig sind.

Infolgedessen lassen sich diese Verbindungen als Antiinflammatorika, (periphere) Analgetika und/oder Lichtschutzmittel, z.B. für kosmetische Zwecke, verwenden.

Die Erfindung betrifft beispielsweise Verbindungen der Formel (I), worin $R_o$ Wasserstoff, einen Niederalkanoyl- oder einen Arylniederalkanoylrest bedeutet, $R_1$ Carboxy, mit einem aliphatischen oder aromatischen Alkohol verestertes Carboxy, Carbamoyl oder mono- oder disubstituiertes Carbamoyl darstellt, $R_2$ einen gesättigten und unsubstituierten aliphatischen Rest bedeutet, $R_3$ eine durch zwei einwertige aliphatische Rest disubstituierte Aminogruppe oder eine durch einen zweiwertigen aliphatischen Rest disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_3$, unsubstituiert sein kann, und ihre Salze, insbesondere pharmazeutisch verwendbare Salze, und Isomeren.

Die Erfindung betrifft beispielsweise Verbindungen der Formel (I), worin $R_o$ Wasserstoff, Niederalkanoyl oder Phenylniederalkanoyl bedeutet, wobei der Phenylrest ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, oder Phenylniederalkanoyl bedeutet, welches sich von einer Phenylalkanniedercarbonsäure der Formel (I) ableitet, wobei die Reste $R_2$ und $R_3$ sowie die Substituenten des Rings A die nachstehend angegebenen Bedeutungen hat und $R_o$ für Wasserstoff oder Niederalkanoyl steht, $R_1$ Carboxy, Niederalkoxycarbonyl, Hydroxyniederalkoxycarbonyl, Niederalkanoyloxy-niederalkoxycarbonyl, Phenylniederalkoxycarbonyl, Phenoxycarbonyl, Carbamoyl, N-Mono-, N,N-Diniederalkylcarbamoyl, N-Mono- oder N,N-Di-phenylniederalkylcarbamoyl, N-Mono-, N,N,Diphenylcarbamoyl, N-Niederalkyl-N-phenylniederalkyl-carbamoyl, N-Niederalkyl-N-phenyl-carbamoyl, N-Phenylniederalkyl-N-phenyl-carbamoyl, Niederalkylen-carbamoyl, Niederalkenyl-carbamoyl, durch Monoaza, N'-Niederalkylmonoaza, Monooxa oder Monothia unterbrochenes Niederalkylencarbamoyl, Niederalkenylencarbamoyl darstellt, wobei Phenyl bzw. Phenoxy jeweils ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und wobei Niederalkylen bzw. Niederalkenylen unverzweigt oder verzweigt und/oder ein oder zwei ortho-anellierte Benzosysteme aufweisen können, $R_2$ Wasserstoff oder Niederalkyl bedeutet und $R_3$ einerseits N,N-Di-niederalkyl-amino, N-Cycloniederalkyl-N-niederalkyl-amino, N-Niederalkyl-N-phenylniederalkyl-amino, N,N-Di-cycloniederalkylniederalkyl-amino, N-Cycloniederalkylniederalkyl-N-phenylniederalkyl-amino oder N,N-Di-phenylniederalkyl-amino bedeutet oder $R_3$ andererseits jeweils 5- bis 8-gliedriges Niederalkylenamino, Niederalkenylenamino, durch Monoaza, N'-Niederalkylmonoaza, Monooxa oder Monothia unterbrochenes Niederalkylenamino, durch Monoaza, N'-Niederalkylmonoaza, Monooxa oder Monothia unterbrochenes Niederalkenylenamino oder ein oder zwei ortho-anellierte Benzosysteme aufweisendes Niederalkylen- bzw. Niederalkenylen-amino darstellt, wobei Niederalkylen bzw. Niederalkenylen unverzweigt oder verzweigt sein und 4 bis 14, insbesondere 4 bis 7, C-Atome aufweisen kann und/oder ein oder zwei ortho-anellierte Benzosysteme aufweisen kann und Phenyl oder Benzo jeweils ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und der aromatische Ring A ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder, bis auf $R_3$, unsubstituiert sein kann, und ihre Salze, insbesondere pharmazeutisch verwendbare Salze, und Isomeren.

Die Erfindung betrifft beispielsweise Verbindungen der Formel (I), worin $R_o$ Wasserstoff, Niederalkanoyloxy oder im Phenylteil unsubstituiertes bzw. ein- oder mehrfach durch Niederalkyl, Niederalkoxy, Hydroxy, Halogen, Niederalkanoyloxy und/oder Trifluormethyl substituiertes Phenylniederalkanoyloxy bedeutet, $R_1$ Carboxy, mit einem Niederalkanol, einem durch Hydroxy, Niederalkoxy, Niederalkanoyloxy oder im Phenylteil unsubstituiertes oder Niederalkyl, Niederalkoxy, Hydroxy, Halogen, Niederalkanoyloxy und/oder Trifluormethyl enthaltendes Phenyl substituiertes Niederalkanol, mit einem unsubstituierten oder Niederalkyl, Niederalkoxy, Hydroxy, Halogen, Niederalkanoyloxy und/oder Trifluormethyl enthaltenden Phenol verestertes Carboxy, Carbamoyl, durch Niederalkyl, im Phenylteil unsubstituiertes bzw. Niederalkyl, Niederalkoxy, Hydroxy, Halogen, Niederalkanoyloxy und/oder Trifluormethyl aufweisendes Phenylniederalkyl monosubstituiertes Carbamoyl, durch Niederalkyl, durch im Phenylteil unsubstituiertes bzw. Niederalkyl, Niederalkoxy, Hydroxy, Halogen, Niederalkanoyloxy und/oder Trifluormethyl aufweisendes Phenylniederalkyl, durch Niederalkylen oder durch Monoaza, N-alkyliertes Monoaza, Monooxa bzw. Monothia unterbrochenes Niederalkylen disubstituiertes Carbamoyl darstellt, $R_2$ Wasserstoff oder Niederalkyl bedeutet, $R_3$ eine durch Niederalkyl, durch im Cycloalkylteil 3- bis 7-gliedriges Cycloalkyl-niederalkyl, durch im Phenylteil unsubstituiertes bzw. Niederalkyl, Niederalkoxy, Hydroxy, Halogen, Niederalkanoyloxy und/oder Trifluormethyl enthaltendes Phenylniederalkyl, durch Niederalkylen, durch Niederalkenylen, durch Aza, N-Niederalkylaza, Oxa oder Thia unterbrochenes Niederalkylen oder durch Aza oder N-Niederalkylaza unterbrochenes Niederalkenylen disubstituierte Aminogruppe darstellt und der aromatische Ring A zusätzlich durch Niederalkyl, Niederalkoxy, Hydroxy, Halogen Niederalkanoyloxy, 3- oder 4-gliedriges Alkylen und/oder Trifluormethyl substituiert sein kann, und ihre Salze, insbesondere pharmazeutisch verwendbare Salze, und Isomeren.

Die Erfindung betrifft insbesondere Verbindungen der Formel

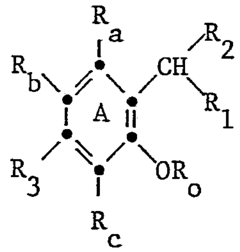

(Ia),

worin $R_o$ Wasserstoff oder Niederalkanoyl, wie Acetyl, bedeutet, $R_1$ Carboxy, Niederalkoxycarbonyl, wie Methoxycarbonyl, Niederalkylencarbamoyl, wie Pyrrolidinocarbonyl, oder Oxa-niederalkylen-carbamoyl, wie 3-Oxapentamethylen-carbamoyl, darstellt, $R_2$ Wasserstoff oder Niederalkyl, wie Methyl, bedeutet, $R_3$ Di-niederalkyl-amino, wie Dimethylamino, Di-cycloalkylniederalkylamino, wie Di-cyclopropylmethylamino, Di-phenylniederalkyl-amino, wie Dibenzylamino, jeweils 5- bis 8-gliedriges Niederalkylenamino, wie Pyrrolidin-1-yl, Niederalkenylenamino, wie Pyrrol-1-yl, Monoaza-niederalkylen-amino, wie Piperazin-1-yl, N'-Niederalkylmonoaza-niederalkylenamino, wie 4-Methyl-piperazin-1-yl, Monooxaniederalkylen-amino, wie Morpholin-4-yl, Monothia-niederalkylenamino, wie Thiomorpholin-4-yl, Monoaza-niederalkenylenamino, wie Imidazol-1-yl, oder N'-Niederalkylmonoaza-niederalkenylenamino, wie 3-Methyl-imidazol-1-yl, darstellt und $R_a$, $R_b$, $R_c$ unabhängig voneinander Wasserstoff, Niederalkyl, wie Methyl, Niederalkoxy, wie Methoxy, Hydroxy, Halogen, wie Chlor, Niederalkanoyloxy, wie Acetyloxy, 3- bzw. 4-gliedriges Alkylen, wie Tetramethylen, oder Trifluormethyl bedeuten, und ihre Salze, insbesondere pharmazeutisch verwendbare Salze, und Isomeren.

Die Erfindung betrifft insbesondere Verbindungen der Formel (Ia), worin $R_o$ Wasserstoff oder Niederalkanoyl, wie Acetyl, oder Phenylniederalkanoyl, welches sich von einer Phenylniederalkancarbonsäure der Formel (I) ableitet, wobei die Reste $R_2$, $R_3$, $R_a$, $R_b$ und $R_c$ die nachstehend angegebenen Bedeutungen haben und $R_o$ Wasserstoff ist, bedeutet, $R_1$ Carboxy, Niederalkoxycarbonyl, wie Methoxycarbonyl, Niederalkanoyloxyniederalkoxycarbonyl, wie Pivaloyloxymethoxycarbonyl, Carbamoyl,

N,N-Di-phenylniederalkyl-carbamoyl, wie N,N-Dibenzylcarbamoyl, Niederalkylencarbamoyl, wie Pyrrolidino-carbonyl, oder durch Monooxa unterbrochenes Niederalkylencarbamoyl, wie 4-Morpholino-carbonyl, darstellt, $R_2$ Wasserstoff oder Niederalkyl, wie Methyl, bedeutet, $R_3$ einerseits N,N-Di-phenylniederalkyl-amino, wie Dibenzylamino, darstellt oder $R_3$ andererseits jeweils 5- bis 8-gliedriges Niederalkylenamino, wie 1-Piperidino, Niederalkenylenamino, wie 1-Pyrrolyl, durch Monooxa unterbrochenes Niederalkylenamino, wie 4-Morpholino, oder ein ortho-anelliertes Benzosystem aufweisendes Niederalkylenamino bzw. Niederalkenylenamino, Indolin-1-yl oder Indol-1-yl bedeutet, und/oder $R_a$, $R_b$ und $R_c$ unabhängig voneinander Wasserstoff, Niederalkyl, wie Methyl, oder Halogen, wie Chlor oder Brom, darstellen, und ihre Salze, insbesondere pharmazeutisch verwendbare Salze, und Isomeren.

Die Erfindung betrifft vor allem Verbindungen der Formel (Ia), worin $R_o$ Wasserstoff oder Niederalkanoyl, insbesondere mit bis und mit 5 C-Atomen, wie Acetyl, bedeutet, $R_1$ Carboxy, Niederalkoxycarbonyl, insbesondere mit bis und mit 5 C-Atomen, wie Methoxycarbonyl, 5- bis 8-gliedriges Niederalkylencarbamoyl, wie Pyrrolidinocarbonyl, 5- bis 8-gliedriges Monooxa-niederalkylencarbamoyl, wie 3-Oxa-pentamethylen-carbamoyl, darstellt, $R_2$ Wasserstoff oder Niederalkyl, insbesondere mit bis und mit 4 C-Atomen, wie Methyl, darstellt, $R_3$ Di-niederalkyl-amino, insbesondere mit bis und mit 4 C-Atomen im Alkylteil, wie N,N-Dimethylamino, 5- bis 8-gliedriges Niederalkylenamino, wie Pyrrolidin-1-yl, 5- bis 8-gliedriges Niederalkenylenamino, wie Pyrrol-1-yl, oder 5- bis 8-gliedriges Monooxa-niederalkylenamino, wie Morpholin-4-yl, bedeutet und $R_a$ sowie $R_c$ Wasserstoff bedeuten und $R_b$ Halogen, insbesondere bis und mit Atomnummer 35, wie Chlor, bedeutet und ihre Salze, insbesondere pharmazeutisch verwendbare Salze und Isomeren.

Die Erfindung betrifft in erster Linie Verbindungen der Formel (Ia), worin $R_o$ Wasserstoff oder Niederalkanoyl mit bis und mit 5 C-Atomen, wie Acetyl, bedeutet, $R_1$ Carboxy oder Niederalkoxycarbonyl mit

bis und mit 5 C-Atomen, wie Methoxycarbonyl, darstellt, $R_2$ Niederalkyl mit bis und mit 4 C-Atomen, wie Methyl, bedeutet, $R_3$ 5- bis 7-gliedriges Niederalkylenamino, wie Pyrrolidin-1-yl, Morpholin-4-yl oder Pyrrol-1-yl darstellt, $R_a$ und $R_c$ jeweils Wasserstoff bedeuten und $R_b$ Niederalkyl mit bis und mit 4 C-Atomen, wie Methyl, oder Halogen bis und mit Atomnummer 35, wie Chlor, darstellt, und ihre Salze, insbesondere pharmazeutisch verwendbare Salze, und Isomeren.

Die Erfindung betrifft in erster Linie Verbindungen der Formel (Ia), worin $R_o$ Niederalkanoyl mit bis und mit 5 C-Atomen, wie Acetyl, darstellt, $R_1$ Niederalkoxycarbonyl mit bis und mit 5 C-Atomen, wie Methoxycarbonyl, bedeutet, $R_2$ Niederalkyl mit bis und mit 4 C-Atomen, wie Methyl, darstellt, $R_3$ Morpholin-4-yl oder Pyrrol-1-yl ist, $R_a$ und $R_c$ Wasserstoff bedeuten und $R_b$ Halogen bis und mit Atomnummer 35, wie Chlor, oder Niederalkyl mit bis und mit 4 C-Atomen, wie Methyl, darstellt, und ihre Salze, insbesondere pharmazeutisch verwendbare Salze, und Isomeren.

Die Erfindung betrifft insbesondere die in den Beispielen genannten neuen Verbindungen, ihre Salze, vor allem pharmazeutisch verwendbare Salze, und Isomeren, ferner die in den Beispielen beschriebenen Verfahren zu ihrer Herstellung.

Die Verbindungen der vorliegenden Erfindung werden nach an sich bekannter Weise hergestellt, beispielsweise indem man Verbindungen der Formel

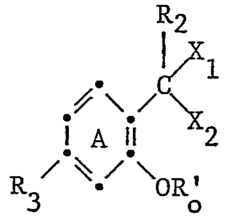

(II),

worin $X_1$ Wasserstoff ist, $X_2$ von $R_1$ verschiedenes, funktionell abgewandeltes Carboxy bedeutet und $R'_o$ die Bedeutung von $R_o$ hat oder worin $X_1$ Wasserstoff ist und $X_2$ gemeinsam mit $R'_o$ die Gruppe >C=O bilden

oder worin $X_1$ gemeinsam mit $X_2$ die Grupe =C=O oder die Gruppe =$C(Hal)_2$ bilden und Hal jeweils für Halogen, steht und $R'_o$ die Bedeutung von $R_o$ hat, oder deren Salze mit Solvolysemitteln behandelt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz übergeführt und/oder gewünschtenfalls ein verfahrensgemäss erhältliches Isomerengemisch in seine Komponenten auftrennt.

Von $R_1$ verschiedenes funktionell abgewandeltes Carboxy $X_2$ ist beispielsweise Cyano, anhydridisiertes Carboxy, gegebenenfalls substituiertes Amidino, gegebenenfalls verestertes Thiocarboxy, gegebenenfalls verestertes Dithiocarboxy, gegebenenfalls substituiertes Thiocarbamoyl, gegebenenfalls verestertes oder anhydridisiertes Carboximidoyl, von verestertem oder amidiertem Carboxy $R_1$ verschiedenes verestertes oder amidiertes Carboxy, durch Hydroxy oder Amino substituiertes Carbamoyl, Trialkoxy- oder Trihalogenmethyl.

Anhydridisiertes Carboxy ist beispielsweise mit einer Mineralsäure, wie Halogenwasserstoffsäure, oder mit einer Carbonsäure, wie einer gegebenenfalls substituierten Niederalkan- bzw. Benzoesäure oder einem Kohlensäurehalogenid-niederalkylhalbester, anhydridisiertes Carboxy. Als Beispiele seien Halogencarbonyl, wie Chlorcarbonyl, Niederalkanoyloxycarbonyl, wie Acetyloxycarbonyl, oder Niederalkoxycarbonyloxycarbonyl, wie Ethoxycarbonyloxycarbonyl, genannt.

Gegebenenfalls substituiertes Amidino ist beispielsweise mit einem aliphatischen, z.B. Niederalkyl-Rest, substituiertes Amidino, wie Amidino oder Niederalkylamidino, z.B. Ethylamidino.

Gegebenenfalls verestertes Thiocarboxy oder Dithiocarboxy weist beispielsweise die im Zusammenhang mit verestertem Carboxy genannte Alkohol- bzw. Hydroxykomponente auf. Herausgegriffen seien als Beispiele Niederalkylthio-carbonyl, wie Ethylthio-carbonyl, Niederalkoxythiocarbonyl, wie Ethoxy-thiocarbonyl, Niederalkylthio-thiocarbonyl, wie Ethylthio-thiocarbonyl, das jeweilige Thiocarboxy sowie Dithiocarboxy.

Gegebenenfalls substituiertes Thiocarbamoyl kann z.B. die unter amidiertem Carboxy genannten Substituenten aufweisen. Als Beispiel seien genannt N-Mono- oder N,N-Diniederalkyl-thiocarbamoyl, wie Methyl- oder Diethyl-thiocarbamoyl, sowie Thiocarbamoyl, wie 4-Thiomorpholinyl- oder 4-Morpholinyl-thiocarbonyl.

Unter Alkoxy- bzw. Halogen-carbimidoyl ist beispielsweise Niederalkoxy-, wie Ethoxy-, bzw. Chlor-carbimidoyl zu verstehen.

Trihalogen- bzw. Trialkoxymethyl ist z.B. Trichlormethyl bzw. Triniederalkoxy-, wie Trimethoxymethyl.

Solvolysemittel sind beispielsweise Wasser, der gewünschten veresterten Carboxygruppe entsprechende Alkohole, Ammoniak oder der gewünschten amidierten Carboxygruppe entsprechende Amine.

Die Behandlung mit einem entsprechenden Solvolysemittel wird gegebenenfalls in Gegenwart einer Säure oder Base, gegebenenfalls unter Kühlen oder Erwärmen, z.B. zwischen -20° und 300°C, und erforderlichenfalls in einem inerten Lösungs- oder Verdünnungsmittel durchgeführt. Neben den Solvolysemitteln können als Lösungsmittel z.B. auch Ether, wie Dioxan oder Tetrahydrofuran, Amide, wie Dimethylformamid, oder deren Gemische als Lösungsmittel verwendet werden.

Als Säuren kommen beispielsweise anorganische oder organische Protonsäuren, wie Mineralsäuren, z.B. Schwefel- oder Halogenwasserstoff-, beispielsweise Chlorwasserstoffsäure, wie Sulfonsäuren, z.B. Niederalkan- oder gegebenenfalls substituierte Benzolsulfonsäure, beispielsweise Methan- oder p-Toluolsulfonsäure, oder wie Carbonsäuren, z.B.

Niederalkancarbonsäuren, beispielsweise Essigsäure, in Frage, während als Basen beispielsweise Alkalimetallhydroxide, z.B. Natrium- oder Kaliumhydroxid, verwendet werden können.

Verbindungen der Formel (II), worin $X_1$ Wasserstoff ist, $X_2$ von $R_1$ verschiedenes funktionell abgewandeltes Carboxy bedeutet und $R'_o$ die Bedeutung von $R_o$ hat und worin $X_1$ Wasserstoff ist und $X_2$ gemeinsam mit $R'_o$ die Gruppe $>C=O$ bilden, werden beispielsweise solvolytisch in entsprechende Verbindungen der Formel (I) übergeführt. Dabei wird beispielsweise die Cyanogruppe, gegebenenfalls substituiertes Amidino, anhydridisiertes Carboxy, gegebenenfalls verestertes Thiocarboxy, gegebenenfalls verestertes Dithiocarboxy, gegebenenfalls substituiertes Thiocarbamoyl, gegebenenfalls verestertes oder anhydridisiertes Carboximidoyl, von verestertem oder amidiertem Carboxy $R_1$ verschiedenes verestertes Carboxy, durch Hydroxy oder Amino substituiertes Carbamoyl, Triniederalkoxymethyl, Niederalkoxyhalogenmethyl oder Trihalogenmethyl zu Carboxy hydrolysiert. Cyano, gegebenenfalls S-verestertes Thiocarboxy, anhydridisiertes Carboxy, von verestertem oder amidiertem Carboxy $R_1$ verschiedenes verestertes oder amidiertes Carboxy und durch Hydroxy oder Amino substituiertes Carbamoyl werden beispielsweise mit einem geeigneten Alkohol zu verestertem Carboxy alkoholysiert und Cyano und anhydridisiertes Carboxy beispielsweise mit Ammoniak bzw. einem geeigneten Amin zu amidiertem Carboxy ammono- bzw. aminolysiert. Gegebenenfalls am Ring A befindliche Niederalkanoyloxy-Reste bzw. Acyloxy-Reste $-OR_o$ können beispielsweise im Verlauf der Hydrolyse zu Hydroxy hydrolysiert werden.

Lactone der Formel (II), d.h. solche Verbindungen der Formel (II), worin $X_1$ Wasserstoff ist und $X_2$ gemeinsam mit $R'_o$ die Gruppe $>C=O$ bilden, werden beispielsweise in Gegenwart einer Säure oder insbesondere Base zu solchen Verbindungen der Formel (I) hydrolysiert, worin R Carboxy bzw. Carboxylat und $R_o$ Wasserstoff darstellen.

In einer bevorzugten Ausführungsform des vorstehenden Verfahrens geht man von Verbindungen der Formel (II) aus, worin $X_1$ Wasserstoff ist und $X_2$ gemeinsam mit $R'_o$ die Gruppe $>C=O$ bilden, und setzt diese mit einem Alkalimetallhydroxid unter Erwärmen, beispielsweise bei etwa 0° bis etwa 150°C, unter hydrolytischer Spaltung des Lactonringes zu Verbindungen der Formel (I) bzw. deren Salzen um, worin $R_1$ Carboxy bzw. Carboxylat und $R_o$ Wasserstoff bedeuten. In nachfolgenden fakultativen Nachfolgereaktionen wird, wenn erwünscht, Carboxy $R_1$ in amidiertes oder verestertes Carboxy $R_1$ und Hydroxy $-OR_o$ in verestertes Hydroxy $OR_o$ überführt.

Ketene der Formel (II), d.h. solche Verbindungen der Formel (II), worin $X_1$ und $X_2$ gemeinsam die Gruppe $=C=O$ bilden und $R'_o$ die Bedeutung von $R_o$ hat, können beispielsweise durch Addition von Wasser, einem geeigneten Alkohol, Ammoniak oder einem geeigneten Amin in entsprechende Verbindungen der Formel (I) oder deren Salze übergeführt werden.

Verbindungen der Formel (II), worin $X_1$ und $X_2$ gemeinsam die Gruppe $=C(Hal)_2$ bilden und $R'_o$ die Bedeutung von $R_o$ hat, können beispielsweise durch Hydrolyse mit Wasser, insbesondere in Gegenwart einer Säure, wie Mineralsäure, z.B. Schwefelsäure, gegebenenfalls unter Erwärmen, wie in einem Temperaturbereich zwischen etwa 50° und etwa 150°C, in solche Verbindungen der Formel (I), worin $R_1$ Carboxy bedeutet, übergeführt werden.

Die Ausgangsstoffe der Formel (II) oder deren Salze, worin $X_1$ Wasserstoff ist, $X_2$ von $R_1$ verschiedenes funktionell abgewandeltes Carboxy darstellt und $R'_o$ die Bedeutung von $R_o$ hat, erhält man auf an sich bekannten Wegen. Beispielsweise geht man von Verbindungen der Formel

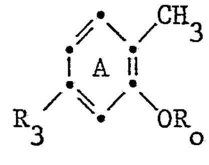

(IIa)

oder deren Salzen aus. Diese werden z.B. mit Halogenierungsmitteln, wie N-Bromsuccinimid, in Gegenwart eines Radikalbildners, wie Benzoylperoxid oder Azobisisobutyronitril, und unter Erwärmen in einem inerten Lösungsmittel, wie Benzol, zu Verbindungen der Formel

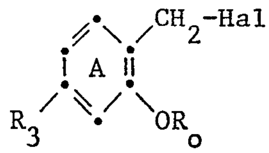

(IIb),

worin Hal Halogen, insbesondere Brom oder Chlor, bedeutet, oder deren Salzen umgesetzt. Die so erhältlichen Verbindungen der Formel (IIb) werden durch Behandlung mit Alkalimetallcyaniden, z.B. Natriumcyanid, gegebenenfalls unter Erwärmen in einem geeigneten Lösungsmittel, wie Dimethylsulfoxid, in die entsprechenden Nitrile übergeführt. In einer fakultativen Massnahme kann in den so erhältlichen Verbindungen der Formel

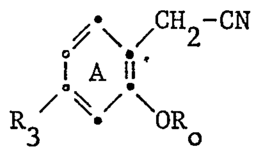

(IIc)

oder deren Salzen der Rest $R_2$ durch Umsetzung mit einer Verbindung $R_2$-Hal, worin Hal Halogen bedeutet, in Gegenwart einer Base, wie eines Alkalimetallamides oder -hydrides, z.B. Natriumamid oder -hydrid, bei niedrigen Temperaturen, z.B. unter 10°C, und in einem geeigneten Lösungsmittel, wie Dimethylformamid, eingeführt werden.

Die Cyanogruppe kann nunmehr, wenn erwünscht, nach an sich bekannter Weise in andere von $R_1$ verschiedene funktionell abgewandelte Carboxygruppen übergeführt werden, beispielsweise in gegebenenfalls substituiertes Amindino, gegebenenfalls substituiertes Thiocarbamoyl, gegebenenfalls verestertes oder anhydridisiertes Carboximidoyl oder von amidiertem oder verestertem Carboxy $R_1$ verschiedenes amidiertes oder verestertes Carboxy.

So kann beispielsweise aus der Cyano-Gruppe z.B. durch Behandeln mit einem Alkohol in Gegenwart einer starken Säure das entsprechende Alkoxycarbamidoyl, durch Behandeln mit Wasserstoffperoxid in Gegenwart einer Protonsäure Carbamoyl, durch Behandeln mit Schwefelwasserstoff in Gegenwart einer anorganischen Base das entsprechende Thiocarbamoyl, durch Umsetzung mit einem Ueberschuss an Alkohol in Gegenwart einer Säure das entsprechende veresterte Carboxy zugänglich gemacht werden. Aus Alkoxycarbamidoyl wiederum lässt sich z.B. durch Behandeln mit Ammoniak, einem primären oder sekundären Amin z.B. entsprechendes Amidino und bei Umsetzung mit mindestens 2 Aequivalenten eines Alkohols z.B. entsprechendes Trialkoxymethyl erhalten.

In einer bevorzugten Ausführungsform gelangt man zu Lactonen der Formel (II), worin $X_1$ Wasserstoff ist und $X_2$ gemeinsam mit $R_o'$ die Gruppe $\rangle C=O$ bilden und worin der Ring A bis auf $R_3$ unsubstituiert oder durch Niederalkyl ein- oder mehrfach substituiert oder gegebenenfalls zusätzlich durch 3- oder 4-gliedriges Alkylen disubstituiert ist, und $R_2$ Methyl darstellt, indem man Verbindungen der Formel

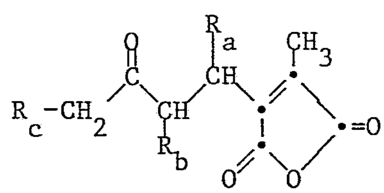

(IId),

worin $R_a$, $R_b$ und $R_c$ unabhängig voneinander Wasserstoff, Niederalkyl oder 3- oder 4-gliedriges Alkylen bedeuten, mit Aminen der Formel $R_3$-H oder deren Säureadditionssalzen umsetzt.

Die Umsetzung erfolgt beispielsweise bei erhöhter Temperatur, z.B. in der Schmelze oder bei der Rückflusstemperatur des Lösungsmittels, z.B. in einem Temperaturbereich von etwa 80° C bis etwa 200° C. Geeignete inerte Lösungsmittel sind beispielsweise höher siedende Kohlenwasserstoffe, wie aromatische Kohlenwasserstoffe, z.B. Benzol, Toluol oder Xylole. Die Amine der Formel $R_3$-H werden insbesondere als Säureadditionssalze, z.B. vorteilhaft als Benzoate, eingesetzt.

Zur Herstellung von Verbindungen der Formel (IId), worin $R_a$ Wasserstoff bedeutet, geht man von Verbindungen der Formel

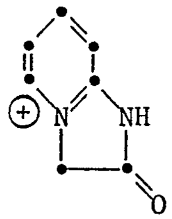 $A^{\ominus}$ (IIe)

aus, die gegebenenfalls im aromatischen Teil substituiert sind und worin $A^{\ominus}$ das Anion einer anorganischen oder organischen Säure darstellt, und setzt diese mit Fumarsäure, Maleinsäure oder Maleinsäureanhydrid in Gegenwart einer Base um, wobei als Basen anorganische oder organische Basen in Frage kommen. Anorganische Basen sind beispielsweise Alkalimetallhydroxide oder -hydride, wie Natrium- oder Kaliumhydroxid oder Natrium- oder Kaliumhydrid. Als organische Amine werden beispielsweise tertiäre Amine, wie Trialkylamine, z.B. Triethylamine oder Tri-n-butylamine, oder cyclische Amine, wie Pyridin, Picolin, Chinolin oder Lutidin, verwendet.

Die auf diesem Wege zunächst erhältlichen freien Verbindungen werden durch Behandeln mit organischen oder anorganischen Säuren in die Salze der Formel

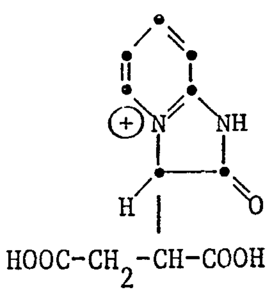 $A^{\ominus}$ (IIf) ,

übergeführt. Diese werden im weiteren Reaktionsverlauf, gegebenenfalls in Gegenwart einer der vorstehend genannten Basen, mit Verbindungen der Formel $R_a-CH = C(R_b)-CO-CH_2-R_c$ (IIg) zu Verbindungen der Formel

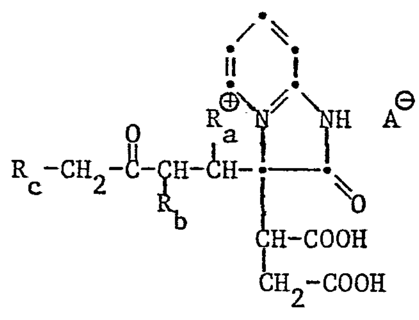

(IIh)

umgesetzt, die im nächsten Reaktionsschritt durch Erhitzen, z.B. auf Temperaturen zwischen 80 und 160°C, unter Decarboxylierung in Verbindungen der Formel

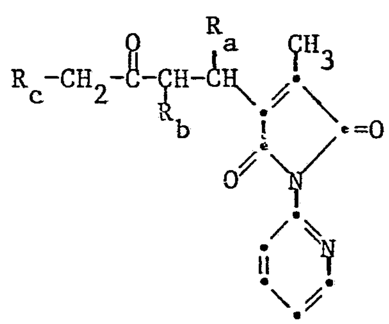

(IIi)

übergeführt werden. Die thermische Ueberführung von Verbindungen der Formel (IIh) in Verbindungen der Formel (IIi) wird beispielsweise in einem gegebenenfalls halogenierten aromatischen Lösungsmittel, wie Benzol, Toluol, Xylole oder Chlorbenzol, oder einer Niederalkancarbonsäure, z.B. Eisessig, durchgeführt. Anschliessend werden die Verbindungen der Formel (IIi) zu Verbindungen der Formel (IId) hydrolysiert. Die Hydrolyse wird beispielsweise in wässrigem oder wässrig-organischem Medium vorgenommen. Als organische Lösungsmittel eignen sich vor allem hochsiedende polare Lösungsmittel, wie Ether, z.B. Dioxan oder Tetrahydrofuran, N,N-Dialkylamide, z.B. N,N-Dimethyl-formamid oder

N,N-Dimethylacetamid, oder cyclische Amide, wie N-Methyl-pyrrolidon. Die Hydrolyse erfolgt beispielsweise mit Hilfe einer anorganischen oder organischen Säure, wobei als anorganische Säuren Mineralsäuren, wie Halogenwasserstoffsäure oder Schwefelsäure, und als organische Säuren Sulfonsäuren, wie Niederalkan- oder gegebenenfalls substituierte Benzolsulfonsäuren, wie Methan- oder p-Toluolsulfonsäure, oder gegebenenfalls substituierte Alkancarbonsäuren, wie Eisessig, in Betracht kommen.

Zur Herstellung von Verbindungen der Formel (IId), worin $R_a$ von Wasserstoff verschieden ist, geht man von Verbindungen der Formel (IIe) aus und setzt diese zunächst mit Verbindungen der Formel (IIg) und anschliessend mit Fumarsäure, Maleinsäure oder insbesondere mit Maleinsäureanhydrid zu Verbindungen der Formel (IIh) um, die wiederum, wie vorstehend beschrieben, weiter zu den entsprechenden Verbindungen der Formel (IId) weiterreagieren.

In einer weiteren vorteilhaften Verfahrensweise gelangt man zu Verbindungen der Formel (II), worin $X_1$ Wasserstoff, $X_2$ von $R_1$ verschiedenes, funktionell abgewandeltes Carboxy bedeutet und $R'_o$ die Bedeutung von $R_o$ hat und worin $R_2$ Wasserstoff ist, indem man von Verbindungen der Formel

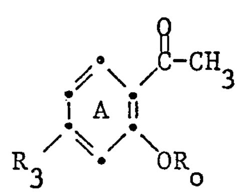

(IIk)

oder deren Salzen ausgeht und diese mit Schwefel und einem primären oder sekundären Amin, vorteilhafterweise mit Morpholin oder Thiomorpholin, oder mit Ammoniumpolysulfid unter Druck umsetzt, analog der Willgerodt(-Kindler)-Reaktion. In einer so erhältlichen Verbindung der Formel (II) bedeutet $X_2$ von $R_1$ verschiedenes substituiertes Carbamoyl bzw. entsprechend substituiertes Thiocarbamoyl, welches in an sich bekannter Weise, z.B. durch entsprechende Solvolyse, in anderes von $R_1$ verschiedenes funktionell abgewandeltes Carboxy $X_2$ überführt werden kann.

Die neuen Verbindungen der Formel (I) können ferner hergestellt werden, indem in Verbindungen der Formel

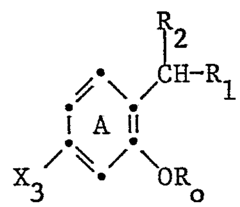

(III)

oder deren Salzen, worin $X_3$ einen in $R_3$ überführbaren Rest darstellt, $X_3$ in $R_3$ überführt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/oder gewünschtenfalls ein verfahrensgemäss erhältliches Isomerengemisch in seine Komponenten auftrennt.

Ein in $R_3$ überführbarer Rest $X_3$ bedeutet beispielsweise Amino oder eine Gruppe der Formel $-NH-A_1-X_4$ bzw. $-NH-A_2-X_5$, wobei $A_1$ einen zweiwertigen Kohlenwasserstoff, z.B. gegebenenfalls verzweigtes Niederalkylen, bedeutet, $X_4$ Wasserstoff, 3- bis 7-gliedriges Cycloalkyl oder Aryl, wie unsubstituiertes oder durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro substituiertes Phenyl darstellt, $A_2$ einen zweiwertigen Kohlenwasserstoffrest, der auch durch Aza, N-Niederalkylaza, Oxa oder Thia unterbrochen sein kann, z.B. Niederalkylen, Niederalkenylen, durch Aza, N-Niederalkylaza, Oxa oder Thia unterbrochenes Niederalkylen oder durch Aza, N-Niederalkylaza, Oxa oder Thia unterbrochenes Niederalkenylen, bedeutet, wobei Niederalkylen bzw. Niederalkenylen jeweils auch verzweigt sein können und ferner auch zusätzlich ein oder zwei orthoanellierte Benzosysteme aufweisen können, und $X_5$ Hydroxy oder reaktionsfähiges verestertes Hydroxy darstellt. Unter reaktionsfähigem verestertem Hydroxy $X_5$ ist beispielsweise mit einer starken anorganischen Mineralsäure, wie Halogenwasserstoffsäure oder Schwefelsäure, mit einer organischen Sulfonsäure, wie Niederalkan- oder gegebenenfalls substituierten Benzolsulfonsäure, z.B. Methan- oder p-Toluolsulfonsäure, ferner mit einer organischen Carbonsäure, wie Niederalkancarbonsäure, z.B. Essigsäure, verestertes Hydroxy zu verstehen, beispielsweise in erster Linie Halogen, wie Chlor oder Brom, Sulfonyloxy, wie p-Toluolsulfonyloxy.

Die Ueberführung von $-NH-A_1-X_4$ in $R_3$ erfolgt in an sich bekannter Weise. So setzt man beispielsweise entsprechende Verbindungen der Formel (III) oder deren Salze mit Verbindungen der Formel $X_4-A_1-X_5$ (IIIa) oder deren Salzen um. Dabei arbeitet man gegebenenfalls in einem inerten Lösungs- oder Verdünnungsmittel, unter Schutzgas, z.B. Stickstoff, und/ oder erforderlichenfalls in Gegenwart eines Kondensationsmittels, wie eines Alkalimetall- oder Erdalkalimetallhydroxides bzw. -carbonats oder eines Erdalkalimetallalkoholats, z.B. Natriumhydroxid, Kaliumhydrogencarbonat oder Natriummethylat, beispielsweise in einem Temperaturbereich von etwa 0° bis 150°C. Als Lösungsmittel eignen sich z.B. aliphatische Alkohole, wie Methanol oder Ethanol, oder aromatische Kohlenstoffe, wie Benzol oder Toluol.

Die Ueberführung von $-NH-A_2-X_5$ in $R_3$ erfolgt in der vorstehend beschriebenen Weise.

Ein Rest $R_3$, der für eine durch einen zweiwertigen Kohlenwasserstoffrest disubstituierte Aminogruppe steht, kann auch direkt eingeführt werden, z.B. durch Umsetzung von Verbindungen der Formel (III), worin $X_3$ Amino bedeutet, oder deren Salzen mit Verbindungen der Formel $X_5-A_2-X_5$ (IIIb). Dabei wird in der vorstehend beschriebenen Weise verfahren. Bei diesen Reaktionen können auch in situ Verbindungen der Formel (III), worin $X_3$ eine Gruppe der Formel $-NH-A_2-X_5$ gebildet werden, die unter den Reaktionsbedingungen direkt zu entsprechenden Verbindungen der Formel (I) weiterreagieren.

Ein Rest $R_3$ kann, sofern dieser nicht-aromatischer Natur ist, weiterhin direkt eingeführt werden, indem man beispielsweise von Verbindungen der Formel (III), worin $X_3$ Wasserstoff, einen Metall-aufweisenden Rest oder gegebenenfalls reaktionsfähiges verestertes Hydroxy bedeutet, oder deren Salzen ausgeht und diese mit Verbindungen der Formel $R_3-X_6$, worin $X_6$ Wasserstoff, einen Metall-enthaltenden Rest oder gegebenenfalls reaktionsfähiges verestertes Hydroxy darstellt, oder deren Salzen umsetzt.

Ein Metall-aufweisender Rest ist beispielsweise ein Alkalimetallatom, wie Lithium oder Natrium. Reaktionsfähiges verestertes Hydroxy bedeutet beispielsweise mit einer Mineral-, wie Halogenwasserstoffsäure, oder einer Sulfonsäure, wie gegebenenfalls substituierte Benzolsulfonsäure, verestertes Hydroxy.

In erster Linie setzt man z.B. Verbindungen der Formel (III) und $R_3-X_6$ um, worin einer der Reste $X_3$ und $X_6$ ein Alkalimetallatom, wie Lithium, und der andere Halogen, wie Brom, darstellt.

Für den Fall, dass $X_3$ Wasserstoff bedeutet und $X_6$ für Hydroxy oder Halogen steht, wird die Umsetzung in Gegenwart einer Lewissäure durchgeführt. Bedeutet $X_3$ Halogen und $X_6$ Wasserstoff, erfolgt die Reaktion in Gegenwart eines Kondensationsmittels.

Zur Herstellung von Ausgangsstoffen der Formel (III) geht man nach an sich bekannter Weise vor und spaltet beispielsweise in Verbindungen der Formel

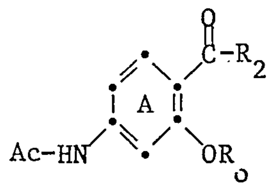

(IIIc)

oder deren Salzen, worin Ac ein Acylrest, wie Niederalkanoyl, z.B. Acetyl, bedeutet, Acyl Ac in Gegenwart einer Base, wie eines Alkalimetallhydroxides, z.B. Natriumhydroxid, ab. Dabei können Niederalkanoyloxygruppen zu Hydroxy hydrolysiert werden, die allerdings gewünschtenfalls wie üblich wieder verestert werden können. In so erhältlichen Verbindungen der Formel

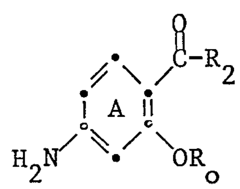

(IIId)

oder deren Salzen wird die Aminogruppe durch Umsetzung mit Benzylhalogeniden, insbesondere mit Benzylchlorid, benzyliert. Anschliessend erfolgt eine Reduktion der Carbonylfunktion, beispielsweise mit Hilfe von gegebenenfalls komplexen Hydriden, z.B. mit Natriumborhydrid.

Hieraus resultieren Verbindungen der Formel

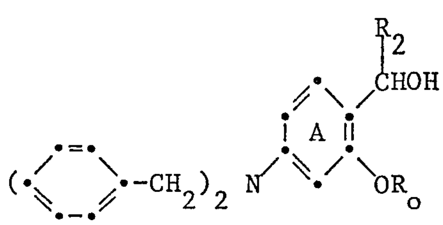

(IIIe)

oder deren Salze.
Diese setzt man beispielsweise mit Alkalimetallcyaniden, wie Natriumcyanid, unter Erwärmen um und solvolysiert anschliessend die Cyanogruppe zu $R_1$. Im nächsten Reaktionsschritt werden die Benzylgruppen hydrogenolytisch in Gegenwart eines Hydrierungskatalysators, wie Platin, abgespalten und die nunmehr freie Aminogruppe durch Behandeln mit Verbindungen der Formel $X_3$-$X_5$ (IIIf) in Gegenwart eines Kondensations-

mittels, wie eines Alkalimetallhydroxides, in den Rest $X_3$ übergeführt, wobei $X_3$ von Wasserstoff, einem Metall-aufweisenden Rest oder gegebenenfalls reaktionsfähigem verestertem Hydroxy verschieden ist.

Verbindungen der Formel (I), worin $R_3$ Pyrrol-1-yl bedeutet, sind erhältlich, indem man Verbindungen der Formel (III), worin $X_3$ Amino bedeutet, oder deren Salze mit 2-Buten-1,4-diol oder einem reaktionsfähig veresterten Derivat davon in Gegenwart einer Protonsäure, wie Niederalkancarbonsäure, zum Pyrrolin-1-yl umsetzt und dehydriert dieses in Gegenwart eines Dehydrierungskatalysators, wie eines Chinons, z.B. 2,3-Dichlor-5,6-dicyano-p-benzochinon oder Tetrachlor-p-benzochinon, oder eines Selenderivates, z.B. Selendioxid, oder eines Elements der VIII. Nebengruppe, wie Palldium, oder indem man Verbindungen der Formel (III), worin $X_3$ für Amino steht, oder deren Salze mit einem 2,5-Diniederalkoxytetrahydrofuran, wie 2,5-Dimethoxy-tetrahydrofuran, z.B. unter Erwärmen, behandelt.

Weiterhin kann der Pyrrolring $R_3$ aufgebaut werden, indem beispielsweise die Aminogruppe $X_3$ in Verbindungen der Formel (III) mit einem gegebenenfalls reaktionsfähig verestertem Derivat von 1,3-Butadien-1,4-diol, z.B. mit 1,4-Dibrom-1,3-butadien, erforderlichenfalls unter Erwärmen und unter Schutzgas z.B. Stickstoff, und in einem inerten Lösungs- oder Verdünnungsmittel umgesetzt wird.

Ferner kann der Pyrrolring $R_3$ analog Knorr-Paal durch Behandeln der Aminogruppe $X_3$ in Verbindungen der Formel (III) mit gegebenenfalls acetalisiertem 1,4-Dioxo-butan aufgebaut werden, wobei unter inerten Bedingungen, beispielsweise unter Schutzgas und Erwärmen und in einem inerten Lösungsmittel gearbeitet werden kann.

Eine weitere Verfahrensvariante zum Aufbau des Pyrrolrings $R_3$ besteht z.B. in der Umsetzung von Verbindungen der Formel (III), worin $X_3$ z.B. die Gruppe der Formel -NH-CH=CH-CH=CH-OH bzw. eine reaktionsfähige veresterte Form, ferner eine tautomere Form davon, die gegebenenfalls acetalisiert ist, darstellt. Hierbei wird die Reaktion vorteilhaft unter inerten Bedingungen und unter Erwärmen durchgeführt.

Reaktionsfähiges verestertes Hydroxy bedeutet in diesem Zusammenhang jeweils z.B. mit einer Mineralsäure, wie Halogenwasserstoffsäure, z.B. Bromwasserstoffsäure, oder mit einer Sulfonsäure, wie Niederalkan- oder gegebenenfalls substituierten Benzolsulfonsäure, p-Toluolsulfonsäure, verestertes Hydroxy.

Ebenso können genügend nucleophile Amine $R_3$-H direkt in solche Verbindungen der Formel (III) eingeführt werden, worin $X_3$ für einen durch $R_3$ ersetzbaren Rest steht. Bedeutet beispielsweise $X_3$ Halogen, insbesondere Chlor, Brom oder Iod, kann die Umsetzung in An- oder Abwesenheit eines Lösungsmittels und je nach Wahl des Halogenatoms bei niedrigen Temperaturen bis zur Siedetemperatur des betreffenden Lösungsmittels erfolgen. Vorteilhaft befindet sich in der Nachbarposition von $X_3$ ein Substituent mit starkem -I- oder -M-Effekt, wie Nitro, Halogen oder Trifluormethyl. In einzelnen Fällen ist es von Vorteil, die Umsetzung unter Druck oder bei erhöhter Temperatur durchzuführen. Vorteilhaft setzt man die Amine im Ueberschuss ein.

Ebenso können genügend nucleophile Amine $R_3$-H direkt in solche Verbindungen der Formel (III) eingeführt werden, worin $R_o$ sowie $X_3$ jeweils für Wasserstoff stehen. Hierzu werden z.B. entsprechende Verbindungen der Formel (III) zunächst mit einem Oxidationsmittel, wie Blei(IV)acetat, z.B. in Gegenwart einer geeigneten Säure, wie Eisessig, und bei Raumtemperatur, behandelt und anschliessend mit entsprechenden Aminen der Formel $R_3$-H in einem inerten Lösungsmittel, wie einem Ether, z.B. Dioxan, unter Erhitzen, z.B. auf Rückflusstemperatur, umgesetzt, woraus insbesondere solche Verbindungen der Formel (I) resultieren können, worin $R_1$ entsprechend amidiertes Carboxy bedeutet.

Werden diese Umsetzungen in Gegenwart einer Base durchgeführt, kann gegebenenfalls vorhandenes Acyl, wie Niederalkanoyloxy zu Hydroxy und/oder verestertes oder amidiertes Carboxy zu Carboxy hydrolysiert werden.

In einer weiteren Arbeitsweise erhält man Verbindungen der Formel (I), worin $R_o$ Wasserstoff ist, indem man in Verbindungen der Formel

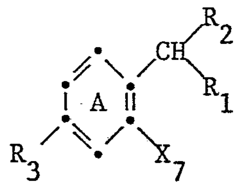

(IV),

worin $X_7$ einen in die Gruppe $-OR_o$ überführbaren Rest darstellt, den Rest $X_7$ in die Gruppe $-OR_o$ überführt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/oder gewünschtenfalls ein verfahrensgemäss erhältliches Isomerengemisch in seine Komponenten auftrennt.

Ein in die Gruppe $-OR_o$ überführbarer Rest $X_7$ ist beispielsweise eine Diazoniumgruppe mit einem Anion einer anorganischen oder organischen Säure als Gegenion.

Die Substitution der Diazoniumgruppe durch Hydroxy erfolgt in an sich bekannter Weise, beispielsweise durch Erwärmen, z.B. auf etwa 100° bis etwa 250°C, in wässriger Lösung. Häufig wird diese Umsetzung in Gegenwart von Säuren, wie Mineralsäuren, insbesondere Schwefel- oder Orthophosphorsäure, durchgeführt und als Gegenion wird das Hydrogensulfation bevorzugt. Zur Vermeidung einer Azokupplung wird das gebildete Phenol laufend aus dem Reaktionsgemisch entfernt, beispielsweise durch Ausschütteln mit einem geeigneten Lösungsmittel.

Die Ausgangsstoffe der Formel (IV) können nach an sich bekanntem Verfahren hergestellt werden, beispielsweise indem man Verbindungen der Formel

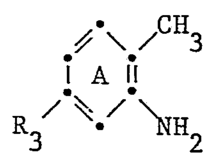

(IVa)

oder deren Salzen ausgeht und die Aminogruppe gegebenenfalls durch Einführung einer Schutzgruppe schützt. Als Schutzgruppen kommen beispielsweise Acyl- oder Benzylgruppen in Betracht. Vorteilhafterweise wird die

Aminogruppe, z.B. mit Benzylchlorid, benzyliert. Die sich hieran anschliessende Halogenierung der Methylgruppe, z.B. die Bromierung mit N-Bromsuccinimid in Gegenwart von Azobisisobutyronitril unter Erwärmen, führt zu den entsprechenden Verbindungen der Formel

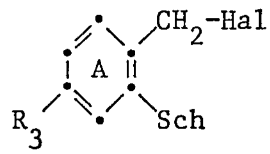

(IVb),

worin Hal Halogen, insbesondere Brom oder Chlor, bedeutet und Sch eine gegebenenfalls geschützte Aminogruppe darstellt. Diese Verbindungen werden anschliessend mit einem Alkalimetallcyanid, wie Natriumcyanid, beispielsweise unter Erwärmen in Dimethylformamid, umgesetzt. In den so erhältlichen Verbindungen der Formel

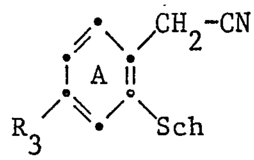

(IVc)

wird, sofern erwünscht, der Rest $R_2$ z.B. durch Umsetzung mit Verbindungen der Formel $R_2$-Hal (IVd) in Gegenwart einer Base, wie eines Alkalimetallhydrides, eingeführt. Im nächsten Reaktionsschritt wird die Cyanogruppe durch übliche Solvolyse in $R_1$ überführt und anschliessend die Aminoschutzgruppe abgespalten. Vorteilhaft werden beispielsweise die die Aminogruppen schützenden Benzylgruppen hydrogenolytisch in Gegenwart eines Hydrierungskatalysators, z.B. Palladium, abgespalten. Die so erhältlichen Verbindungen der Formel

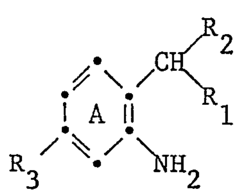

(IVe)

oder deren Salze werden z.B. bei niedrigen Temperaturen mit einer Mineralsäure, wie Schwefelsäure, und wässriger Alkalimetall-, wie Natriumnitritlösung, versetzt. Die dabei intermediär entstehenden Verbindungen der Formel (IV), worin $X_7$ eine Diazoniumgruppe mit einem entsprechenden Gegenion bedeutet, wie vorstehend beschrieben, weiter zu Verbindungen der Formel (I) umgesetzt.

Ein in die Gruppe $OR_o$ überführbarer Rest $X_7$ kann weiterhin z.B. verethertes Hydroxy oder von $OR_o$ verschiedenes Acyloxy bedeuten.

Verethertes Hydroxy stellt beispielsweise mit einem aliphatischen Alkohol verethertes Hydroxy dar, wobei als aliphatischer Alkohol beispielsweise ein gegebenenfalls substituiertes Alkanol, wie Niederalkanol, in Frage kommt. Beispiele für derartige Ether sind gegebenenfalls durch Hydroxy, Halogen, Alkoxy, z.B. Niederalkoxy, Carboxy bzw. ein funktionelles Derivat davon, Nitro, gegebenenfalls substituiertes Amino, Aryl, wie gegebenenfalls substituiertes Phenyl, Alkylthio, Alkansulfinyl, Alkansulfonyl, Alkanoyl substituiertes Alkoxy, wie entsprechendes Niederalkoxy.

Verethertes Hydroxy lässt sich beispielsweise üblicherweise mit Hilfe der Etherspaltung in Hydroxy $OR_o$ überführen, so beispielsweise durch Behandeln mit einer starken Protonsäure, wie Halogenwasserstoffsäure, z.B. Brom- oder Iodwasserstoffsäure, oder mit einer geeigneten Lewissäure, wie einem Halogenid von Elementen der 3. Hauptgruppe, z.B. Bortribromid. Die Etherspaltung mit einer Protonsäure wird vorteilhaft bei erhöhter Temperatur, z.B. bei etwa 150° bis 250°C, und die Spaltung mit einer Lewissäure vorteilhaft unter Kühlen, z.B. bei etwa -78° bis 0°C, ferner auch bei Raumtemperatur, durchgeführt. Weiterhin können entsprechende Ether auch mit Hilfe von stark nucleophilen Reagentien, wie Alkalimetallniederalkanolaten, z.B. Natriummethylat, starken Amiden, z.B. Methyl- oder Triethylamin, oder einem Thiophenolat, z.B. Natrium-p-methyl-thiophenolat, gespaltet werden, wobei vorteilhaft bei erhöhter Temperatur gearbeitet wird. Die Etherspaltung kann z.B. in An- oder Abwesenheit eines Lösungsmittels und bei Temperaturen von etwa 0° bis etwa 250°C durchgeführt werden. Als Lösungsmittel kommen z.B. halogenierte Kohlenwasserstoffe, wie entsprechende Halogen-Niederalkane, z.B. Methylenchlorid, in Frage.

Von Acyloxy $OR_o$ verschiedenes Acyloxy $X_7$ ist beispielsweise Aroyloxy, wie gegebenenfalls substituiertes Alkanoyloxy, wobei als Substituenten von

Aroyloxy, z.B. Benzyloxy z.B. die eingangs für Phenylreste genannten Substituenten und als Substituenten von Alkanoyloxy, wie Niederalkanoyloxy, z.B. Hydroxy, Halogen, Alkoxy, Carboxy bzw. funktionelle Derivate davon, Nitro, gegebenenfalls substituiertes Amino, Aryl, wie gegebenenfalls substituiertes Phenyl, Alkylthio, Alkansulfinyl, Alkansulfonyl oder Alkanoyloxy in Frage kommen.

Entsprechendes Acyloxy $X_7$ wird in an sich bekannter Weise, z.B. durch Hydrolyse in Hydroxy $OR_o$ übergeführt. So wird die Hydrolyse beispielsweise in Gegenwart einer Protonsäure, wie Mineralsäure, oder vorteilhaft in Gegenwart einer Base, wie einem Alkalimetallhydroxid oder -karbonat, gegebenenfalls unter Erwärmen und z.B. in einem inerten Lösungs- oder Verdünnungsmittel durchgeführt. Dabei kann ebenfalls funktionell abgewandeltes Carboxyl $R_1$ zu Carboxy hydrolysiert werden. Die Hydrolyse des Esters $OR'_o$ in OH kann z.B. in einem inerten Lösungsmittel, wie einem Niederalkanol, Ether, z.B. Dioxan, Wasser, einem Amid, wie Dimethylformamid, sowie Gemischen derselben und in einem Temperaturbereich von etwa -20° bis etwa 300°C durchgeführt werden. Unter diesen Hydrolysebedingungen kann ebenso von Carboxy verschiedenes $R_1$ hydrolysiert werden.

Das Ausgangsmaterial der Formel (IV), worin $X_7$ für verethertes oder von $OR_o$ verschiedenes Acyloxy bedeutet, kann, sofern nicht bekannt, nach an sich bekannten Verfahren hergestellt werden. So geht man beispielsweise von einem entsprechenden 3-Nitrophenol aus, verethert, z.B. mit Hilfe eines entsprechenden Alkohols in Gegenwart einer starken Mineralsäure und unter Erwärmen, oder verestert, z.B. mit Hilfe eines entsprechenden Acylhalogenids, die phenolische OH-Gruppe. Anschliessende Reduktion der Nitrogruppe, z.B. mit Wasserstoff in Gegenwart eines Hydrierungskatalysators, führt zum entsprechenden Amin, welches analog zu der vorstehend beschriebenen Weise in $R_3$ überführt werden kann. Die so erhältlichen Verbindungen der Formel

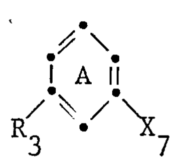

werden beispielsweise mit einem Oxalylhalogenid-Derivat in Gegenwart einer Lewis-Säure, wie Aluminiumchlorid, acyliert und das resultierende Glyoxylsäurederivat analog der Wolff-Kishner-Reaktion oder der Methode von Huang-Minlon z.B. mit Hydrazin in einem hochsiedenden Lösungsmittel in Gegenwart einer Base, wie Natriumhydroxid, gekocht und das intermediär gebildete Hydrazon thermisch zersetzt, wobei die Carbonylgruppe zur Methylengruppe reduziert wird. Anschliessend kann fakultativ durch Umsetzung mit einem Halogenid $R_2$-Hal in Gegenwart einer Base, wie Natriumamid, der Rest $R_2$ eingeführt werden.

Die erfindungsgemässen Verbindungen können weiterhin hergestellt werden, indem man Verbindungen der Formel

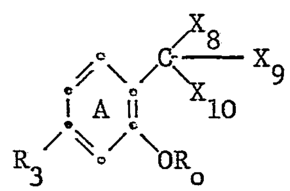

(V)

oder deren Salze, worin $X_8$ und $X_9$ jeweils Carboxy bedeuten, und $X_{10}$ die Bedeutung von $R_2$ hat, worin $X_8$ die Bedeutung von $R_1$ und $X_9$ die von $R_2$ hat und $X_{10}$ Hydroxy, funktionell abgewandeltes Hydroxy, durch einen Kohlenwasserstoffrest substituiertes Mercapto, oder sekundäres Amino darstellt, worin $X_8$ die Bedeutung von $R_1$ hat und $X_9$ und $X_{10}$ gemeinsam Oxo, Thioxo oder gegebenenfalls substituiertes Hydrazono bedeuten, oder worin $X_8$ die Bedeutung von $R_1$ hat und $X_9$ und $X_{10}$ gemeinsam die Gruppen = $R_2'$ oder eine tautomere Form hierzu bilden und $R_2'$ für einen zweiwertigen aliphatischen Rest steht, durch Reduktion in entsprechende Verbindungen der Formel (I) überführt und/oder, wenn erwünscht, ein verfahrensgemäss erhältliches Salz in die freie Verbindung oder in ein anderes Salz, eine verfahrensgemäss erhältliche freie Verbindung in eine andere freie Verbindung oder in ein Salz überführt und/oder gewünschtenfalls ein verfahrensgemäss erhältliches Isomeren-Gemisch in seine Komponenten auftrennt.

Funktionell abgewandeltes Hydroxy ist beispielsweise verethertes Hydroxy, wie mit einem Niederalkanol, z.B. Methanol, verethertes Hydroxy oder reaktionsfähiges verestertes Hydroxy, z.B. mit starken Mineralsäuren, mit organischen Sulfonsäuren, wie Niederalkan- oder gegebenenfalls substituierte Benzolsulfonsäure, oder mit organischen Carbonsäuren, wie Niederalkancarbonsäure, verestertes Hydroxy.

Sekundäres Amino ist beispielsweise Dialkylamino, wie Diniederalkylamino, oder gegebenenfalls im Phenylteil substituiertes Di-phenylsulfamoyl, insbesondere Di-(p-toluol)- oder Di-(p-bromphenyl)-sulfamoyl.

Durch einen Kohlenwasserstoffrest substituiertes Mercapto bedeutet beispielsweise durch einen Alkylrest substituiertes Mercapto, wobei der Alkylrest seinerseits gegebenenfalls z.B. durch einen aromatischen, wie gegebenenfalls substituierten Phenylrest substituiert sein kann, wie Niederalkylthio, z.B. Methyl- oder Ethylthio, oder Phenylniederalkylthio, z.B. Benzylthio.

Hydrazono kann beispielsweise durch einen Sulfonylrest, wie gegebenenfalls substituierten Phenylsulfonylrest, z.B. p-Toluolsulfonyl, oder durch einen gegebenenfalls substituierten Phenylrest substituiert sein.

Ein zweiwertiger aliphatischer Rest ist z.B. ein Niederalkyliden- oder Niederalkenylidenrest und als tautomere Form von $= R_2'$ kommt z.B. ein entsprechender Niederalkenylenrest in Frage, wobei Niederalkenylen eine oder mehrere Doppelbindungen aufweist.

Die Reduktion erfolgt nach an sich bekannter Weise, beispielsweise unter inerten Bedingungen, wie unter Schutzgas, z.B. Stickstoff, in einem inerten Lösungs- oder Verdünnungsmittel, gegebenenfalls unter Druck und/oder unter Kühlen oder Erwärmen.

Die Decarboxylierung von Verbindungen der Formel (V), worin $X_8$ und $X_9$ jeweils Carboxy bedeuten und $X_{10}$ die Bedeutung von $R_2$ hat, wird unter

Erhitzen, beispielsweise in einem Temperaturbereich von etwa 100° bis etwa 300°C, gegebenenfalls in Gegenwart eines Uebergangsmetalles oder einer Legierung davon, z.B. Kupfer oder Kupferbronze, oder eines Amins, wie eines basischen Stickstoffheterocyclus, z.B. Pyridin oder Chinolin, oder eines Alkylamins, wie Triniederalkylamins, durchgeführt und führt zu Verbindungen der Formel (I), worin $R_1$ Carboxy darstellt, oder deren Salzen.

Die reduktive Ueberführung von $X_{10}$ in Verbindungen der Formel (V), worin $X_8$ die Bedeutung von $R_1$ und $X_9$ die von $R_2$ hat und $X_{10}$ Hydroxy, funktionell abgewandeltes Hydroxy, Dialkylamino oder mit einem Kohlenwasserstoffrest substituiertes Mercapto, insbesondere Niederalkylthio, darstellt, mit Wasserstoff, erfolgt beispielsweise durch Hydrierung in Gegenwart eines Hydrierungskatalysators, wie eines Elementes der VIII. Nebengruppe des Periodensystems oder dessen Derivat, z.B. Oxid, wobei der Katalysator gegebenenfalls auf einem Trägermaterial, wie Aktivkohle oder Erdalkalimetallcarbonat, bzw. -sulfat aufgezogen sein kann. Die Hydrierung wird vorteilhaft unter Kühlen oder Erwärmen, z.B. zwischen etwa -80° bis etwa 200°C, vor allem zwischen Raumtemperatur und 100°C, zweckmässig in einem geeigneten Lösungsmittel, wie Wasser, einem Niederalkanol, z.B. Ethanol oder Isopropanol, einem Ether, wie Dioxan, einer Niederalkancarbonsäure, z.B. Essigsäure, oder Gemischen derselben, durchgeführt.

Als Beispiele für solche Katalysatoren sind Raney-Nickel oder Palladium/Kohle, ferner Platin, Platinoxid oder Palladium, zu nennen. Erforderlichenfalls wird die Hydrierung in Gegenwart einer Säure oder insbesondere einer Base durchgeführt. Entsprechende Säuren sind Protonsäuren, wie Mineralsäuren, z.B. Halogenwasserstoffsäuren, ferner Carbonsäuren, wie Niederalkancarbonsäuren. Als Basen kommen beispielsweise Alkalimetallhydroxide, -carbonate oder -acetate, Amine, wie Niederalkylamine oder basische Heterocyclen, wie Pyridin oder Chinolin, in Frage.

In entsprechenden Verbindungen der Formel (V), worin $X_{10}$ Hydroxy bedeutet, kann die Hydroxygruppe ebenso durch roten Phosphor und/oder Jodwasserstoffsäure unter Erwärmen beispielsweise auf etwa 100 bis etwa 250°C, vorteilhaft mit rotem Phosphor und Iodwasserstoffsäure, in Wasserstoff überführt werden.

Die reduktive Ueberführung von Hydroxy $X_{10}$, welches mit einer organischen Sulfonsäure verestert ist, wie p-Toluolsulfonyloxy, kann beispielsweise mit Hilfe eines üblichen Reduktionsmittels, wie einer Alkalimetall-Legierung, z.B. Natriumamalgam, in einem protischen Lösungsmittel oder mit einem gegebenenfalls komplexen Hydrid, wie Hydriden mit Elementen der 1. und/oder 3. Hauptgruppe, z.B. Lithiumborhydrid, durchgeführt werden.

Verbindungen der Formel (V), worin $X_9$ und $X_{10}$ gemeinsam Oxo oder Thioxo bedeuten, lassen sich zu solchen Verbindungen der Formel (I) reduzieren, worin $R_2$ für Wasserstoff steht, indem die Oxo- bzw. Thioxogruppe beispielsweise analog der Clemensen-Reduktion, z.B. mit einem Metall, wie gegebenenfalls amalgamiertem Zink, in einer Protonsäure, wie Mineralsäure, z.B. Chlorwasserstoffsäure, oder insbesondere nach Wolff-Kishner mit Hydrazin in einem (inerten hochsiedenden) Lösungsmittel, wie einem Alkohol, gegebenenfalls unter Druck, bei höherer Temperatur und in Gegenwart einer Base, wie eines Alkalimetallhydroxids, oder gemäss der Variante nach Huang-Minlon in einem hochsiedenden Lösungsmittel, wie einem entsprechenden Ethylenglykol, reduziert wird. Die Reduktion mit Hydrazin kann ebenfalls mit einer Base, wie einem Alkalimetallalkanolat, z.B. in Dimethylsulfoxid bei Raumtemperatur, durchgeführt werden.

Ebenfalls zu Verbindungen der Formel (I), worin $R_2$ für Wasserstoff steht, kann man gelangen, indem man z.B. Verbindungen der Formel (V), worin $X_9$ und $X_{10}$ für gegebenenfalls substituiertes Hydrazono, insbesondere p-Toluolsulfonylhydrazono, steht und $X_8$ die Bedeutung von $R_1$ hat, mit Hilfe eines geeigneten Reduktionsmittels, insbesondere

einem gegebenenfalls komplexen Hydrid, z.B. einem Hydrid aus Elementen der 1. und/oder 3. Hauptgruppe, z.B. Natriumborhydrid, reduziert.

Ausgangsverbindungen der Formel (V), worin $X_8$ die Bedeutung von $R_1$ hat und $X_9$ und $X_{10}$ gemeinsam die Gruppe = $R_2'$ oder eine tautomere Form davon bilden, können beispielsweise durch katalytische Hydrierung in solche Verbindungen der Formel (I) überführt werden, worin $R_2$ von Wasserstoff verschieden ist. Die Hydrierung kann in an sich bekannter Weise in der vorstehend beschriebenen Weise unter Verwendung der angegebenen Katalysatoren durchgeführt werden.

Grundsätzlich eignen sich z.B. die entsprechenden in Houben-Weyl, Bd. 4/1c (1980) und Bd. 4/1d (1981) beschriebenen Reduktionsmethoden.

Ausgangsstoffe der Formel (V), worin $X_8$ und $X_9$ jeweils Carboxy bedeuten und $X_{10}$ die Bedeutung von $R_2$ hat, sind nach an sich bekannten Verfahren herstellbar. Beispielsweise geht man von Verbindungen der Formel

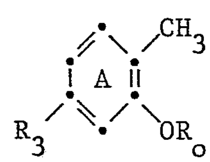

(Va)

oder deren Salzen aus und setzt diese mit einem Halogenierungsmittel, z.B. mit N-Bromsuccinimid in Gegenwart eines Radikalbildners, wie Benzoylperoxid, bei erhöhter Temperatur, um. In den so erhältlichen Verbindungen der Formel

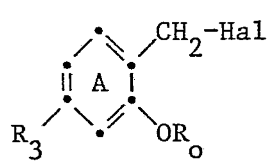

(Vb)

oder deren Salzen, worin Hal Halogen, insbesondere Brom- oder Chlor, bedeutet, wird das Halogenatom durch Umsetzung mit einem Alkalimetallcyanid, wie Natriumcyanid, durch die Cyanogruppe substituiert. Anschliessend folgt die Umsetzung mit einem Dialkylcarbonat, z.B. Diethylcarbonat, in Gegenwart einer Base, wie eines Alkalimetalles, z.B. Natrium, zu Verbindungen der Formel

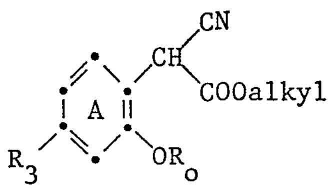

(Vc),

worin Alkyl ein den Dialkylcarbonat entsprechender Alkylrest darstellt, oder deren Salzen. Gewünschtenfalls wird nun der von Wasserstoff verschiedene Rest $R_2$ durch Umsetzung mit Verbindungen der Formel $R_2$-Hal (Vd) in Gegenwart einer Base, wie Alkalimetallalkoholat, z.B. Natriummethylat, eingeführt. Die anschliessende Hydrolyse der Cyanogruppe sowie der Alkoxycarbonylgruppe führt zu den gewünschten Verbindungen der Formel (V).

Zu Ausgangsstoffen der Formel (V), worin $X_8$ die Bedeutung von $R_1$ und $X_9$ die von $R_2$ hat, und $X_{10}$ Hydroxy oder funktionell abgewandeltes Hydroxy darstellt, gelangt man beispielsweise, indem man Verbindungen der Formel

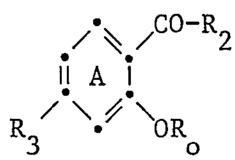

(Ve)

oder deren Salzen mit Cyaniden, wie Natriumcyanid, in Gegenwart einer Protonsäure, wie Chlorwasserstoffsäure, zu Cyanhydrinen der Formel

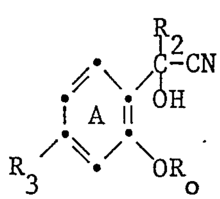

(Vf)

oder deren Salzen umsetzt. Im nächsten Reaktionsschritt folgt die Solvolyse der Cyanogruppe zu $R_1$ und, wenn erwünscht, die Verestertung oder Veretherung der Hydroxygruppe oder $R_o$.

Entsprechende Ausgangsstoffe der Formel (V), worin $X_{10}$ sekundäres Amino darstellt, erhält man beispielsweise durch Umsetzung von Verbindungen der Formel

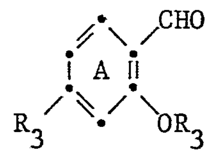

(Vg)

oder deren Salzen mit einer Lösung von Ammoniumchlorid und Natriumcyanid oder mit Natriumcyanid und Ammoniumcarbonat mit sich anschliessender Hydrolyse des gebildeten Hydantoins mit Hilfe eines Alkalimetallhydroxids und gewünschtenfalls sich anschliessender Einführung des von Wasserstoff verschiedenen Restes $R_2$ durch Umsetzung mit Verbindungen der Formel (Vd) in Gegenwart von Basen, z.B. Natriumethylat in resultierenden Verbindungen der Formel

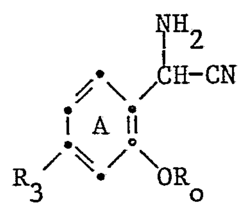

(Vh)

oder deren Salzen. Im nächsten Reaktionsschritt kann die Ueberführung der Aminogruppen in eine sekundäre Aminogruppe erfolgen. So gelangt man z.B. durch Reaktion mit Ameisensäure/Formaldehyd zu einer Dimethylaminogruppe. Schliesslich wird die Cyanogruppe durch Solvolyse in bekannter Weise in den Rest $R_1$ überführt.

Zur Herstellung von Ausgangsstoffen der Formel (V), worin $X_8$ die Bedeutung von $R_1$ hat und $X_9$ und $X_{10}$ gemeinsam die Gruppe = $R_2'$ oder eine tautomere Form davon, bilden, geht man von Verbindungen der Formel (Vf) oder deren Salzen aus. Diese werden beispielsweise mit Hilfe einer Säure, wie Mineralsäure, z.B. Schwefelsäure oder Phosphorsäure bzw. Polyphosporsäure, deren Salze, wie Kaliumhydrogensulfat, oder deren Anhydriden, z.B. Thionylchlorid, zu entsprechenden Verbindungen der Formel (V) dehydratisiert und die Cyanogruppe solvolytisch in $R_1$ überführt.

Eine andere Herstellungsweise für Verbindungen der Formel (I), worin $R_1$ Carboxy oder verestertes Carboxy bedeutet, besteht darin, dass man in Verbindungen der Formel

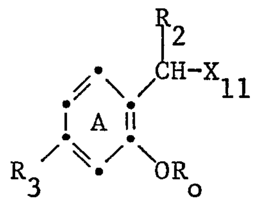

(VI)

oder deren Salzen, worin $X_{11}$ ein oxidativ in $R_1$ überführbarer Rest bedeutet, $X_{11}$ oxidativ in $R_1$ überführt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/oder gewünschtenfalls ein verfahrensgemäss erhältliches Isomerengemisch in seine Komponenten auftrennt.

Ein oxidativ in $R_1$ überführbarer Rest $X_{11}$ ist beispielsweise Hydroxymethyl, mit einer Carbonsäure, wie gegebenenfalls substituierten Niederalkancarbonsäure, z.B. Essigsäure, verestertes Hydroxymethyl, mit einem Alkohol, wie Niederalkanol, z.B. Methanol oder Ethanol, verethertes Hydroxymethyl, Formyl, hydratisiertes oder acetalisiertes Formyl, oder Gruppen der Formeln $-CH=CH-X_{14}$, $-CH=C(Ar)_2$, $-CH(OH)-CH(OH)-X_{14}$, $-CH(OH)-CO-X_{14}$, $-CH(OH)-CO-O-X_{14}$, $-CO-CO-X_{14}$, $-CH(NH_2)-CO-X_{14}$, $-CO-COOH$ bedeutet, in denen $X_{14}$ für Wasserstoff, einen aliphatischen Rest, z.B. gegebenenfalls substituierten Niederalkylrest, oder einen Arylrest steht und unter Ar ein Arylrest und unter diesem z.B. ein gegebenenfalls substituierter Phenylrest zu verstehen ist.

Die Oxidation erfolgt in an sich bekannter Weise unter Verwendung geeigneter Oxidationsmittel in einem inerten Lösungs- oder Verdünnungsmittel und erforderlichenfalls unter Kühlen oder Erwärmen, z.B. von etwa 0° bis etwa 150°C.

Als Oxidationsmittel eigenen sich beispielsweise Sauerstoff, Ozon, Peroxide, wie Wasserstoffperoxid, oder Peroxide von organischen Carbonsäuren, wie Trifluorperessigsäure oder m-Chlorperbenzoesäure, oxidierende Verbindungen von Uebergansmetallen, insbesondere solche mit Elementen der I., VI., VII. oder VIII. Nebengruppe des Periodensystems, wie Kupferverbindungen, z.B. Kupferchromit, wie Silberverbindungen, z.B. Silber(I)oxid oder Silberpicolinat, wie Chromverbindungen, z.B. Chromylchlorid, Chromtrioxid, Alkalimetallchromate oder -dichromate, wie Kaliumdichromat, wie Manganverbindungen, z.B. Mangandioxid oder Alkalimetallpermanganate, oder wie Halogen-Sauerstoff-Verbindungen, z.B. Alkalimetalliodate oder -periodate, ferner wie Halogen, z.B. Brom oder Chlor, Halogen-Sauerstoff-Verbindungen, z.B. Alkalimetallhypochlorite, -iodate, -periodate oder Periodsäure, Stickstoffsäuren oder durch Anhydride, z.B. Salpetersäure oder entsprechende Anhydride konzentrierte Schwefelsäure. Erforderlichenfalls können auch Gemische von Oxidationsmitteln eingesetzt werden.

Dabei wird häufig in Gegenwart von Basen, wie Alkalimetallhydroxiden oder -carbonaten, z.B. Natriumhydroxid oder -carbonat, oder wie Aminen, z.B. cyclische Amine, z.B. Pyridin, oder Niederalkylamine, z.B. Triethylamin, oder von Protonsäuren, wie Mineralsäuren, z.B. Schwefelsäure oder Halogenwasserstoffsäure, oder organischen Carbonsäuren, wie Niederalkancarbonsäuren, z.B. Essigsäure, und gegebenenfalls unter Kühlen oder Erwärmen gearbeitet.

Als Lösungs- bzw. Verdünnungsmittel kommen beispielsweise Wasser, Ether, wie Dioxan oder Ethylenglykoldiethylether, Ketone, wie Aceton, Alkohole, wie die Niederalkanole Methanol oder Ethanol, Amide, wie Dimethylformamid, Carbonsäuren, wie die Niederalkancarbonsäure Essigsäure, oder Gemische derselben in Frage.

Hydroxymethyl bzw. mit einer Carbonsäure verestertes Hydroxymethyl $X_{11}$ wird beispielsweise unter Erwärmen mit Hilfe von Kaliumdichromat in Schwefelsäure zu Carboxy oxidiert, wobei die Oxidation über die Formylstufe verläuft. Formyl, hydratisiertes oder acetalisiertes wird beispielsweise mit Hilfe von Silber(I)oxid in Natronlauge oder von Kaliumpermanganat in Sodalösung unter Erwärmen in Carboxy überführt, während die Gruppe $X_{11}$: $-CH=CH-X_{14}$ beispielsweise mittels Ozon und Wasserstoffperoxid über die Formylstufe zu Carboxy oxidiert wird. Verethertes Hydroxymethyl lässt sich beispielsweise mit Kaliumpermanganat in wässrigem Pyridin bei Raumtemperatur in verestertes Carboxy überführen.

Die Formylgruppe $X_{11}$ kann vorteilhaft im Verlauf von Oxidationsreaktionen in situ gebildet oder aus einer funktionell abgewandelten Form in Freiheit gesetzt werden. Die in-situ-Bildung von Formyl erfolgt insbesondere aus solchen Resten $X_{11}$, die in erster Linie Hydroxymethyl oder Gruppen der Formeln $-CH=CH-X_{14}$, $-CH(OH)-CH(OH)-X_{14}$ oder $-CH(OH)-CO-X_{14}$, ferner $-CH=C(Ar)_2$, $-CO-CO-X_{14}$, $-CH(OH)-CO-OX_{14}$ oder $-CH(NH_2)-CO-X_{14}$ bedeuten. Die Freisetzung der Formylgruppe $X_{11}$ erfolgt z.B. aus einem ihrer Acetale oder Imine sowie aus sonstigen Formylmaskierungsgruppen. Acetalisiertes Formyl bedeutet z.B. mit Niederalkanolen oder einem Niederalkandiol acetalisiertes Formyl, wie Diniederalkoxy-, z.B. Dimethoxy- oder Diethoxymethyl, oder Niederalkylendioxy-, z.B. Ethylen- oder Trimethylendioxy-methyl. Ferner kann Formyl aus den entsprechenden Thioacetalen freigesetzt werden. Imine sind z.B. gegebenenfalls substituierte N-Benzylimine oder N-(2-Benzothiazolyl)-imin.

Die Oxidation der übrigen Reste $X_{11}$ zu Carboxy können vorteilhaft in situ, häufig über die Formylstufe, erfolgen und werden z.B. folgendermassen durchgeführt: $X_{11}$ $-CH(OH)-COO-X_{14}$, $-CH=CH-X_{14}$ und $-CH(OH)-CH(OH)-X_{14}$ z.B. mit Hilfe von Natriumperiodat in Gegenwart katalytischer Mengen Kaliumpermanganat, $X_{11}$ Hydroxymethyl, $-CH(NH_2)-CO-X_{14}$, $-CH(OH)-CO-X_{14}$ und $-CO-CO-X_{14}$ z.B. mit Hilfe von sodaalkalischer Permanganatlösung, schwefelsaurer Kaliumdichromatlösung oder konzentrierter Salpetersäure, $X_{11}$ $-CH=C(Ar)_2$, wobei Ar insbesondere jeweils Phenyl bedeutet, analog Barbier-Wieland, z.B. mit Chromtrioxid in Eisessig, und $X_{11}$ $-CO-COOH$ z.B. durch Behandeln mit konzentrierter Schwefelsäure oder mit Wasserstoffperoxid in verdünnter Natronlauge (Decarbonylierung).

Ausgangsstoffe der Formel (VI), worin $X_{11}$ Hydroxymethyl, verestertes oder veräthertes Hydroxymethyl bedeutet, sind erhältlich, indem man beipielsweise Verbindungen der Formel

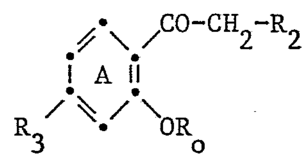

(VIa)

mit einem Gemisch von Trimethylsulfoniummethylsulfat und Natriummethylat beispielsweise bei Zimmertemperatur in Acetonitril umsetzt. In den resultierenden Verbindungen der Formel

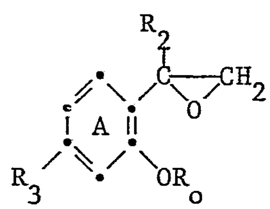

(VIb)

wird im folgenden Reaktionsschritt der Oxiran-Ring, beispielsweise in Gegenwart einer Lewissäure, wie Aluminiumchlorid, zur Verbindung der Formel (VI) geöffnet, worin $X_{11}$ Formyl darstellt. In fakultativen Zusatzreaktionen kann das Formyl gewünschtenfalls in an sich bekannter Weise acetalisiert oder zu Hydroxymethyl reduziert werden. Die Hydroxymethylgruppe wiederum kann, wenn erwünscht, verestert oder verethert werden.

Entsprechende Ausgangsstoffe der Formel (VI) können ebenfalls erhalten werden, indem man beispielsweise Verbindungen der Formel (VIa) mit Halogenacetonitril, z.B. Chloracetonitril, bei tiefen Temperaturen und einer Base, wie eines Alkalimetallalkanolats, z.B. Natriummethylat, behandelt und das resultierende Glycidonitril z.B. mit Hilfe einer Base, wie eines Alkalimetallhydroxides, z.B. Natronlauge, unter Erwärmen hydrolysiert. Anschliessend werden die so erhaltenen Verbindungen der Formel

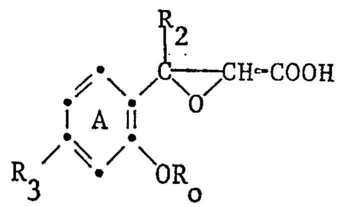

(VIc)

unter Erhitzen, z.B. bei Rückflusstemperatur von Toluol, decarboxyliert, wobei Verbindungen der Formel (VI) anfallen, worin $X_{11}$ Formyl bedeutet. Mit Hilfe von fakultativen Zusatzmassnahmen kann das Formyl nach an sich bekannter Weise acetalisiert bzw. zu Hydroxylmethyl reduziert werden. Letzteres wiederum kann, wenn erwünscht, verestert oder verethert werden.

Ausgangsstoffe der Formel (VI), worin $X_{11}$ eine Gruppe der Formel $-CH=CH-X_{14}$ darstellt, können hergestellt werden, indem man z.B. Verbindungen der Formel

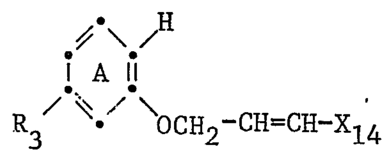

(VId)

auf hohe Temperaturen, z.B. auf 250°C, erhitzt und anschliessend in so erhältlichen Verbindungen der Formel

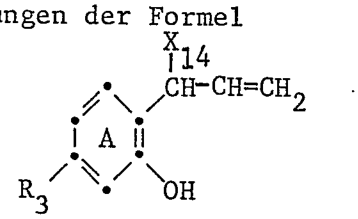

(VIe)

die Hydroxygruppe gewünschtenfalls in $OR_o$ überführt, z.B. durch Veresterung, z.B. Acetylierung mit Acetanhydrid/Pyridin, gewünschtenfalls den Rest $R_2$ durch Umsetzung mit einer Verbindung der Formel $R_2$-H in Gegenwart einer Base, z.B. Natriumamid in flüssigen Ammoniak, einführt. Die anschliessende Oxidation von so erhältlichen Verbindungen der Formel

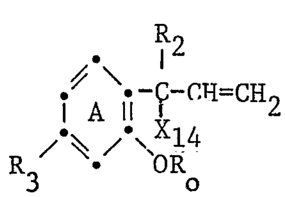

(VIf)

mit Ozon und einem Peroxid, z.B. 30 %igem Wasserstoffperoxid, bei Raumtemperatur führt zu Verbindungen der Formel (I), worin $R_1$ Carboxy bedeutet.

Zur Herstellung von Verbindungen der Formel VI, worin $X_{11}$ einen oxidativ in $R_1$ überführbaren Rest bedeutet, geht man ebenso beispielsweise von einem der Formel I entsprechenden Salicylsäurederivat aus und reduziert die Carboxygruppe zur Hydroxymethylgruppe, wobei als Reduktionsmittel z.B. ein komplexes Hydrid, wie Lithiumaluminiumhydrid, verwendet wird. Nach Substitution der Hydroxygruppe durch ein Halogenatom, z.B. durch Behandeln mit einem Halogenierungsreagens, wie Thionylchlorid, wird die resultierende Halogenmethylverbindung z.B. mit einem Halogenid der Formel Hal-$X_{11}$ in Gegenwart von Magnesium und Kupfer(I)iodid umgesetzt. Bevorzugte Verbindungen der Formel Hal-$X_{11}$ sind beispielsweise solche, worin $X_{11}$ für die Gruppe der Formel $-CH=CH-X_{14}$ oder $-CH=C(Ar)_2$ steht. Aus den so erhältlichen Verbindungen der Formel VI, worin $X_{11}$ für $-CH=CH-X_{14}$ steht, gelangt man z.B. durch Ozonolyse und Spaltung des Ozonids durch Zink/Eisessig zu Formyl $X_{11}$ bzw. durch Hydroxylierung der Doppelbindung, z.B. mit Osmiumtetroxid, durch partielle bzw. vollständige Oxidation der Hydroxyverbindungen zu entsprechenden Oxoderivaten oder zu solchen Verbindungen, worin $X_{11}$ für folgende Gruppen steht: $-CH(OH)-CH(OH)-X_{14}$, $-CH(OH)-CO-X_{14}$ oder $-CO-CO-X_{14}$.

Die entsprechende α-Ketocarbonsäure der Formel VI, d.h. $X_{11}$ stellt die Gruppe der Formel -CO-COOH dar, ist zugänglich indem man z.B. ein der Formel (I) entsprechendes Salicylsäurederivat mit Phosgen behandelt und das resultierende Säurechlorid z.B. mit Kupfer-(I)- oder Natriumcyanid umsetzt und die Cyanogruppe zur Carboxygruppe hydrolysiert, nach deren Veresterung sich auch solche Verbindungen der Formel VI herstellen lassen, worin $X_{11}$ für die Gruppe $-CO-CO-X_{14}$ steht.

Eine weitere Arbeitsweise zur Herstellung von Verbindungen der Formel (I) besteht darin, dass man in einer Verbindung der Formel

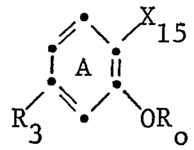

(VIII)

oder ein Salz davon, worin $X_{15}$ ein in die Gruppe der Formel $-CH(R_2)-R_1$ überführbarer Rest bedeutet, $X_{15}$ durch Umlagerung in die Gruppe der Formel $-CH(R_2)-R_1$ überführt und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/oder gewünschtenfalls ein verfahrensgemäss erhältliches Isomerengemisch in seine Komponenten auftrennt.

Verbindungen der Formel (VIII), worin $X_{15}$ die Gruppe der Formel $-CH(R_2)-C(=\bar{N}-\overset{\oplus}{N}\equiv N|)-X_{16}$ bzw. $-CH(R_2)-C(=N-OH)-X_{16}$ und $X_{16}$ einen gegebenenfalls substituierten aliphatischen Rest bedeutet, können gemäss der Schmidt- bzw. der Beckmann-Umlagerung in N-monosubstituierte Carbamoyl($R_1$)-Verbindungen der Formel (I) umgelagert werden. Die Schmidt- bzw. Beckmann-Umlagerung erfolgt in an sich bekannter Weise. So behandelt man z.B. die betreffenden Azide bzw. Oxime mit sauren Katalysatoren, wie starken Protonsäuren, z.B. Schwefelsäure, anorganischen Säurehalogeniden, z.B. Phosphor(V)chlorid, oder Sulfochloriden, z.B. Benzolsulfochlorid, gegebenenfalls in einem inerten Lösungsmittel, wie einem halogenierten Kohlenwasserstoff, z.B. dem Halogenniederalkan Chloroform, oder einem Aromaten, z.B. Benzol, in einem Temperaturbereich von etwa -30 bis etwa 150°C.

Verbindungen der Formel (VIII), worin $X_{15}$ für die Gruppe der Formel $-CH(R_2)-CO-CH_2-N_2$ steht, lassen sich nach analogen Methoden gemäss der Wolff-Umlagerung zu solchen Verbindungen der Formel (II) umlagern, worin $R_1$ gegebenenfalls verestertes oder amidiertes Carboxy darstellt. So führt man die Umsetzung z.B. unter Erhitzen und/oder

Bestrahlen mit energiereichem Licht, z.B. UV-Licht, und/oder in Anwesenheit eines Katalysators, z.B. eines Edelmetalls bzw. Edelmetalloxids, wie Kupfer, Silber oder Silberoxid, in einem inerten Lösungsmittel, wie einem Ether, z.B. Dioxan oder Tetrahydrofuran, durch, wobei vorteilhaft in einem Temperaturbereich von etwa 0° bis etwa 150°C gearbeitet wird. Durch Zusatz von Wasser, Alkohol, Ammoniak oder Amin kann die Reaktion zur Bildung von freier Carbonsäure, veresterter oder amidierter Carbonsäure $R_1$ gelenkt werden.

Verbindungen der Formel (VIII), worin $X_{15}$ für die Gruppe der Formel $-CO-CH_2-Hal$ und Hal für Halogen, wie Chlor, Brom, ferner Iod steht, lassen sich in an sich bekannter Weise analog der Faworskij-Umlagerung in solche Verbindungen der Formel (I) überführen, worin $R_1$ Carboxy bedeutet und $R_2$ Wasserstoff ist. Die entsprechende Umlägerung lässt sich z.B. durch Erhitzen mit starken Basen, wie Alkalimetallhydroxiden, oder durch Behandeln mit Ag(I)-Verbindungen, wie Silber(I)oxid bzw. Silber(I)nitrat unter Erwärmen in einem Lösungsmittel, wie Wasser und/oder Niederalkanol, durchführen.

Die oxidative Umlagerung von Verbindungen der Formel (VIII), worin $X_{15}$ für die Gruppe der Formel $-CO-CH_2-R_2$ steht, erfolgt z.B. mit Hilfe des Oxidationsmittels Thallium(III)nitrat, wobei vorzugsweise in einem Alkohol, wie Niederalkanol, gegebenenfalls in Gegenwart von Spuren starker Protonsäure, wie Perchlorsäure, oder von Trimethylorthoformat gearbeitet wird. Ferner kann ein inertes Lösungsmittel, wie ein gegebenenfalls halogenierter Kohlenwasserstoff, z.B. Hexan- oder Chloroform, oder ein Ether, z.B. Dioxan verwendet werden. Das Oxidationsmittel kann auch auf einem geeigneten Trägermaterial [Lit. J. Am. Chem. Soc. 98, 6750 (1976)] aufgezogen sein.

Führt man die Umsetzung in einem Niederalkanol durch, werden Verbindungen der Formel (I) erhalten, wobei $R_1$ Niederalkoxycarbonyl darstellt.

Die oxidative Umlagerung von Verbindungen der Formel (VIII), worin $X_{15}$ für die Gruppe der Formel $-CO-CH_2-R_2$ steht und $R_2$ Wasserstoff dar-

stellt, analog der Willgerodt-Kindler-Reaktion, wird mit wässrigen Ammoniumpolysulfid, im allgemeinen unter Druck, oder mit Schwefel und einem primären oder tertiären Amin in einem inerten Lösungsmittel und gegebenenfalls unter Erwärmen durchgeführt. Dabei werden Verbindungen der Formel (I) erhalten, worin $R_1$ amidiertes Carboxy oder ein entsprechendes Thiocarbamoyl bzw. Ammoniumcarboxylat bedeutet und $R_2$ Wasserstoff ist. Als Lösungsmittel eignen sich z.B. Ether, wie Dioxan oder Tetrahydrofuran, oder Niederalkanole, wie Ethanol. Vorteilhaft führt man die Umsetzung durch längeres Kochen unter Rückfluss durch.

Die Ausgangsstoffe der Formel (VIII) sind bekannt oder werden nach analogen Verfahren hergestellt.

Ein allgemeines Verfahren zur Herstellung von Verbindungen der Formel (VIII) besteht z.B. darin, dass man eine Verbindung der Formel

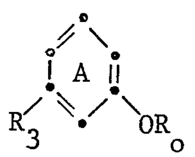

(VIIIa)

oder ein Salz davon mit einer Verbindung der Formel Hal-$X_{15}$ umsetzt, worin Hal für Halogen, wie Chlor oder Brom, steht. Die Umsetzung erfolgt z.B. in Gegenwart einer starken Säure, wie Polyphosphorsäure, oder insbesondere in Gegenwart einer Lewissäure, wie Aluminiumchlorid.

Eine weitere Verfahrensvariante zur Herstellung von Verbindungen der Formel (I), oder deren Salzen oder Isomeren besteht darin, dass in einer Verbindung der Formel

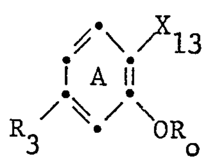

(VII),

worin $X_{13}$ einen in die Gruppe der Formel $-CH(R_2)-R_1$ (VIIa) überführbaren Rest bedeutet, oder einem Salz oder Isomeren davon der Rest $X_{13}$ in die Gruppe der Formel (VIIa) überführt wird und/oder, wenn erwünscht,

ein verfahrensgemäss erhältliches Salz in die freie Verbindung oder in ein anderes Salz überführt wird, eine verfahrensgemäss erhältliche freie Verbindung in ein Salz oder in eine andere freie Verbindung überführt und/oder gewünschtenfalls ein verfahrensgemäss erhältliches Isomerengemisch in seine Komponenten aufgetrennt wird.

Ein in die Gruppe der Formel (VIIa) überführbarer Rest $X_{13}$ bedeutet beispielsweise eine Gruppe der Formel -Mg-Hal bzw. $-CH(R_2)-$ Mg-Hal, wobei Hal jeweils für Halogen, insbesondere Chlor oder Brom, steht.

In eine Verbindung der Formel (VII), worin $X_{13}$ die Gruppe -Mg-Hal darstellt, wird die Gruppe der Formel (VIIa) in an sich bekannter Weise eingeführt. Beispielsweise setzt man eine entsprechende Verbindung der Formel (VII) mit einer Verbindung der Formel 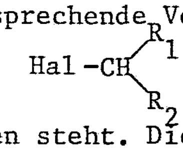 (VIIb), oder einem Salz davon um, wobei Hal für Halogen steht. Die Umsetzung erfolgt erforderlichenfalls unter Kühlen in einem inerten Lösungs- oder Verdünnungsmittel, wie einem Ether, z.B. Diniederalkylether oder cyclischen Ether, gegebenenfalls unter Schutzgas, wie Stickstoff, vorzugsweise bei Temperaturen zwischen etwa -80° und etwa der Siedetemperatur des Lösungsmittels.

Entsprechende Ausgangsstoffe der Formel (VII), worin $X_{13}$ die Gruppe -Mg-Hal bedeutet, oder deren Salze oder Isomeren werden nach an sich bekannten Methoden hergestellt, beispeilsweise durch Umsetzung von Verbindungen der Formel

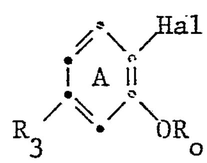

(VIId)

oder deren Salzen mit Magnesium in einem Ether, wie Tetrahydrofuran. Die entsprechenden Verbindungen der Formel (VIId) sind bekannt oder sind in analoger Weise zugänglich.

In Verbindungen der Formel (VII), worin $X_{13}$ die Gruppe der Formel $-CH(R_2)-Mg-Hal$ bedeutet, oder deren Salzen oder Isomeren kann die Gruppe der Formel (VIIa), worin $R_1$ Carboxy bedeutet, durch Behandeln entsprechender Verbindungen der Formel (VII) mit Kohlendioxid einführen. Dabei wird erforderlichenfalls unter Kühlen in einem inerten Lösungsmittel, wie einem Ether, z.B. ein Diniederalkylether oder ein cyclischer Ether, und gegebenenfalls unter Schutzgas, z.B. Stickstoff, gearbeitet.

Entsprechende Ausgangsstoffe der Formel (VII), worin $X_{13}$ die Gruppe der Formel $-CH(R_2)-Mg-Hal$ bedeutet, können beispielsweise erhalten werden, indem man in Verbindungen der Formel

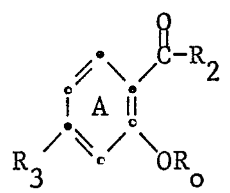

(VIIe)

oder einem Salz davon, die Oxogruppe mit einem Reduktionsmittel, wie einem gegebenenfalls komplexen Hydrid, z.B. Lithiumalluminiumhydrid oder Natriumborhydrid, unter leichtem Erwärmen zur Hydroxygruppe reduziert. Diese wird anschliessend durch Halogen substituiert, beispielsweise durch Behandeln mit einem Phosphorhalogenid, z.B. Phosphorbromid oder -chlorid, erforderlichenfalls unter Kühlen, z.B. auf 0°C. Eine so erhältliche Verbindung der Formel

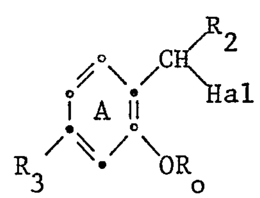

(VIIf)

oder deren Salzen setzt man nunmehr mit Magnesium zur entsprechenden Verbindung der Formel (VII) um, wobei in einem inerten Lösungsmittel, wie einem Ether, z.B. Dioxan, gearbeitet wird.

Eine erfindungsgemäss erhältliche Verbindung der Formel (I) kann in an sich bekannter Weise in eine andere Verbindung der Formel (I) umgewandelt werden.

Falls der Ring A durch Niederalkylthio substituiert ist, kann man dieses auf übliche Weise zu entsprechendem Niederalkansulfinyl bzw. -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Uebergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des Niederalkylthio zum Niederalkansulfonyl. Jedoch setzt man hierbei üblicherweise das Oxidationsmittel im Ueberschuss ein.

Ist der Ring A durch Niederalkansulfinyl bzw. -sulfonyl substituiert, lässt sich dieser nach an sich bekannten Methoden zu entsprechendem Niederalkylthio, von Niederalkansulfonylderivaten ausgehend, auch zu Niederalkan-sulfinyl reduzieren. Als Reduktionsmittel eignen sich beispielsweise katalytisch aktivierter Wasserstoff, wobei Edelmetalle bzw. Oxide, wie Palladium, Platin oder Rhodium bzw. deren Oxide, verwendet werden, gegebenenfalls auf geeignetem Trägermaterial, wie Aktivkohle oder Bariumsulfat, aufgezogen. Weiterhin kommen reduzierende Metallkationen, wie Zinn II-, Blei II-, Kupfer I-, Mangan II-, Titan II-, Vanadium II-, Molybdän III- oder Wolfram III-Verbindungen,

Halogenwasserstoffe, wie Chlor-, Brom- oder Iodwasserstoff, Hydride, wie komplexe Metallhydride, z.B. Lithiumaluminium-, Natriumbor-, Tributylzinnhydrid, Phosphorverbindungen, wie Phorphorhalogenide, z.B. Phosphortrichlorid, -bromid, Phosphorpentachlorid oder Phosphoroxichlorid, Phosphine, wie Triphenylphosphin, oder Phosphorpentasulfid-Pyridin, oder Schwefelverbindungen, wie Mercaptane, Thiosäuren, wie Thiophosphorsäuren oder Dithiocarbonsäuren, Dithionit oder Schwefel-Sauerstoff-Komplexe, wie Iod-Pyridin-Schwefeldioxidkomplex, in Frage.

Weist der aromatische Ring als Substituenten ein Wasserstoffatom auf, so kann dieses mit Hilfe eines Halogenierungsmittels in üblicher Weise durch ein Halogenatom ersetzt werden.

So erfolgt die Substitution von Wasserstoff durch Brom z.B. durch Bromierung mit Brom analog "Methoden der Organischen Chemie", Houben-Weyl (4. Edition), Vol, 5/4, S. 233-249, in einem inerten Lösungsmittel. Weiterhin kann mit Hilfe folgender Bromierungsmittel bromiert werden: Hypobromsäure, Acylhypobromite oder andere organische Bromverbindungen, z.B. N-Bromsuccinimid, N-Bromacetamid, N-Bromphthalimid, Pyridiniumperbromid, Dioxandibromid, 1,3-Dibrom-5,5-dimethylhydantoin, 2,4,4,6-Tetrabrom-2,5-cyclohexadien-1-on.

Die entsprechende Chlorierung kann z.B. wie in Houben-Weyl (4. Edition), Vol. 5/3, S. 651-673, beschrieben durchgeführt werden, vorteilhaft mit elementaren Chlor, z.B. in einem halogenierten Kohlenwasserstoff, wie Chloroform, und unter Kühlen, z.B. auf etwa -10° bis etwa +10°C.

Der Ersatz von Wasserstoff durch Iod kann z.B. mit elementarem Iod in Gegenwart von Quecksilberoxid oder Salpetersäure erfolgen. Anstelle von elementem Iod lässt sich beispielsweise ein Alkalimetalliodid in Gegenwart eines Thallium(III)difluoracetat gemäss Tetrahedron Letters (1969), S. 2427 als Iodierungsmittel verwenden.

Ferner kann der Benzoteil des Ringsystems und/oder ein weiterer aromatischer Ring beispielsweise mit einem Niederalkanol bzw. einem Niederalkylhalogenid oder einem Phosphorsäureniederalkylester in Gegenwart von Lewis-Säuren alkyliert werden (Friedel-Crafts-Alkylierung). In einer Verbindung der Formel (I), worin ein aromatischer Ring Brom enthält, kann beispielsweise das Brom durch Umsetzung mit einem Niederalkylbromid in Gegenwart eines Alkalimetalls durch Niederalkyl ersetzt werden.

Enthält ein aromatischer Ring als Substituenten ein Wasserstoffatom, so kann dieses in an sich bekannter Weise durch eine Acylgruppe ausgetauscht werden. So kann beispielsweise die Einfügung der Acylgruppe analog der Friedel-Crafts-Acylierung (cf. G.A. Olah, Friedel-Crafts and Related Reactions, Vol. I, Interscience, New York, 1963-1965) durchgeführt werden, z.B. durch Umsetzung eines reaktiven funktionellen Acylderivates, wie eines Halogenids oder Anhydrids, einer organischen Carbonsäure in Gegenwart einer Lewis-Säure, wie Aluminium-, Antimon-(III)- oder -(V)-, Eisen(III)-, Zink(II)-chlorid oder Brotrifluorid.

Enthält der aromatische Ring als Substituenten Hydroxy, so lässt sich dieses nach an sich bekannter Weise verethern. Die Umsetzung mit einer Alkoholkomponente, z.B. mit einem Niederalkanol, wie Ethanol, in Gegenwart von Säuren, z.B. Mineralsäure, wie Schwefelsäure, oder von Dehydratisierungsmitteln, wie Dicyclohexylcarbodiimid, führt zu Niederalkoxy. Umgekehrt kann man Ether in Phenole spalten, indem man die Etherspaltung mittels Säuren, wie Mineralsäuren, z.B. Halogenwasserstoffsäure, wie Bromwasserstoffsäure, oder wie Lewissäuren, z.B. Halogeniden von Elementen der 3. Hauptgruppe, wie Bortribromid, oder mittels Pyridin-hydrochlorid oder Thiophenol durchführt.

Weiter lässt sich Hydroxy in Niederalkanoyloxy umwandeln, beispielsweise durch Umsetzung mit einer gewünschten Niederalkancarbonsäure, wie Essigsäure, oder einem reaktionsfähigen Derivat davon, beispielsweise in Gegenwart einer Säure, wie eine Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels, wie Dicyclohexylcarbodiimid. Umgekehrt kann verestertes Hydroxy, z.B. durch Basen-Katalyse, zu Hydroxy solvolysiert werden.

Freie, veresterte und amidierte Carboxygruppen $R_1$ kann man ineinander, beispielsweise eine freie Carboxygruppe in üblicher Weise in eine veresterte Carboxylgruppe $R_1$ überführen, vorzugsweise durch Umsetzung mit einem entsprechenden Alkohol oder einem reaktionsfähigen Derivat, wie einem Carbon-, Phosphorig-, Schweflig- oder Kohlensäureester, z.B. einem Niederalkancarbonsäureester, Triniederalkylphosphit, Diniederalkylsulfit oder dem Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfonsäureester, des entsprechenden Alkohols oder einem davon abgeleiteten Olefin.

Die Umsetzung mit dem entsprechenden Alkohol erfolgt vorteilhaft in Gegenwart eines sauren Katalysators, wie einer Protonensäure, z.B. von Chlor- oder Bromwasserstoff-, Schwefel-, Phosphor-, Bor-, Benzolsulfon- und/oder Toluolsulfonsäure, oder einer Lewissäure, z.B. von Bortrifluorid-Etherat, in einem inerten Lösungsmittel, insbesondere einem Ueberschuss des eingesetzten Alkohols und erforderlichenfalls in Gegenwart eines wasserbindenden Mittels und/oder unter destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester, bei-

spielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols, erforderlichenfalls unter, z.B. azeotroper, Abdestillation des Reaktionswassers. Ausgehend von einem Mineralsäure- oder Sulfonsäureester setzt man die zu veresternde Säure vorteilhaft in Form eines Salzes, z.B. des Natrium-, Kalium- oder Calciumhydroxids oder -carbonats, in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triethylamin oder Pyridin, erforderlichenfalls in einem inerten Lösungsmittel, wie einer der vorstehenden tertiären Stickstoffbasen oder eines polaren Lösungsmittels, z.B. in Dimethylformamid und/oder bei erhöhter Temperatur um.

Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder vor allem eines basischen Katalysators, z.B. von Natrium- oder Kaliumhydroxid, vorteilhaft in einem inerten Lösungsmittel, wie einem Ether, z.B. in Diethylether oder Tetrahydrofuran, erfolgen.

Eine freie Carboxylgruppe $R_1$ kann ferner durch Umsetzung mit Ammoniak oder einem mindestens ein Wasserstoffatom aufweisenden Amin in üblicher Weise unter Dehydratisierung des intermediär gebildeten Ammoniumsalzes, z.B. durch azeotrope Destillation mit Benzol oder Toluol oder trockenes Erhitzen, in eine amidierte Carboxylgruppe $R_1$ überführt werden.

Die vorstehend beschriebenen Umwandlungen freier in veresterte oder amidierte Carboxylgruppen $R_1$ können aber auch so durchgeführt werden, dass man eine Verbindung der Formel I, worin $R_1$ Carboxyl ist, zunächst in üblicher Weise in ein reaktionsfähiges Derivat, beispielsweise

mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol oder Cyanmethylalkohol, oder ein reaktives Amid, z.B. das von Imidazol oder 3,5-Dimethylpyrazol abgeleitete Amid, überführt und das erhaltene reaktionsfähige Derivat dann in üblicher Weise, z.B. wie nachstehend für die Umesterung, Umamidierung bzw. gegenseitige Umwandlung veresterter und amidierter Carboxylgruppen $R_1$ beschrieben, mit einem entsprechenden Alkohol, Ammoniak oder dem entsprechenden mindestens ein Wasserstoffatom aufweisenden Amin zu der gewünschten Gruppe $R_1$ umsetzt.

Ferner kann eine veresterte Carboxylgruppe $R_1$ in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, zur freien Carboxylgruppe $R_1$ oder z.B. durch Umsetzung mit Ammoniak oder dem entsprechenden, mindestens ein Wasserstoffatom aufweisenden Amin in eine amidierte Carboxylgruppe $R_1$ überführt werden.

Eine veresterte Carboxylgruppe $R_1$ kann ferner in üblicher Weise, z.B. durch Umsetzung mit einem entsprechenden Metallalkoholat, z.B. dem Natrium- oder Kaliumalkoholat des entsprechenden Alkohols, oder mit diesem selbst in Gegenwart eines Katalysators, beispielsweise einer starken Base, z.B. von Natrium- oder Kaliumhydroxid, oder einer starken Säure, wie einer Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Aetherat, zu einer anderen veresterten Carboxylgruppe $R_1$ umgesetzt werden.

Eine amidierte Carboxylgruppe $R_1$ kann in üblicher Weise, z.B. durch Hydrolyse in Gegenwart eines Katalysators, beispielsweise einer starken Base, wie eines Alkalimetall- oder Erdalkalimetallhydroxides oder -carbonates, z.B. von Natrium- oder Kaliumhydroxid oder -carbonat, oder einer starken Säure, wie einer Mineralsäure, z.B. von Salzsäure, Schwefelsäure oder Phosphorsäure in die freie Carboxylgruppe $R_1$ umgewandelt werden.

Enthalten die Verbindungen der Formel (I) ungesättige Reste, wie Niederalkenyl oder Niederalkenylengruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at. und bei Temperaturen zwischen etwa -80° bis etwa 200°C, vor allem zwischen Raumtemperatur und etwa 100°C durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Umgekehrt können in cyclischen Systemen $R_3$ eine oder mehrere Mehrfachbindungen eingeführt werden, Hierzu verwendet man geeignete Dehydrierungsmittel, beispielsweise Nebengruppenelemente, vorzugsweise solche der VIII Nebengruppe des Periodensystems, z.B. Palladium oder Platin, oder entsprechende Edelmetallderivate, z.B. Ruthenium-triphenylphosphid-chlorid, wobei die Mittel auf geeigneten Trägermaterialien aufgezogen sein können. Weitere bevorzugte Dehydrierungsmittel sind beispielsweise Chinone, wie p-Benzochinone, z.B. Tetrachlor-p-benzochinon oder 2,3-Dichlor-5,6-dicyano-p-benzochinon, oder wie Antrachinone, z.B. Phenanthren-9,10-chinon. Ferner können N-Halogensuccinimide,

wie N-Chlorsuccinimid, Manganverbindungen, wie Bariummanganat oder Mangandioxid, und Selenderivate, wie Selendioxid oder Diphenylselenium-bis-trifluoracetat, verwendet werden.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neue Verbindung einschliesslich ihrer Salze kann auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Ueberführung in diastereomere Salze oder Ester, z.B. durch Umsetzung eines sauren Endstoffes mit einer mit der racemischen Säure Salze bildenden optisch aktiven Base oder einer optisch aktiven Carbonsäure oder einem reaktiven Derivat davon, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, ihre Verwendung, z.B. als Arzneimittelwirkstoffe, Formulierungsverfahren und Verfahren zu ihrer Herstellung bilden ebenfalls einen Gegenstand der Erfindung.

Die Ausgangsstoffe der Formeln II, III, IV, V, VI, VII und VIII, die speziell für die Herstellung der erfindungsgemässen Verbindungen entwickelt werden, die Verfahren zu ihrer Herstellung sowie deren Verwendung bilden ebenfalls einen Gegenstand der Erfindung. Insbesondere Verbindungen der Formel (VI), worin $X_{11}$ gegebenenfalls verestertes oder verethertes Hydroxymethyl bzw. gegebenenfalls acetalisiertes Formyl bedeutet, Verfahren zu deren Herstellung sowie deren Verwendung, z.B. als Ausgangsstoffe oder Arzneimittelwirkstoffe, ferner pharmazeutische Präparate und deren Herstellung bilden einen weiteren Gegenstand der Erfindung.

Bei den erfindungsgemässen pharmazeutischen Präparaten, welche die erfindungsgemässe Verbindung oder pharmazeutisch verwendbare Salze davon enthalten, handelt es sich um solche zur topischen Anwendung, ferner zur enteralen, wie oralen oder rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand, sowie von der Applikationsweise ab.

Die neuen pharmazeutischen Präparate enthalten z.B. von etwa 10 % bis etwa 80 %, vorzugsweise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Regulier- und Schmiermittel, z.B. Kieselsäure, Talk, Stearinsäure oder Salze davon, wie Magnesium- oder Calciumstearat,

und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder supsendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Als topisch anwendbare pharmazeutische Präparate kommen in erster Linie Creme, Salben, Pasten, Schäume, Tinkturen und Lösungen in Frage, die von etwa 0,1 bis etwa 5 % des Wirkstoffs enthalten.

Creme sind Oel-in-Wasser Emulsionen die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachse oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme verhindern, z.B. Polyalkohole, wie Glycerin, Sorbit, Propylenglykol und/oder Polyethylenglykole, ferner Konservierungsmittel, Riechstoffe etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachse enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden z.B. aus Druckbehältern verabreicht und sind in Aerosolform vorliegende flüssige Oel-in Wasser-Emulsionen, wobei halogenierte Kohlenwasserstoffe, wie Chlorfluorniederalkane, z.B. Dichloridfluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-ethanolische Grundlage auf, der u.a. Polyalkohole, z.B. Glycerin, Glykole, und/oder Polyethylenglykol, Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei der Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Dosierung des Wirkstoffs hängt von der Warmblüter-Spezies dem Alter und dem individuellen Zustand sowie der Applikationsweise ab. Im Normalfall ist für einen etwa 75 kg schweren Warmblüter bei oraler Applikation eine ungefähre Tagesdosis von etwa 100 bis etwa 600 mg vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: 5,4 g (0,02 Mol) 5-Chlor-3-methyl-6-morpholino-benzofuran-2(3H)-on werden in 40 ml 1n Natronlauge bei 50° C gelöst. Nach dem Abkühlen wird mit Ether gewaschen und die wässrige Phase anschliessend mit 1n Salzsäure auf pH 2,0 gestellt. Das dabei gebildete Oel wird in Ether aufgenommen.

Nach dem Verdampfen des Ethers wird 2-(5-Chlor-2-hydroxy-4-morpholino-phenyl)-propionsäure als farblose Kristalle vom Schmelzpunkt 198 bis 200° C erhalten.

Das Ausgangsmaterial kann wie folgt hergestellt werden:

Ein Gemisch von 341 g (2 Mol) des Hydrochlorids von Imidazo[1,2-a]-pyridin-2-(3H)-on in 700 ml Wasser wird unter Rühren portionenweise mit einer heissen Lösung von 80 g (2 Mol) Natronlauge in 200 ml Wasser versetzt. Anschliessend wird eine Lösung von 250,7 g (2,16 Mol) Maleinsäure in 600 ml Wasser so zugetropft, dass die Innentemperatur des Reaktionsgemisches zwischen 40° C und 45° C bleibt. Nach 30 Stunden bei Raumtemperatur (20 bis 25° C) wird auf 5° C gekühlt, der gebildete Niederschlag abfiltriert, das Filtrat im Vakuum auf etwa die Hälfte eingeengt und das ausgefallene Produkt abgenutscht. Die vereinigten Rückstände werden mit wenig kaltem Methanol gewaschen und bei 50° C im Vakuum getrocknet. Es resultieren 400 g 3-(1,2-Dicarboxyethyl)-imidazo[1,2-a]pyridin-2(3H)-on vom Schmelzpunkt 193°C (Zers.). Das erhaltene Produkt wird bei Raumtemperatur während 6 Stunden mit 650 ml konz. Salzsäure verrührt. Nach Kühlen auf 5° C wird der Niederschlag abfiltriert, das Filtrat im Vakuum auf etwa die Hälfte eingeengt und das ausgefallene Produkt abgenutscht. Die vereinigten Rückstände werden mit Aceton gewaschen und bei 50° C im Vakuum getrocknet.
Man erhält so das Hydrochlorid von 3-(1,2-Dicarboxyethyl)-imidazo[1,2-a]pyridin-2(3H)-on vom Schmelzpunkt 205° C (Zers.).

Ein Gemisch von 114,7 g (0,4 Mol) des Hydrochlorids von 3-(1,2-Dicarboxyethyl)-imidazo[1,2-a]pyridin-2(3H)-on, 36,4 g (0,52 Mol) Methyl-vinylketon, 150 ml Methanol und 150 ml Wasser wird während 36 Stunden bei Raumtemperatur gerührt und anschliessend bei ca. 45° C im Vakuum zur Trockene eingedampft. Das erhaltene Rohprodukt wird in 300 ml Eisessig aufgenommen, mit 15 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch von 150 ml 6M Schwefelsäure und 150 ml Tetrahydrofuran versetzt und während 8 Stunden bei 60° C gehalten. Nach dem Entfernen des Tetrahydrofurans im Vakuum wird das Reaktionsgemisch mit Wasser verdünnt, mit Methylenchlorid ausgezogen und über Kieselgel filtriert. Destillation des Rohproduktes im Hochvakuum (115° C bis 125° C/8 Pa) ergibt das 4- Methyl-3-(3-oxo-butyl)-maleinsäureanhydrid als spektroskopisch einheitliches blassgelbes Oel.

Ein Gemisch von 18,2 g (0,1 Mol) 4-Methyl-3-(3-oxobutyl)-maleinsäureanhydrid und 22 g (0,105 Mol) Morpholiniumbenzoat in 400 ml Benzol wird während 48 Stunden am Wasserabscheider unter Rückfluss erhitzt. Das Benzol wird im Vakuum entfernt,der Rückstand in Methylenchlorid aufgenommen und die organische Phase zweimal mit gesättigter Natriumbicarbonatlösung extrahiert. Das nach dem Trocknen und Entfernen des Methylenchlorids verbleibende Rohprodukt wird mit Petrolether/Ether an Kieselgel chromatographiert. Es werden blassgelbe Kristalle erhalten, die aus Methylenchlorid/Ether umkristallisiert werden.

Man erhält so das 3-Methyl-6-morpholino-benzofuran-2(3H)-on vom Schmelzpunkt 118 bis 121° C.

Zu einem Gemisch aus 14,7 g (0,063 Mol) 3-Methyl-6-morpholino-benzofuran-2(3H)-on in 100 ml Chloroform wird bei 0 bis 5° C unter Rühren eine kalte Lösung von Chlor in Chloroform getropft, bis im Dünnschichtchromatogramm kein Edukt mehr sichtbar ist. Das Reaktionsgemisch wird mit Methylenchlorid verdünnt und nacheinander mit 10%iger Natriumthiosulfatlösung, verdünnter Natriumbicarbonatlösung und Wasser gewaschen. Das nach dem Trocknen und Eindampfen der organischen Phase verbleibende Rohprodukt wird mit Petrolether/Ether an Kieselgel chromatographiert. Nach Umkristallisation der reinen Fraktionen aus Ether/Petrolether erhält man das 5-Chlor-3-methyl-6-morpholino-benzofuran-2(3H)-on vom Schmelzpunkt 103 bis 105° C.

<u>Beispiel 2:</u> Ein Gemisch von 19,6 g (0,1 Mol) 4-Methyl-3-(2-methyl-3-oxobutyl)-maleinsäureanhydrid und 21,3 g (0,11 Mol) Pyrrolidiniumbenzoat in 400 ml Benzol wird während 30 Stunden am Wasserabscheider unter Rückfluss erhitzt. Das Benzol wird im Vakuum entfernt und der Rückstand zwischen Ether und gesättigter Natriumbicarbonatlösung verteilt. Das nach dem Trocknen und Eindampfen der organischen Phase verbleibende Rohprodukt wird an Kieselgel chromatographiert. Elution mit Petrolether/Ether und anschliessende Umkristallisation der reinen Fraktionen aus Ether/Petrolether liefert 3,5-Dimethyl-6-(pyrrolidin-1-yl)-benzofuran-2(3H)-on vom Schmelzpunkt 67 bis 69° C. Durch Steigerung der Fliessmittelpolarität (Ether/Methanol) werden aus den folgenden Fraktionen 2-[2-Hydroxy-5-methyl-4-(pyrrolidin-1-yl)-phenyl]-propionsäurepyrrolidid erhalten. Umkristallisation aus Aceton ergibt reines Produkt vom Schmelzpunkt 178 bis 180° C.

Das Ausgangsmaterial kann wie folgt hergestellt werden:

Ein Gemisch aus 172 g (0,6 Mol) Hydrochlorid von 3-(1,2-Dicarboxyethyl)-imidazol[1,2-a]pyridin-2(3H)-on, 65,5 g (0,78 Mol) 3-Methyl-3-buten-2-on, 220 ml Methanol und 220 ml Wasser wird während 36 Stunden

bei Raumtemperatur gerührt und anschliessend bei ca. 45° C im Vakuum zur Trockene eingedampft. Das erhaltene Rohprodukt wird in 400 ml Eisessig aufgenommen, mit 22,5 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch aus 225 ml 6 M Schwefelsäure und 225 ml Tetrahydrofuran versetzt und während 8 Stunden am Rückfluss erhitzt. Nach dem Entfernen von Tetrahydrofuran im Vakuum wird das Reaktionsgemisch mit Wasser verdünnt und mit Methylenchlorid ausgezogen. Das nach dem Trocknen und Eindampfen der organischen Phase verbleibende Rohprodukt wird mit Petrolether/Ether an Kieselgel chromatographiert. Die anschliessende Destillation (100° C/8·$10^{-2}$ Torr) ergibt 4-Methyl-3-(2-methyl-3-oxobutyl)-maleinsäureanhydrid als blassgelbes Oel.

Beispiel 3: Ein Gemisch von 19,6 g (0,1 Mol) 4-Methyl-3-(2-methyl-3-oxobutyl)-maleinsäureanhydrid und 23,0 g (0,11 Mol) Morpholiniumbenzoat in 400 ml Benzol wird während 60 Stunden am Wasserabscheider unter Rückfluss erhitzt. Das Benzol wird im Vakuum entfernt und der Rückstand zwischen Methylenchlorid und gesättigter Natriumbicarbonatlösung verteilt. Durch weiteres Vorgehen, wie in Beispiel 2 beschrieben , werden 3,5-Dimethyl-6-morpholino-benzofuran-2(3H)-on vom Schmelzpunkt 108 bis 109° C und 2-(2-Hydroxy-5-methyl-4-morpholino-phenyl)-propionsäuremorpholid vom Schmelzpunkt 183 bis 185° C erhalten.

Beispiel 4: Zu einer Lösung von 0,9 g (0,039 Mol) Natrium in 100 ml Methanol werden 9,5 g (0,035 Mol) 5-Chlor-3-methyl-6-morpholino-benzofuran-2(3H)-on gegeben. Nach 3 Stunden bei Raumtemperatur wird im Vakuum zur Trockne eingedampft und der Rückstand in 50 ml Dimethylsulfoxid gelöst. Dazu werden unter Rühren 5,7 g (0,04 Mol) Methyljodid getropft. Nach 16 Stunden bei Raumtemperatur wird die Lösung mit 300 ml Wasser und 100 ml Hexan versetzt und der ausgefallene Niederschlag abfiltriert. Das Filtrat wird mehrmals mit Hexan extrahiert. Nach dem Verdampfen des Hexans wird ein kristalliner Rückstand erhalten. Die rohen Kristalle werden aus Isopropylether umkristallisiert. Es wird 2-(5-Chlor-2-methoxy-4-morpholino-phenyl)-propionsäuremethylester als farblose Kristalle erhalten, Schmelzpunkt 88 bis 89° C.

Beispiel 5: Zu einer Lösung von 0,5 g Natrium (0,022 Mol) in 50 ml Methanol werden 5,4 g (0,02 Mol) 5-Chlor-3-methyl-6-morpholino-benzofuran-2(3H)-on gegeben und 3 Stunden bei Raumtemperatur stehen gelassen. Anschliessend wird im Vakuum zur Trockene eingedampft, der Rückstand in kaltem Wasser gelöst und mit Ether gewaschen. Die wässrige Phase wird unter Eiskühlung mit verdünnter Salzsäure kongosauer gestellt und mit Ether extrahiert. Nach dem Verdampfen des Ethers werden farblose Kristalle erhalten, die aus Methanol umkristallisiert werden.
Man erhält den 2-(5-Chlor-2-hydroxy-4-morpholino-phenyl)-propionsäuremethylester vom Schmelzpunkt 148 bis 149° C.

Beispiel 6: Zu einer Lösung von 5,4 g (0,02 Mol) 5-Chlor-3-methyl-6-morpholino-benzofuran-2(3H)-on in 25 ml Ether werden 2,0 g (0,023 Mol) Morpholin gegeben. Nach 3 Stunden wird der gebildete Niederschlag abfiltriert, wobei farblose Kristalle, das 2-(5-Chlor-2-hydroxy-4-morpholino-phenyl)-propionsäure-morpholid vom Schmelzpunkt 198 bis 199° C, erhalten werden.

Beispiel 7: 6 g 0,019 Mol) 2-(5-Chlor-2-methoxy-4-morpholino-phenyl)-propionsäuremethylester werden in 100 ml 2n Salzsäure 2 Stunden am Rückfluss gekocht. Anschliessend wird mit verdünnter Natronlauge auf pH 2,5 gestellt und mehrmals mit Ether extrahiert. Nach dem Abdampfen des Ethers werden Kristalle erhalten, die aus Essigester/Petrolether (1:1) umkristallisiert werden. Man erhält so 2-(5-Chlor-2-methoxy-4-morpholino-phenyl)-propionsäure als grobe Prismen vom Schmelzpunkt 164 bis 165° C.

Beispiel 8: Eine Suspension von 3,0 g (0,01 Mol) 2-(5-Chlor-2-hydroxy-4-morpholino-phenyl)-propionsäure-methylester und 0,03 g 4-Dimethylamino-pyridin in 30 ml Essigsäureanhydrid werden während 5 Minuten auf dem Wasser-Bad auf 50° C erwärmt und gelöst. Nach 1 Stunde Raumtemperatur wird im Vakuum zur Trockene eingedampft und der Rückstand mit Methylenchlorid an Kieselgel chromatographiert. Es werden farblose Kristalle erhalten, die aus Isopropylether umkristallisiert werden. Man erhält so den 2-(2-Acetoxy-5-chlor-4-morpholino-phenyl)-propionsäure-methylester vom Schmelzpunkt 104 bis 105° C .

Beispiel 9: Eine Lösung von 11,07 g (30 mMol) 5-Chlor-2-methoxy-4-(piperidin-1-yl)-phenylthioessigsäuremorpholinamid in 120 ml Eisessig und 30 ml konz. Salzsäure wird während 22 Stunden unter Rückfluss gekocht. Das Reaktionsgemisch wird abgekühlt, mit Wasser verdünnt und mit Methylenchlorid extrahiert. Die vereinigten Methylenchloridphasen werden mit Wasser gewaschen, über Natriumsulfat getrocknet und am Hochvakuumrotationsverdampfer eingedampft. Nach Chromatographieren an Kieselgel mit Chloroform/Methanol (19:1) erhält man die 5-Chlor-2-methoxy-4-(piperidin-1-yl)-phenylessigsäure, die nach Umkristallisation mit Methylenchlorid/Hexan bei 120 bis 122° C schmilzt.

In analoger Weise erhält man die 5-Chlor-2-methoxy-4-(4-morpholino)-phenylessigsäure vom Schmelzpunkt 141 bis 143° C.

Das Ausgangsmaterial kann wie folgt hergestellt werden:
Eine Lösung von 96 g (0,72 Mol) Aluminiumtrichlorid in 180 ml abs. Nitromethan wird unter Stickstoff-Atmosphäre und Eis/Methanol-Kühlung zu einem Gemisch von 106,2 g (0,60 Mol) 3,4-Dichloranisol [H. Jamarlik et al, Comptes Rendus Acad. Sci. Ser. C 273 (25), 1756 (1971)] und 51,1 ml (0,72 Mol) Acetylchlorid während ca. 30 Minuten so zugetropft, dass der Innentemperaturbereich zwischen 0 und 5° C liegt. Anschliessend wird noch 1 Stunde bei etwa 4 bis 6° C weitergerührt, dann auf Eis gegossen und mit Methylenchlorid extrahiert. Die organischen Extrakte werden mit Wasser gewaschen, vereinigt, über Natriumsulfat

getrocknet und am Vakuumrotationsverdampfer eingedampft. Nach Umkristallisation aus Methanol/Wasser erhält man das 4,5-Dichlor-2-methoxy-acetophenon vom Schmelzpunkt 93 bis 95° C.

Eine Lösung von 76,7 g (0,35 Mol) 4,5-Dichlor-2-methoxy-acetophenon in 750 ml Piperidin wird während 7 Stunden bei 170° C im Autoklaven gehalten. Das Reaktionsgemisch wird eingedampft, in Essigester aufgenommen und mit Wasser gewaschen. Die Essigester-Extrakte werden vereinigt, über Natriumsulfat getrocknet und am Vakuumrotationsverdampfer eingedampft. Der Rückstand wird an Kieselgel mit Methylenchlorid chromatographiert. Man erhält so das 5-Chlor-2-hydroxy-4-(N-piperidino)-acetophenon vom Schmelzpunkt 68 bis 70° C.

In analoger Weise erhält man das 5-Chlor-2-hydroxy-4-(N-morpholino)-acetophenon vom Schmelzpunkt 102 bis 103° C.

Eine Lösung von 32,5 g (128 mMol) 5-Chlor-2-hydroxy-4-(N-piperidino)-acetophenon und 75 ml (166 mMol) einer etwa 40 %igen methanolischen Lösung von Benzyltriethylammoniumhydroxid (Triton B) in 65 ml Tetrahydrofuran wird auf 0° C abgekühlt. Währen d ca. 6 Minuten werden 14,6 ml (154 mMol) Dimethylsulfat so zugetropft, dass die Innentemperatur nicht über 5° C steigt. Das Reaktionsgemisch wird noch 1 Stunde bei 0° gerührt, dann während etwa 30 Minuten unter Rückfluss gekocht. Das Reaktionsgemisch wird nun auf 400 ml Wasser gegossen und mit Essigester extrahiert. Die vereinigten Essigesterphasen werden mit Wasser gewaschen, über Natriumsulfat getrocknet und am Vakuumrotationsverdampfer eingedampft. Der Rückstand wird aus Methylenchlorid/Hexan umkristallisiert, und man erhält das 5-Chlor-2-methoxy-4-(N-piperidino)-acetophenon vom Schmelzpunkt 119 bis 120° C.

In analoger Weise erhält man das 5-Chlor-2-methoxy-4-(N-morpholino)-acetophenon vom Schmelzpunkt 143 bis 145° C.

Eine Lösung von 18,2 g (68 mMol) 5-Chlor-2-methoxy-4-(piperidin-1-yl)-acetophenon und 4,36 g (136 mMol) Schwefel in 68 ml Morpholin wird während 5 Stunden bei 90° C gehalten. Das Reaktionsgemisch wird abgekühlt, mit Essigester verdünnt und mit Wasser gewaschen. Die vereinigten Essigester-Extrakte werden über Natriumsulfat getrocknet und am Vakuumrotationsverdampfer eingedampft. Nach Umkristallisation aus Methylenchlorid/Methanol erhält man das 5-Chlor-2-methoxy-4-(piperidin-1-yl)-phenylthioessigsäuremorpholinamid vom Schmelzpunkt 137 bis 139°C.

In analoger Weise erhält man das 5-Chlor-2-methoxy-4-(4-morpholino)-phenylthioessigsäuremorpholinamid vom Schmelzpunkt 160 bis 162,5° C.

Beispiel 10: Eine Lösung von 8,5 g (30 mMol) 5-Chlor-2-methoxy-4-(4-piperidin-1-yl)-phenylessigsäure in 150 ml 48%iger Bromwasserstoffsäure wird während 15 Stunden unterRückfluss gekocht. Das Reaktionsgemisch wird abgekühlt, mit Wasser verdünnt und mit gesättigter Natriumbicarbonat-Lösung auf pH 3 bis 4 gestellt. Anschliessend wird mit Essigester extrahiert, die vereinigten organischen Phasen mit Wasser gewaschen, über Natriumsulfat getrocknet und am Hochvakuumrotationsverdampfer eingedampft. Man erhält so einen dunkelgrauen Schaum von 5-Chlor-2-hydroxy-4-(piperidin-1-yl)-phenylessigsäure.

Analog erhält man die 2-Hydroxy-4-(4-morpholino)-phenylessigsäure.

Beispiel 11: Eine Lösung von 4,03 g (16,0 mMol) 5-Chlor-3-methyl-6-(pyrrolidin-1-yl)-benzofuran-2(3H)-on in 160 ml Methanol wird unter $N_2$-Atmosphäre bei Raumtemperatur während ca. 2 Minuten mit 160 ml 0,1 N NaOH versetzt und während ca. 60 Minuten bei Raumtemperatur gerührt. Das Lösungsmittel wird anschliessend eingeengt und der Rückstand gefriergetrocknet. Es resultierten 2-(5-Chlor-2-hydroxy-4-(pyrrolidin-1-yl)-phenyl)-propionsäure-Natriumsalz, Schmelzpunkt über 200°C unter Zersetzung.

In analoger Weise wird 2-(5-Chlor-2-hydroxy-4-morpholinophenyl)-pro-

propionsäure-Natriumsalz vom Schmelzpunkt über 200°C (Zersetzung).

Beispiel 12: 59 g 4-Methyl-3-(2-methyl-3-oxo-butyl)-maleinsäureanhydrid und 240 g Dibenzylammoniumbenzoat werden in 1000 ml Benzol mit einem Wasserabscheider 48 Stunden am Rückfluss erhitzt. Anschliessend wird im Vakuum zur Trockene eingedampft und der Rückstand in Methylenchlorid über Kieselgel chromatographiert. Das erhaltene Oel kristallisiert aus Isopropylether. Man erhält so das 2-(4-Dibenzylamino-2-hydroxy-5-methyl-phenyl)-propionsäure-dibenzylamid vom Smp. 140 bis 141° C.

Das Ausgangsmaterial kann wie folgt hergestellt werden:

Ein Gemisch aus 172 g (0,6 Mol) Hydrochlorid von 3-(1,2-Dicarboxyethyl)-imidazo[1,2-a]pyridin-2(3H)-on, 65,5 g (0,78 Mol) 3-Methyl-3-buten-2-on, 220 ml Methanol und 220 ml Wasser wird während 36 Stunden bei Raumtemperatur gerührt und anschliessend bei ca. 45° im Vakuum zur Trockne eingedampft. Das erhaltene Rohprodukt wird in 400 ml Eisessig aufgenommen, mit 22,5 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch aus 225 ml 6 M Schwefelsäure und 225 ml Tetrahydrofuran versetzt und während 8 Stunden am Rückfluss erhitzt. Nach dem Entfernen von Tetrahydrofuran im Vakuum wird das Reaktionsgemisch mit Wasser verdünnt und mit Methylenchlorid ausgezogen. Das nach dem Trocknen und Eindampfen der organischen Phase verbleibende Rohprodukt wird mit Petrolether/Ether an Kieselgel chromatographiert. Die anschliessende Destillation (100°C/$8 \cdot 10^{-2}$ Torr) ergibt 4-Methyl-3-(2-methyl-3-oxobutyl)-maleinsäureanhydrid als blassgelbes Oel.

Beispiel 13: 20 g 2-(4-Dibenzylamino-2-hydroxy-5-methyl-phenyl)-propionsäure-dibenzylamid werden in 40 ml 2nSalzsäure und 40 ml Eisessig 3 Stunden am Rückfluss gekocht. Anschliessend wird im Vakuum zur Trockene eingedampft und der Rückstand zwischen Ether und ln Natronlauge verteilt. Durch Ansäuern auf pH = 1 mit Salzsäure und Extrahieren erhält man die 2-(4-Dibenzylamino-2-hydroxy-5-methyl-phenyl)-propionsäure, die zur Reinigung in Methylenchlorid über Kieselgel chromatographiert wird, Smp. 174 bis 175° C.

Beispiel 14: 2,3 g (0,01 Mol) 3,5-Dimethyl-6-(pyrrol-1-yl)-benzofuran-2(3H)-on werden mit 15 ml ln Natronlauge und 50 ml Ether während 5 Minuten geschüttelt. Die Säure wird isoliert, indem man die Natronlauge mit konzentrierter Salzsäure auf pH 1 stellt und mit Ether extrahiert.

Nach Umkristallisation aus Isopropylether/Petrolether wird die 2-[2-Hydroxy-5-methyl-4-(pyrrol-1-yl)-phenyl]-propionsäure vom Smp. 73-74° erhalten.

Das Ausgangsmaterial kann beispielsweise wie folgt erhalten werden:

Ein Gemisch von 19,6 g (0,1 Mol) 4-Methyl-3-(2-methyl-3-oxobutyl)-maleinsäureanhydrid und 20 g (0,105 Mol) 3-pyrrolinium-benzoat in 250 ml Benzol wird während 5 Stunden am Wasserabscheider unter Rückfluss erhitzt. Das Benzol wird im Vakuum abgedampft und der Rückstand zwischen Ether und gesättigter Natriumbicarbonatlösung verteilt. Das nach dem Trocknen und Eindampfen der organischen Phase verbleibende Rohprodukt wird an Kieselgel chromatographiert. Elution mit Diisopropylether und anschliessende Umkristallisation

der reinen Fraktionen aus Isopropylether liefert das 3,5-Dimethyl-6-(pyrrol-1-yl)-3a,6-dihydro-benzofuran-2(3H)-on vom Smp. 116-117°.

Beispiel 15: Ein Gemisch aus 9,0g(0,04 Mol) 3,5-Dimethyl-6-(pyrrol-1-yl)-benzofuran-2(3H)-on und 2,4 g (0,045 Mol) Natriummethylat in 40 ml Methanol wird während 90 Minuten bei Raumtemperatur gerührt. Das Methanol wird im Vakuum abgedampft und der Rückstand in 100 ml Ether gelöst. Zu dieser Lösung wird bei 0-5° innert 30 Minuten eine Lösung von 4,5 g (0,057 Mol) Acetylchlorid in 25 ml Ether getropft. Es wird 14 Stunden bei Raumtemperatur gerührt und anschliessend mit Wasser und eiskalter 1n Natronlauge gewaschen. Die nach dem Abdampfen des Ethers erhaltenen Neutralteile werden mit einem Gemisch aus Methylenchlorid/Hexan (3:1) an Kieselgel chromatographiert. Umkristallisation der reinen Eluate aus Hexan ergibt 2-[2-Acetoxy-5-methyl-4-(pyrrol-1-yl)-phenyl]-propionsäuremethylester vom Smp. 70-71°.

Beispiel 16: Ein Gemisch aus 5,5 g (23,8 mMol) 3,5-Dimethyl-6-(pyrrolidin-1-yl)-benzofuran-2(3H)-on, 1,28 g (23,8 mMol) Natriummethylat in 40 ml Methanol wird während 90 Minuten bei Raumtemperatur gerührt. Das Methanol wird im Vakuum entfernt und der Rückstand in 90 ml Tetrahydrofuran aufgenommen. Zu diesem Gemisch werden bei 0-5° während 30 Minuten 1,9 ml (26,7 mMol) Acetylchlorid getropft. Es wird eine Stunde bei Raumtemperatur nachgerührt, das Tetrahydrofuran im Vakuum entfernt, der Rückstand in Methylenchlorid aufgenommen und die organische Phase mit verdünnter Natriumbicarbonatlösung extrahiert. Das nach dem Trocknen und Eindampfen des Methylenchlorids erhaltene Rohprodukt wird mit Petrolether/Ether an Kieselgel chromatographiert. Destillation der reinen Fraktionen im Kugelrohr 150°C/$6 \cdot 10^{-2}$ Torr) ergibt 2-[2-Acetoxy-5-methyl-4-(pyrrolidin-1-yl)-phenyl]-propionsäuremethylester.

Beispiel 17: Zu einer Lösung von 2,7 g (0,01 Mol) 2-(5-Chlor-2-hydroxy-4-morpholino-phenyl)-propan-1-ol in 20 ml Aceton wird unter Rühren bei 15-20°C innert 15 Minuten eine Lösung von 3,0 g (0,025

Mol) Chromsäure in 20%iger Schwefelsäure getropft. Nach Zugabe von 10 ml Methanol wird filtriert und das Filtrat im Vakuum eingeengt. Nun wird mit verdünnter Natronlauge auf pH 1-2 gestellt und mehrmals mit Ether extrahiert. Nach Trocknen und Abdampfen des Ethers wird der Rückstand aus Ether umkristallisiert. Auf diese Weise erhält man 2-(5-Chlor-2-hydroxy-4-morpholino-phenyl)-propionsäure vom Smp. 198-200°.

Das Ausgangsmaterial kann beispielsweise wie folgt erhalten werden:

Zu einer Suspension von 0,8 g Lithiumaluminiumhydrid (0,02 Mol) in 50 ml abs. Ether werden unter Stickstoffatmosphäre, Rühren und Eiskühlung bei 0-5° innert 30 Minuten 2,7 g (0,01 Mol) 5-Chlor-3-methyl-6-morpholino-benzofuran-2(3H)-on gelöst in 100 ml abs. Ether getrofpt. Anschliessend wird 3 Stunden bei Raumtemperatur gerührt. Durch vorsichtiges Zutropfen von ca. 10 ml Wasser unter Eiskühlung wird der Lithiumaluminiumkomplex zerlegt. Mittels 1n Salzsäure wird schwach angesäuert und 5 mal mit Chloroform extrahiert. Das so erhaltene Rohprodukt wird aus Essigester umkristallisiert. Auf diese Weise wird 2-(5-Chlor-2-hydroxy-4-morpholino-phenyl)-propan-1-ol vom Smp. 176-177° isoliert.

<u>Beispiel 18:</u> Eine methanolische Lösung von 26,9 g (0,10 Mol) 5-Chlor-2-methoxy-4-morpholino-acetophenon wird portionsweise unter Rühren mit 3,8 g (0,10 mMol) Natriumborhydrid versetzt und eine Stunde bei Raumtemperatur gerührt. Das Methanol wird am Vakuumrotationsverdampfer eingeengt und der Rückstand zwischen verdünnter Salzsäure und Methylenchlorid verteilt. Die organischen Phasen werden vereinigt, über Natriumsulfat getrocknet und eingedampft. Der Rückstand wird in 60 ml abs. Methylenchlorid aufgenommen und während 2 Stunden unter Stickstoffatmosphäre zu einem Gemisch von 17,8 g (0,15 Mol) Thionylchlorid und 120 ml abs. Methylenchlorid getropft. Anschliessend wird noch 1 Stunde gerührt, das Lösungsmittel am Vakuumrotationsverdampfer eingeengt, und der Rückstand zwischen Natriumbicarbonatlösung und Methylenchlorid verteilt. Die organischen Phasen werden neutral

gewaschen, vereinigt, über Natriumsulfat getrocknet und eingeengt. Der Rückstand wird in 100 ml abs. Tetrahydrofuran aufgenommen und zu einer Suspension von 2,4 g (0,10 Gramm Atom) Magnesium-Spänen in 20 ml abs. Tetrahydrofuran so getropft, dass das Reaktionsgemisch leicht unter Rückfluss kocht. Anschliessend wird noch 2 Stunden unter Rückfluss weiter gekocht. Die auf Raumtemperatur abgekühlte Lösung wird vorsichtig zu ca. 50 g mit abs. Tetrahydrofuran überschichtetem Trockeneis getropft. Das Reaktionsgemisch wird auf Raumtemperatur erwärmt, mit verdünnter Salzsäure angesäuert und dreimal mit Methylenchlorid extrahiert. Die organischen Phasen werden neutral gewaschen, vereinigt, über Natriumsulfat getrocknet und am Vakuumrotationsverdampfer eingeengt. Umkristallisation des Rohprodukts aus Essigester/Petrolether ergibt 2-(5-Chlor-2-methoxy-4-morpholino-phenyl)-propionsäure vom Schmelzpunkt 164-165°.

Beispiel 19: In einem gut ventilierten Abzug werden ca. 27 g (1,0 Mol) flüssige Blausäure aus einer Druckflasche mit Stickstoff in einem Eis/Kochsalz gekühlten Sulfierkolben gebracht. Während ca. 2 Minuten wird 134,9 g (0,50 Mol) 5-Chlor-2-methoxy-4-morpholino-acetophenon und 250 mg (2,9 mMol) Piperidin zugegeben. Das gebildete Cyanhydrin wird nach 30 Minuten bei 0° mit 100 ml Aether verdünnt und mit Stickstoff in 300 ml konzentrierte Salzsäure gepresst, die mit Eis/Kochsalz gekühlt und gut gerührt wird. Das Gemisch wird anschliessend mit Salzsäuregas gesättigt und dann während ca. 15 Stunden bei Raumtemperatur stehen gelassen. Das auskristallisierte Amid wird abgenutscht, mit Wasser gewaschen und ohne Reinigung mit 750 ml 20%iger wässriger Kaliumhydroxidlösung während 3 Stunden unter Rückfluss gekocht. Das Reaktionsgemisch wird gekühlt, mit 6n Salzsäure angesäuert und dreimal mit Ether extrahiert. Die Etherphasen werden neutral gewaschen, vereinigt, über Natriumsulfat getrocknet und am Vakuumrotationsverdampfer eingeengt. Die so erhaltene rohe 2-Hydroxy-2-[5-chloro-2-methoxy-4-morpholino-phenyl]propionsäure wird bei Raumtemperatur portionenweise zu 300 ml konzentrierter Schwefelsäure gegeben. Nach ca. 10 Minuten Rühren wird das Reaktionsgemisch auf

2 kg Eis gegossen und mit Ether dreimal extrahiert. Die Etherextrakte werden mit Wasser neutral gewaschen, vereinigt, über Natriumsulfat getrocknet und am Vakuumrotationsverdampfer eingeengt. Der Rückstand wird in 700 ml Methanol aufgenommen, mit 7 g Palladium/Kohle versetzt und bei Raumtemperatur hydriert. Der Katalysator wird abfiltriert und das Lösungsmittel am Vakuumrotationsverdampfer eingeengt. Umkristallisation des Rohprodukts aus Essigester/Petrolether ergibt 2-(5-Chlor-2-methoxy-4-morpholino-phenyl)-propionsäure vom Schmelzpunkt 164-165°.

Beispiel 20: Eine Lösung von 2,86 g (10,0 mMol) 5-Chlor-2-methoxy-4-morpholino-phenylessigsäure in 50 ml gesättigter methanolischer Salzsäure wird während 12 Stunden unter Rückfluss gekocht. Das Reaktionsgemisch wird am Vakuumrotationsverdampfer eingeengt und der Rückstand in Methylenchlorid aufgenommen und dreimal mit Wasser gewaschen. Die organische Phase wird über Natriumsulfat getrocknet und am Vakuumrotationsverdampfer eingeengt. Der so erhaltene 5-Chlor-2-methoxy-4-morpholino-phenylessigsäuremethylester wird portionenweise unter starkem Rühren zu einem Gemisch von 514 mg (13 mMol) Natriumamid in 60 ml flüssigem Ammoniak gegeben. Anschliessend werden 2,84 g (20 mMol) Methyliodid zugetropft. Es wird 2 Stunden gerührt und anschliessend der Ammoniak verdampft. Der Rückstand wird zwischen verdünnter Salzsäure und Ether verteilt. Die Etherphasen werden über Natriumsulfat getrocknet und eingedampft. Umkristallisation des Rückstandes aus Isopropylether ergibt 2-(5-Chloro-2-methoxy-4-morpholino-phenyl)-propionsäuremethylester, Smp. 88-89°.

Beispiel 21: Ein Gemisch aus 4 g (12,8 mMol) 5-Brom-3-methyl-6-morpholino-benzofuran-2(3H)-on, 0,7 g (13 mMol) frisch bereitetes Natriummethylat in 25 ml Methanol wird während 45 Minuten bei Raumtemperatur gerührt. Das Methanol wird im Vakuum entfernt und der Rückstand in 50 ml Tetrahydrofuran aufgenommen. Zu diesem Gemisch werden bei 0-5°C während 2 Stunden 1,4 ml (19,7 mMol) Acetylchlorid getropft. Nach Stehen bei Raumtemperatur während 72 Stunden wird das

Tetrahydrofuran im Vakuum entfernt und der Rückstand mit Petrolether/Ether an Kieselgel chromatographiert. Anschliessende Umkristallisation der reinen Fraktionen aus Ether/Petrolether ergibt 2-(2-Acetoxy-5-brom-4-morpholino-phenyl)-propionsäuremethylester vom Smp. 114-115°C.

Das Ausgangsmaterial kann beispielsweise wie folgt erhalten werden:

Zu einem Gemisch aus 15 g (0,064 Mol) 3-Methyl-6-morpholino-benzofuran-2(3H)-on in 120 ml Chloroform wird bei 0-5°C unter Rühren während einer Stunde eine Lösung von 11 g (0,069 Mol) Brom in 50 ml Chloroform getropft. Anschliessend wird während 30 Minuten bei Raumtemperatur nachgerührt. Das Reaktionsgemisch wird mit Methylenchlorid versetzt und nacheinander mit 10%iger Natriumthiosulfatlösung, verdünnter Natriumbicarbonatlösung und Wasser gewaschen. Das nach dem Trocknen und Eindampfen der organischen Phase verbleibende Rohprodukt wird mit Petrolether/Ether an Kieselgel chromatographiert. Nach Umkristallisation der reinen Fraktionen aus Ether/Petrolether erhält man das 5-Brom-3-methyl-6-morpholino-benzofuran-2(3H)-on vom Smp. 99-100°C.

<u>Beispiel 22:</u> Eine Lösung von 132,9 g (0,759 Mol) 4-Methyl-3-nitro-anisol in 1,1 Liter Methanol wird mit 12,4 g Palladium auf Kohle versetzt und bei Raumtemperatur hydriert. Der Katalysator wird abfiltriert und das Filtrat am Vakuumrotationsverdampfer eingeengt. Umkristallisation aus Isopropanol/Wasser ergibt 3-Amino-4-methyl-anisol vom Smp. 43-44°.

Eine Lösung von 88,4 g (0,64 Mol) 3-Amino-4-methyl-anisol in 1,4 Liter Eisessig wird auf 106° erwärmt und bei dieser Temperatur während 30 Minuten mit 114 g (0,86 Mol) 2,5-Dimethoxy-tetrahydrofuran versetzt. Anschliessend wird sofort auf Raumtemperatur abgekühlt und am Vakuumrotationsverdampfer eingeengt. Destillation des Rückstandes am Hochvakuum liefert das 4-Methyl-3-(pyrrol-1-yl)-anisol, Kp. 93-95°/0,04 Torr. $R_f$ (Toluol/Essigester = 10:1) : 0,57.

Eine Lösung von 86,6 g (0,46 Mol) 4-Methyl-3-(pyrrol-1-yl)-anisol in 1,5 Liter absolutem Methylenchlorid wird mit Aceton/Trockeneis auf -78° abgekühlt. Bei dieser Temperatur werden 231,7 g (0,92 Mol) Bortribromid zugetropft. Anschliessend wird das Kühlbad entfernt und das Reaktionsgemisch auf 0 bis 5° erwärmt, dann auf 2 Liter Eis/Wasser gegossen, die Methylenchloridphasen abgetrennt und mit gesättigter Kochsalzlösung gewaschen. Die wässrigen Phasen werden noch 2 mal mit Methylenchlorid nachextrahiert. Die organischen Phasen werden vereinigt, über Natriumsulfat getrocknet und am Vakuumrotationsverdampfer eingeengt. Destillation des Rückstandes am Hochvakuum ergibt das 4-Methyl-3-(pyrrol-1-yl)-phenol, Kp. 105-107°/0,03 Torr, $R_f$ (Toluol/Essigester = 10:1) : 0,38.

Eine Suspension von 53,4 g (0,31 Mol) 4-Methyl-3-(pyrrol-1-yl)-phenol und 53,7 g (0,39 Mol) Kaliumcarbonat in 600 ml absolutem Aceton werden unter Rückfluss während 1 Stunde mit 45,7 g (0,39 Mol) Crotylbromid versetzt und anschliessend noch $4\frac{1}{2}$ Stunden weitergekocht. Das Reaktionsgemisch wird abgekühlt und mit 800 ml Wasser verdünnt. Das Aceton wird am Vakuumrotationsverdampfer abgedampft und der Rückstand mehrmals mit Methylenchlorid extrahiert. Die organischen Phasen werden mit Wasser gewaschen, vereinigt, über Natriumsulfat getrocknet und am Vakuumrotationsverdampfer eingeengt. Kurzfiltration an etwa 800 g Kieselgel mit Methylenchlorid ergibt 1-[4-Methyl-3-(pyrrol-1-yl)]-phenoxy-2-buten als hellgelbes Oel, $R_f$ (Hexan/Ether = 9:1) : 0,45, $R_f$ (Toluol/Essigester = 10:1) : 0,68.

Eine Lösung von 60 g (0,26 Mol) 1-[4-Methyl-3-(pyrrol-1-yl)]-phenoxy-2-buten in 170 ml absolutem N,N-Diethylanilin wird während 5 Stunden unter Rückfluss gekocht. Das Reaktionsgemisch wird abgekühlt, mit Methylenchlorid verdünnt und mit 6N Salzsäure angesäuert. Die Wasserphase wird abgetrennt und nochmals mit Methylenchlorid extrahiert. Die organischen Phasen werden neutral gewaschen, vereinigt, über Natriumsulfat getrocknet und am Vakuumrotationsverdampfer eingeengt. Chromatographie an Kieselgel mit Hexan/Ether (9:1) ergibt 3-[2-Hydroxy-5-methyl-4-(pyrrol-1-yl)-phenyl]-1-buten. $R_f$ (Hexan/Ether = 9:1): 0,17, $R_f$ (Toluol/Essigester = 10:1): 0,45.

Eine Lösung von 26,7 g (0,12 Mol) 3-[2-Hydroxy-5-methyl-4-(pyrrol-1-yl)-phenyl]-1-buten in 370 ml Essigsäureanhydrid wird mit einigen Tropfen Pyridin versetzt und während 2 Stunden bei Raumtemperatur gerührt. Das Reaktionsgemisch wird auf Eis gegossen und mit Methylenchlorid 3 mal extrahiert. Die Methylenchloridphasen werden mit verdünnter Natriumbicarbonatlösung, dann mit Wasser neutral gewaschen, vereinigt, über Natriumsulfat getrocknet und am Vakuumrotationsverdampfer eingeengt. Filtration an wenig Kieselgel mit Methylenchlorid ergibt 3-[2-Acetoxy-5-methyl-4-(pyrrol-1-yl)-phenyl]-1-buten, $R_f$ (Toluol/Essigester = 10:1) : 0,55.

Eine Lösung von 2,7 g (10 mMol) 3-[2-Acetoxy-5-methyl-4-(pyrrol-1-yl)-phenyl]-1-buten in 40 ml absolutem Methylenchlorid wird mit Aceton/Trockeneis auf -78° abgekühlt und solange Ozon durchgeblasen bis die blaue Farbe nicht mehr verschwindet. Dann werden 2 ml Dimethylsulfid zugegeben und das Kühlbad entfernt. Das Reaktionsgemisch wird am Vakuumrotationsverdampfer schonend eingeengt, der Rückstand in 50 ml Ethanol gelöst und mit einer Lösung von 3,7 g (23 mMol) Silbernitrat in 5 ml Wasser versetzt. Zu diesem Gemisch wird während etwa 15 Minuten eine Lösung von 75 ml einer 1N Kaliumhydroxidlösung zugetropft. Das heterogene Gemisch wird während weiteren 2 Stunden weitergerührt. Das Reaktionsgemisch wird filtriert und der Rückstand mit Ethanol gewaschen. Das alkalische Filtrat wird über Nacht bei Raumtemperatur stehengelassen und mit Methylenchlorid extrahiert. Die alkalische Lösung wird mit 6n Salzsäure unter Kühlung vorsichtigt angesäuert und mit Methylenchlorid mehrmals extrahiert. Die organischen Phasen werden noch zweimal mit Wasser gewaschen, vereinigt, über Natriumsulfat getrocknet und am Vakuumrotationsverdampfer eingeengt. Umkristallisation aus Diisopropylether/Petrolether ergibt 2-[2-Hydroxy-5-methyl-4-(pyrrol-1-yl)-phenyl]-propionsäure vom Smp. 73-74°.

Beispiel 23: Zu 81,1 g (0,5 Mol) 4-Methyl-2-(1-methyl-2-propenyl)-phenol werden bei Raumtemperatur unter Rühren während 1 Stunde 42,6 ml (0,6 Mol) Acetylchlorid getropft. Anschliessend wird das Reaktionsgemisch auf 100° erhitzt und während 2 Stunden bei dieser Temperatur belassen. Nach dem Abkühlen wird vorsichtig mit Wasser versetzt und mit Methylenchlorid extrahiert. Die organische Phase wird über Natriumsulfat getrocknet und eingedampft. Anschliessende Destillation des verbleibenden Rückstandes (64-70°/4 x $10^{-2}$ Torr) ergibt 4-Methyl-2-(1-methyl-2-propenyl)-phenylacetat als blassgelbes Oel.

Ein Gemisch aus 20,4 (0,1 Mol) 4-Methyl-2-(1-methyl-2-propenyl)-phenylacetat und 100 mg (0,4 mMol) Osmiumtetroxid in 300 ml Dioxan und 100 ml Wasser wird während 30 Minuten bei Raumtemperatur gerüht, anschliessend während 30 Minuten portionenweise mit 42,8 g (0,2 Mol) Natriumperiodat versetzt und eine Stunde nachgerührt. Der entstandene Niederschlag wird abfiltriert und mit Dioxan/Wasser (1:1) nachgewaschen. Die wässrig-organische Phase wird im Vakuum auf etwa 1 Drittel eingeengt und mit Methylenchlorid ausgezogen. Das nach dem Trocknen und Entfernen des Methylenchlorids erhaltene ölige Rohprodukt wird in 100 ml Aceton aufgenommen und durch halbstündiges Eintropfen einer Lösung von 7,2 g (72 mMol) Chromtrioxid und 6,2 ml konz. Schwefelsäure in 40 ml Wasser oxidiert. Anschliessend wird mit 3 ml Methanol und 200 ml Wasser versetzt, das Aceton im Vakuum entfernt, die wässrige Phase mit Ether extrahiert und die Etherlösung 3 mal mit 10%iger Natronlauge ausgezogen. Die alkalische wässrige Lösung wird während 3 Stunden bei Raumtemperatur stehengelassen, anschliessend mit konz. Salzsäure auf pH 3 gestellt und mit Ether extrahiert. Das nach dem Trocknen und Entfernen des Ethers erhaltene Oel wird während 2 Stunden mit 300 ml gesättigter methanolischer Salzsäure verrührt. Anschliessend wird das Methanol im Vakuum entfernt und der Rückstand zwischen Ether und verdünnter Natriumbicarbonatlösung verteilt. Das nach dem Trocknen und Eindampfen der organischen Phase erhaltene Rohprodukt wird mit Methylenchlorid an Kieselgel chromatographiert. Anschliessende Umkristallisation der reinen Fraktionen aus Methylenchlorid/Petrolether ergibt 2-(2-Hydroxy-5-methylphenyl)-propionsäuremethylester vom Smp. 104-106°.

Ein Gemisch aus 5,8 g (30 mMol) 2-(2-Hydroxy-5-methylphenyl)-propionsäuremethylester, 36,5 g (82 mMol) Bleitetraacetat und 150 ml Eisessig wird während 36 Stunden bei Raumtemperatur gerührt. Der Eisessig wird im Vakuum entfernt und der Rückstand mit 300 ml Wasser versetzt. Der entstandene Niederschlag wird abfiltriert und gründlich mit Ether gewaschen. Das Filtrat wird mit Ether ausgezogen. Die vereinigten Etherphasen werden über Natriumsulfat getrocknet und im Vakuum eingedampft. Das verbleibende rötliche Oel wird in 80 ml Dioxan aufgenommen, mit 8,7 ml (106 mMol) Pyrrolidin versetzt und während 5 Stunden unter Rückfluss gekocht. Das Dioxan wird im Vakuum entfernt und der Rückstand mit Methylenchlorid/Aceton an Kieselgel chromatographiert. Nach Umkristallisation der reinen Fraktionen aus Aceton erhält man 2-[2-Hydroxy-5-methyl-4-(pyrrolidin-1-yl)-phenyl]-propionsäurepyrrolidid vom Smp. 178-180°.

Beispiel 24: Ein Gemisch von 19,6 g (0,1 Mol) 4-Methyl-3-(2-methyl-3-oxobutyl)-maleinsäureanhydrid und 48,2 g Indoliniumbenzoat in 52 ml Benzol wird während 6 Stunden am Wasserabscheider unter Rückfluss erhitzt. Anschliessend wird das Benzol im Vakuum abgedampft und der Rückstand zwischen Aether und 1n Salzsäure verteilt. Die organische Phase wird mit gesättigter Natriumbicarbonatlösung gewaschen und nach dem Trocknen eingeengt. Das so erhaltene rohe 2-[5-Methyl-2-hydroxy-4-(indolin-1-yl)-phenyl]-propionsäure-indolinylamid schmilzt bei 176-178°.

Beispiel 25: 44 g 2-(4-Dibenzylamino-2-hydroxy-5-methyl-phenyl)-propionsäure-dibenzylamid werden in 450 ml Dioxan gelöst und mit 10 g Palladium auf Kohle (5%ig) bei Raumtemperatur und Normaldruck mit Wasserstoff reduziert. Anschliessend wird filtriert, das Filtrat zur Trockne eingedampft und der Rückstand aus Essigester umkristallisiert. Man erhält auf diese Weise das 2-(4-Amino-2-hydroxy-5-methyl-phenyl)-propionsäure-dibenzylamid vom Smp. 166-167°.

3,7 g (0,01 Mol) 2-(4-Amino-2-hydroxy-5-methyl-phenyl)-propionsäure-dibenzylamid werden in 20 ml Dioxan suspendiert und unter Rühren bei Raumtemperatur mit 2 ml 2,5-Dimethoxy-tetrahydrofuran und 1,4 ml 37%iger Salzsäure versetzt. Nach 30 Minuten wird das Lösungsmittel im Vakuum entfernt und der Rückstand zwischen Ether und Wasser verteilt. Die organische Phase wird mit gesättigter Natriumbicarbonatlösung gewaschen, getrocknet und zur Trockne eingedampft. Der Rückstand wird mit Methylenchlorid an Kieselgel chromatographiert. Umkristallisation der reinen Eluate aus Isopropylether ergibt 2-[2-Hydroxy-5-methyl-4-(pyrrol-1-yl)-phenyl]-propionsäure-dibenzylamid vom Smp. 150-151°.

Das Ausgangsmaterial kann wie folgt hergestellt werden: 59 g 4-Methyl-3-(2-methyl-3-oxo-butyl)-maleinsäureanhydrid und 240 g Dibenzylammoniumbenzoat werden in 1000 ml Benzol mit einem Wasserabscheider 48 Stunden am Rückfluss gekocht. Anschliessend wird im Vakuum zur Trockene eingedampft und der Rückstand über Kieselgel chromatographiert. Das erhaltene Oel kristallisiert aus Isopropylether. Man erhält so das 2-(4-Dibenzylamino-2-hydroxy-5-methyl-phenyl)-propionsäure-dibenzylamid vom Smp. 140-141°.

<u>Beispiel 26</u>: In analoger Weise wie im Beispiel 14 beschrieben erhält man die 2-[2-Hydroxy-5-methyl-6-(2,5-dimethyl-pyrrol-1-yl)-phenyl]-propionsäure.

Das Ausgangsmaterial kann wie folgt hergestellt werden:

5,3 g (0,03 Mol) 6-Amino-3,5-dimethyl-benzofuran-2(3H)-on, 4,1 g (0,037 Mol) Acetonylaceton, 50 ml Benzol und 0,5 ml Eisessig werden 14 Stunden unter Rückfluss erhitzt. Nach dem Abkühlen wird mit Wasser, gesättigter Natriumbicarbonatlösung und 1n Salzsäure gewaschen. Anschliessend wird das Benzol im Vakuum abgedampft und der Rückstand mit Methylenchlorid über Kieselgel chromatographiert. Nach Kristallisation der reinen Eluate erhält man 3,5-Dimethyl-6-(2,5-dimethyl-pyrrol-1-yl)-benzofuran-2(3H)-on vom Smp. 94-95°.

Beispiel 27: 3,0 g (0,01 Mol) 2-(5-Chloro-2-methoxy-4-morpholino-phenyl)-propionsäure werden in 20 ml 48%iger Bromwasserstoffsäure 1 Stunde unter Rückfluss erhitzt. Anschliessend wird im Vakuum zur Trockne eingedampft, der Rückstand in verdünnter Natronlauge gelöst, mit verdünnter Salzsäure auf pH 1-2 gestellt und mehrmals mit Ether extrahiert. Nach dem Trocknen und Abdampfen des Ethers erhält man die rohe Säure, die aus wenig Ether umkristallisiert werden kann. Auf diese Weise erhält man 2-(5-Chloro-2-hydroxy-4-morpholino-phenyl)-propionsäure vom Smp. 198-200°.

Beispiel 28: Tabletten, enthaltend 25 mg Wirkstoff, z.B. 2-(5-Chlor-2-hydroxy-4-morpholino-phenyl)-propionsäuremethylester oder ein Salz, z.B. das Hydrochlorid, davon, können folgendermassen hergestellt werden:

Bestandteile (für 1000 Tabletten):

| | |
|---|---|
| Wirkstoff | 25,0 g |
| Lactose | 100,7 g |
| Weizstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35°C getrocknet, durch ein Sieb mit 1,2 mm Maschenweite getrieben und zu beidseitig konkaven Tabletten von etwa 6 mm Durchmesser verpresst.

Beispiel 29: Kautabletten enthaltend 30 mg Wirkstoff, z.B. 2-(5-Chlor-2-hydroxy-4-(pyrrolidin-1-yl)-phenyl)-propionsäure-Natriumsalz oder ein Salz, z.B. das Hydrochlorid davon, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%ige Gelatinelösung | q.s. |

Herstellung: Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50°C getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 100 mm Durchmesser mit Bruchrille auf der Oberseite verpresst.

Beispiel 30: Tabletten enthaltend 100 mg Wirkstoff, z.B. 2-(5-Chlor-2-hydroxy-4-morpholino-phenyl)-propionsäure-Natriumsalz oder ein Salz, z.B. das Hydrochlorid, davon, können folgendermassen hergestellt werden:

Zusammensetzung (für 1000 Tabletten):

| | |
|---|---|
| Wirkstoff | 100,0 g |
| Lactose | 248,5 g |

| | |
|---|---|
| Maisstärke | 17,5 g |
| Polyethylenglykol 6000 | 5,0 g |
| Talkum | 15,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35°C getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Patentansprüche (für alle benannten Länder ausser Oesterreich)

1. Phenol-Derivate, insbesondere solche der allgemeinen Formel

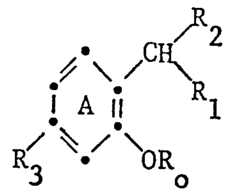

(I),

worin $R_o$ Wasserstoff oder einen Acylrest darstellt, $R_1$ Carboxy, verestertes Carboxy oder amidiertes Carboxy bedeutet, $R_2$ für Wasserstoff oder einen aliphatischen Rest steht, $R_3$ eine durch zwei einwertige Kohlenwasserstoffreste oder durch einen zweiwertigen Kohlenwasserstoffrest disubstituierte Aminogruppe darstellt und der aromatische Ring A zusätzlich substituiert sein kann, und ihre Salze und Isomeren.

2. Verbindungen der Formel (I) gemäss Anspruch 1, worin $R_o$ Wasserstoff, einen Niederalkanoyl- oder einen Arylniederalkanoylrest bedeutet, $R_1$ Carboxy, mit einem aliphatischen oder aromatischen Alkohol verestertes Carboxy, Carbamoyl oder mono- oder disubstituiertes Carbamoyl darstellt, $R_2$ einen gesättigten und unsubstituierten aliphatischen Rest bedeutet, $R_3$ eine durch zwei einwertige aliphatische Rest disubstituierte Aminogruppe oder eine durch einen zweiwertigen aliphatischen Rest disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_3$, unsubstituiert sein kann, und ihre Salze, insbesondere pharmazeutisch verwendbare Salze, und Isomeren.

3. Verbindungen der Formel (I) gemäss Anspruch 1, worin $R_o$ Wasserstoff, Niederalkanoyl oder Phenylniederalkanoyl bedeutet, wobei der Phenylrest ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederakanoyl und/oder Nitro substituiert oder unsubstituiert sein kann, oder Phenylniederalkanoyl bedeutet, welches sich von einer Phenylniederalkancarbonsäure der Formel I ableitet, wobei die Reste $R_2$ und $R_3$ sowie die Substituenten des Rings A die nachstehend angegebenen Bedeutungen haben und $R_o$ für Wasserstoff oder Niederalkanoyl steht, $R_1$ Carboxy, Niederalkoxycarbonyl, Hydroxyniederalkoxycarbonyl, Niederalkanoyloxy-niederalkoxycarbonyl, Phenylniederalkoxycarbonyl, Phenoxycarbonyl, Carbamoyl, N-Mono-, N,N-Diniederalkylcarbamoyl, N-Mono- oder N,N-Di-phenylniederalkylcarbamoyl, N-Mono-, N,N-Diphenylcarbamoyl, N-Niederalkyl-N-phenylniederalkyl-carbamoyl, N-Niederalkyl-N-phenyl-carbamoyl, N-Phenylniederalkyl-N-phenyl-carbamoyl, Niederalkylen-carbamoyl, Niederalkenyl-carbamoyl, durch Monoaza, N'-Niederalkylmonoaza, Monooxa oder Monothia unterbrochenes Niederalkylencarbamoyl, Niederalkenylencarbamoyl darstellt, wobei Phenyl bzw. Phenoxy jeweils ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und wobei Niederalkylen bzw. Niederalkenylen unverzweigt oder verzweigt und/oder ein oder zwei ortho-anellierte Benzosysteme aufweisen können, $R_2$ Wasserstoff oder Niederalkyl bedeutet und $R_3$ einerseits N,N-Di-niederalkyl-amino, N-Cycloniederalkyl-N-niederalkyl-amino, N-Niederalkyl-N-phenylniederalkyl-amino, N,N-Di-cycloniederalkylniederalkyl-amino, N-Cycloniederalkylniederalkyl-N-phenylniederalkyl-amino oder N,N-Di-phenylniederalkyl-amino bedeutet oder $R_3$ andererseits jeweils 5- bis 8-gliedriges Niederalkylenamino, Niederalkenylenamino, durch Monoaza, N'-Niederalkylmonoaza, Monooxa oder Monothia unterbrochenes Niederalkylenamino, durch Monoaza, N'-Niederalkylmonoaza, Monooxa oder Monothia unterbrochenes Niederalkenylenamino oder ein oder zwei ortho-anellierte Benzosysteme aufweisendes Niederalkylen- bzw. Niederalkenylen-amino darstellt, wobei Niederalkylen bzw. Niederalkenylen unverzweigt

oder verzweigt sein und 4 bis 14, insbesondere 4 bis 7, C-Atome aufweisen kann und/oder ein oder zwei ortho-anellierte Benzosysteme aufweisen kann und Phenyl oder Benzo jeweils ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und der aromatische Ring A ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder, bis auf $R_3$, unsubstituiert sein kann, und ihre Salze, insbesondere pharmazeutisch verwendbare Salze, und Isomeren.

4. Verbindungen der Formel (I) gemäss Anspruch 1, worin $R_o$ Wasserstoff, Niederalkanoyloxy oder im Phenylteil unsubstituiertes bzw. ein- oder mehrfach durch Niederalkyl, Niederalkoxy, Hydroxy, Halogen, Niederalkanoyloxy und/oder Trifluormethyl substituiertes Phenylniederalkanoyloxy bedeutet, $R_1$ Carboxy, mit einem Niederalkanol, einem durch Hydroxy, Niederalkoxy, Niederalkanoyloxy oder im Phenylteil unsubstituiertes oder Niederalkyl, Niederalkoxy, Hydroxy, Halogen, Niederalkanoyloxy und/oder Trifluormethyl enthaltendes Phenyl substituiertes Niederalkanol, mit einem unsubstituierten oder Niederalkyl, Niederalkoxy, Hydroxy, Halogen, Niederalkanoyloxy und/oder Trifluormethyl enthaltenden Phenol verestertes Carboxy, Carbamoyl, durch Niederalkyl, im Phenylteil unsubstituiertes bzw. Niederalkyl, Niederalkoxy, Hydroxy, Halogen, Niederalkanoyloxy und/oder Trifluormethyl aufweisendes Phenylniederalkyl monosubstituiertes Carbamoyl, durch Niederalkyl, durch im Phenylteil unsubstituiertes bzw. Niederalkyl, Niederalkoxy, Hydroxy, Halogen, Niederalkanoyloxy und/oder Trifluormethyl aufweisendes Phenylniederalkyl, durch Niederalkylen oder durch Monoaza, N-alkyliertes Monoaza, Monooxa bzw. Monothia unterbrochenes Niederalkylen disubstituiertes Carbamoyl darstellt, $R_2$ Wasserstoff oder

Niederalkyl bedeutet, $R_3$ eine durch Niederalkyl, durch im Cycloalkylteil 3- bis 7-gliedriges Cycloalkyl-niederalkyl, durch im Phenylteil unsubstituiertes bzw. Niederalkyl, Niederalkoxy, Hydroxy, Halogen, Niederalkanoyloxy und/oder Trifluormethyl enthaltendes Phenylniederalkyl, durch Niederalkylen, durch Niederalkenylen, durch Aza, N-Niederalkylaza, Oxa oder Thia unterbrochenes Niederalkylen oder durch Aza oder N-Niederalkylaza unterbrochenes Niederalkenylen disubstituierte Aminogruppe darstellt und der aromatische Ring A zusätzlich durch Niederalkyl, Niederalkoxy, Hydroxy, Halogen Niederalkanoyloxy, 3- oder 4-gliedriges Alkylen und/oder Trifluormethyl substituiert sein kann, und ihre Salze, insbesondere pharmazeutisch verwendbare Salze, und Isomeren.

5. Verbindungen gemäss Anspruch 1 der Formel

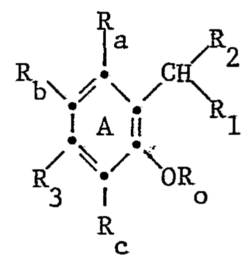

(Ia),

worin $R_o$ Wasserstoff oder Niederalkanoyl bedeutet, $R_1$ Carboxy, Niederalkoxycarbonyl, Niederalkylencarbamoyl oder Oxa-niederalkylen-carbamoyl darstellt, $R_2$ Wasserstoff oder Niederalkyl, bedeutet, $R_3$ Di-niederalkyl-amino, Di-cycloalkylniederalkylamino, Di-phenylniederalkyl-amino, jeweils 5- bis 8-gliedriges Niederalkylenamino, Niederalkenylenamino, Monoaza-niederalkylen-amino, N'-Niederalkylmonoaza-niederalkylenamino, Monooxaniederalkylen-amino, Monothianiederalkylenamino, Monoaza-niederalkenylen-amino oder N'-Niederalkylmonoaza-niederalkenylenamino darstellt und $R_a$, $R_b$, $R_c$ unabhängig voneinander Wasserstoff, Niederalkyl, Niederalkoxy, Hydroxy, Halogen, Niederalkanoyloxy, 3- bzw. 4-gliedriges Alkylen oder Trifluormethyl bedeuten und ihre Salze und Isomeren.

6. Verbindungen der Formel (Ia) gemäss Anspruch 1, worin $R_o$ Wasserstoff oder Niederalkanoyl bedeutet oder Phenylniederalkanoyl bedeutet, welches sich von einer Phenylniederalkancarbonsäure der Formel (I) ableitet, wobei die Reste $R_2$, $R_3$, $R_a$, $R_b$ und $R_c$ die nachstehend angegebenen Bedeutungen haben und $R_o$ Wasserstoff ist, $R_1$ Carboxy, Niederalkoxycarbonyl, Niederalkanoyloxy-niederalkoxycarbonyl, Carbamoyl, N,N-Di-phenylniederalkyl-carbamoyl, Niederalkylencarbamoyl, oder durch Monoxa unterbroches Niederalkylencarbamoyl, darstellt, $R_2$ Wasserstoff oder Niederalkyl bedeutet, $R_3$ einerseits N,N-Di-phenylniederalkyl-amino darstellt oder $R_3$ andererseits jeweils 5- bis 8-gliedriges Niederalkylenamino, Niederalkenylenamino, durch Monooxa unterbrochenes Niederalkylenamino oder ein ortho-anelliertes Benzosystem aufweisendes Niederalkylenamino bzw. Niederalkenylamino bedeutet, und/oder $R_a$, $R_b$ und $R_c$ unabhängig voneinander Wasserstoff, Niederalkyl, oder Halogen darstellen, und ihre Salze, insbesondere pharmazeutisch verwendbare Salze, und Isomeren.

7. Verbindungen der Formel (Ia) gemäss Anspruch 1, worin $R_o$ Wasserstoff oder Niederalkanoyl bedeutet, $R_1$ Carboxy, Niederalkoxycarbonyl, 5- bis 8-gliedriges Niederalkylencarbamoyl, 5- bis 8-gliedriges Monooxaniederalkylencarbamoyl darstellt, $R_2$ Wasserstoff oder Niederalkyl darstellt, $R_3$ Di-niederalkyl-amino, 5- bis 8-gliedriges Niederalkylenamino, 5- bis 8-gliedriges Niederalkenylenamino oder 5- bis 8-gliedriges Monooxa-niederalkylenamino bedeutet und $R_a$ sowie $R_c$ Wasserstoff bedeuten und $R_b$ Halogen bedeutet und ihre Salze und Isomeren.

8. Verbindungen der Formel (Ia) gemäss Anspruch 1, worin $R_o$ Wasserstoff oder Niederalkanoyl mit bis und mit 5 C-Atomen bedeutet, $R_1$ Carboxy oder Niederalkoxycarbonyl mit bis und mit 5 C-Atomen darstellt, $R_2$ Niederalkyl mit bis und mit 4 C-Atomen bedeutet, $R_3$ 5- bis 7-gliedriges Niederalkylenamino, Morpholin-4-yl oder Pyrrol-1-yl darstellt, $R_a$ und $R_c$ jeweils Wasserstoff bedeuten und $R_b$ Niederalkyl mit bis und mit 4 C-Atomen, oder Halogen bis und mit Atomnummer 35 darstellt, und ihre Salze, insbesondere pharmazeutisch verwendbare Salze, und Isomeren.

9. Verbindungen der Formel (Ia) gemäss Anspruch 1, worin $R_o$ Niederalkanoyl mit bis und mit 5 C-Atomen darstellt, $R_1$ Niederalkoxycarbonyl mit bis und mit 5 C-Atomen bedeutet, $R_2$ Niederalkyl mit bis und mit 4 C-Atomen darstellt, $R_3$ Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt und ihre Salze und Isomeren.

10. 2-(5-Chlor-2-hydroxy-4-morpholino-phenyl)-propionsäure oder ein Salz oder Isomeres davon.

11. 2-[2-Hydroxy-5-methyl-4-(pyrrolidin-1-yl)-phenyl]-propionsäure-pyrrolidid oder ein Salz oder Isomeres davon.

12. 2-(2-Hydroxy-5-methyl-4-morpholino-phenyl)-propionsäuremorpholid oder ein Salz oder Isomeres davon.

13. 2-(5-Chlor-2-hydroxy-4-morpholino-phenyl)-propionsäuremethylester oder ein Salz oder Isomeres davon.

14. 2-(5-Chlor-2-hydroxy-4-morpholino-phenyl)-propionsäure-morpholid oder ein Salz oder Isomeres davon.

15. 2-(2-Acetoxy-5-chlor-4-morpholino-phenyl)-propionsäure-methylester oder ein Salz oder Isomeres davon.

16. 5-Chlor-2-hydroxy-4-(piperidin-1-yl)-phenylessigsäure oder ein Salz davon.

17. 2-(5-Chlor-2-hydroxy-4-(pyrrolidin-1-yl)-phenyl-propionsäure-Natriumsalz oder ein Isomeres davon.

18. 2-(5-Chlor-2-hydroxy-4-morpholinophenyl)-propionsäure-Natriumsalz oder ein Isomeres davon.

19. 2-(4-Dibenzylamino-2-hydroxy-5-methyl-phenyl)-propionsäure-dibenzylamid oder ein Salz oder Isomeres davon.

20. 2-(4-Dibenzylamino-2-hydroxy-5-methyl-phenyl)-propionsäure oder ein Salz oder Isomeres davon.

21. 2-[2-Hydroxy-5-methyl-4-(pyrrol-1-yl)-phenyl]-propionsäure oder ein Salz oder Isomeres davon.

22. 2-[2-Acetoxy-5-methyl-4-(pyrrol-1-yl)-phenyl]-propionsäuremethylester oder ein Salz oder Isomeres davon.

23. 2-[2-Acetoxy-5-methyl-4-(pyrrolidin-1-yl)-phenyl]-propionsäuremethylester oder ein Salz oder Isomeres davon.

24. 2-(2-Acetoxy-5-brom-4-morpholino-phenyl)-propionsäuremethylester oder ein Salz oder Isomeres davon.

25. 2-[2-Hydroxy-5-methyl-2-(pyrrolidin-1-yl)-phenyl]-propionsäurepyrrolidid oder ein Salz oder Isomeres davon.

26. 2-[5-Methyl-2-hydroxy-4-(indolin-1-yl)-phenyl]-propionsäureindolinylamid oder ein Salz oder Isomeres davon.

27. 2-[2-Hydroxy-5-methyl-4-(pyrrol-1-yl)-phenyl]-propionsäure-dibenzylamid oder ein Salz oder Isomeres davon.

28. Verbindung gemäss einem der Ansprüche 2, 3, 6, 8 und 21-27 mit antiinflammatorischen und/oder analgetischer Wirkung.

29. Verbindung gemäss einem der Ansprüche 1, 4, 5, 7 und 9-20 mit antiinflammatorischer und/oder analgetischer Wirkung.

30. Verbindung gemäss einem der Ansprüche 1-27 mit Wirkung als Lichtschutzmittel.

31. Die in den Beispielen 14-27 genannten neuen Verbindungen.

32. Die in den Beispielen 1-13 genannten neuen Verbindungen.

33. Verbindungen gemäss einem der Ansprüche 1-29 zur therapeutischen Behandlung des tierischen und menschlichen Körpers.

34. Pharmazeutische Präparate, enthaltend eine Verbindung gemäss einem der Ansprüche 1-29 neben üblichen pharmazeutischen Hilfs- und Trägerstoffen.

35. Verfahren zur Herstellung von Phenol-Derivaten, insbesondere solche der allgemeinen Formel

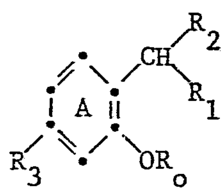

(I),

worin $R_o$ Wasserstoff oder einen Acylrest darstellt, $R_1$ Carboxy, verestertes Carboxy oder amidiertes Carboxy bedeutet, $R_2$ für Wasserstoff oder einen aliphatischen Rest steht, $R_3$ eine durch zwei einwertige Kohlenwasserstoffreste oder durch einen zweiwertigen Kohlenwasserstoffrest disubstituierte Aminogruppe darstellt und der aromatische Ring A zusätzlich substituiert sein kann, und ihre Salze und Isomeren, dadurch gekennzeichnet, dass man Verbindungen der Formel

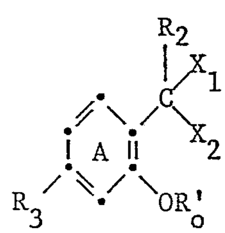

(II),

worin $X_1$ Wasserstoff ist, $X_2$ von $R_1$ verschiedenes, funktionell abgewandeltes Carboxy bedeutet und $R_o'$ die Bedeutung von $R_o$ hat oder worin $X_1$ Wasserstoff ist und $X_2$ gemeinsam mit $R_o'$ die Gruppe $>C=O$ bilden oder worin $X_1$ gemeinsam mit $X_2$ die Grupe $=C=O$ oder die Gruppe $=C(Hal)_2$ bilden und Hal jeweils für Halogen, steht und $R_o'$ die Bedeutung von $R_o$ hat, oder deren Salze mit Solvolysemitteln behandelt, oder in Verbindungen der Formel

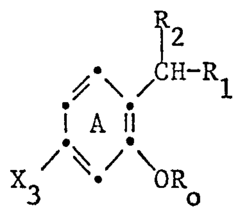

(III)

oder deren Salzen, worin $X_3$ einen in $R_3$ überführbaren Rest darstellt, $X_3$ in $R_3$ überführt, oder in Verbindungen der Formel

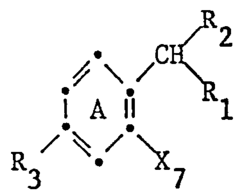

(IV),

worin $X_7$ einen in die Gruppe $-OR_o$ überführbaren Rest darstellt, den Rest $X_7$ in die Gruppe $-OR_o$ überführt, oder Verbindungen der Formel

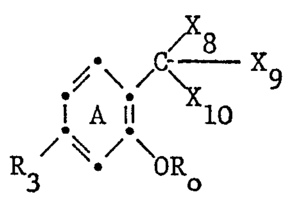

(V)

oder deren Salze, worin $X_8$ und $X_9$ jeweils Carboxy bedeuten, und $X_{10}$ die Bedeutung von $R_2$ hat, worin $X_8$ die Bedeutung von $R_1$ und $X_9$ die von $R_2$ hat und $X_{10}$ Hydroxy, funktionell abgewandeltes Hydroxy, durch einen Kohlenwasserstoffrest·substituiertes Mercapto, oder sekundäres Amino darstellt, worin $X_8$ die Bedeutung von $R_1$ hat und $X_9$ und $X_{10}$ gemeinsam Oxo, Thioxo oder gegebenenfalls substituiertes Hydrazono bedeuten, oder worin $X_8$ die Bedeutung von $R_1$ hat und $X_9$ und $X_{10}$ gemeinsam die Gruppen = $R_2'$ oder eine tautomere Form hierzu bilden und $R_2'$ für einen zweiwertigen aliphatischen Rest steht, durch Reduktion in entsprechende Verbindungen der Formel (I) überführt, oder in Verbindungen der Formel

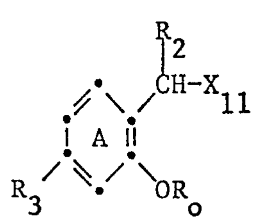

(VI)

oder deren Salzen, worin $X_{11}$ ein oxidativ in $R_1$ überführbarer Rest bedeutet, $X_{11}$ oxidativ in $R_1$ überführt, oder eine Verbindung der Formel

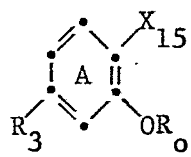

(VIII)

oder ein Salz davon, worin $X_{15}$ ein in die Gruppe der Formel $-CH(R_2)-R_1$ überführbarer Rest bedeutet, $X_{15}$ durch Umlagerung in die Gruppe der Formel $-CH(R_2)-R_1$ überführt, oder in einer Verbindung der Formel

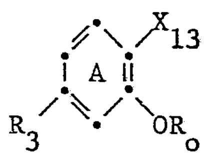

(VII),

worin $X_{13}$ einen in die Gruppe der Formel $-CH(R_2)-R_1$ (VIIa) überführbaren Rest bedeutet, oder einem Salz oder Isomeren davon der Rest $X_{13}$ in die Gruppe der Formel (VIIa) überführt wird und/oder, wenn erwünscht, ein verfahrensgemäss erhältliches Salz in die freie Verbindung oder in ein anderes Salz überführt, eine verfahrensgemäss erhältliche freie Verbindung in ein Salz oder in eine andere freie Verbindung überführt und/oder gewünschtenfalls ein verfahrensgemäss erhältliches Isomerengemisch in seine Komponenten auftrennt.

36. Verwendung von Verbindungen gemäss einem der Ansprüche 1-29 in einem Verfahren zur Behandlung entzündlicher und/oder rheumatischer Erkrankungen und/oder von Schmerzzuständen.

37. Das Verfahren der Beispiele 1-27 und die danach erhältlichen neuen Verbindungen.

38. Die in dem Verfahren gemäss Anspruch 35 verwendeten neuen Ausgangsstoffe und Zwischenprodukte.

Patentansprüche: (für Oesterreich)

1. Verfahren zur Herstellung von Phenol-Derivaten, insbesondere solche der allgemeinen Formel

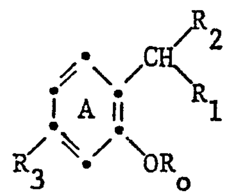

(I),

worin $R_o$ Wasserstoff oder einen Acylrest darstellt, $R_1$ Carboxy, verestertes Carboxy oder amidiertes Carboxy bedeutet, $R_2$ für Wasserstoff oder einen aliphatischen Rest steht, $R_3$ eine durch zwei einwertige Kohlenwasserstoffreste oder durch einen zweiwertigen Kohlenwasserstoffrest disubstituierte Aminogruppe darstellt und der aromatische Ring A zusätzlich substituiert sein kann, und ihre Salze und Isomeren, dadurch gekennzeichnet, dass man Verbindungen der Formel

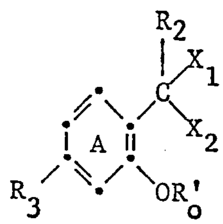

(II),

worin $X_1$ Wasserstoff ist, $X_2$ von $R_1$ verschiedenes, funktionell abgewandeltes Carboxy bedeutet und $R'_o$ die Bedeutung von $R_o$ hat oder worin $X_1$ Wasserstoff ist und $X_2$ gemeinsam mit $R'_o$ die Gruppe >C=O bilden oder worin $X_1$ gemeinsam mit $X_2$ die Grupe =C=O oder die Gruppe $=C(Hal)_2$ bilden und Hal jeweils für Halogen, steht und $R'_o$ die Bedeutung von $R_o$ hat, oder deren Salze mit Solvolysemitteln behandelt, oder in Verbindungen der Formel

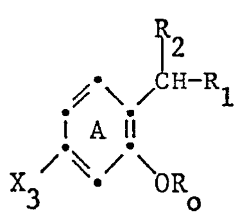

(III)

oder deren Salzen, worin $X_3$ einen in $R_3$ überführbaren Rest darstellt, $X_3$ in $R_3$ überführt, oder in Verbindungen der Formel

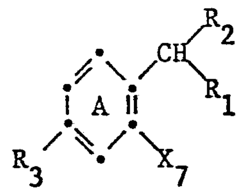

(IV),

worin $X_7$ einen in die Gruppe $-OR_o$ überführbaren Rest darstellt, den Rest $X_7$ in die Gruppe $-OR_o$ überführt, oder Verbindungen der Formel

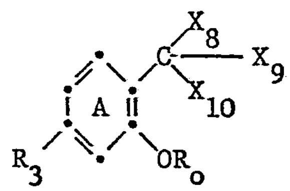

(V)

oder deren Salze, worin $X_8$ und $X_9$ jeweils Carboxy bedeuten, und $X_{10}$ die Bedeutung von $R_2$ hat, worin $X_8$ die Bedeutung von $R_1$ und $X_9$ die von $R_2$ hat und $X_{10}$ Hydroxy, funktionell abgewandeltes Hydroxy, durch einen Kohlenwasserstoffrest substituiertes Mercapto, oder sekundäres Amino darstellt, worin $X_8$ die Bedeutung von $R_1$ hat und $X_9$ und $X_{10}$ gemeinsam Oxo, Thioxo oder gegebenenfalls substituiertes Hydrazono bedeuten, oder worin $X_8$ die Bedeutung von $R_1$ hat und $X_9$ und $X_{10}$ gemeinsam die Gruppen $= R'_2$ oder eine tautomere Form hierzu bilden und $R'_2$ für einen zweiwertigen aliphatischen Rest steht, durch Reduktion in entsprechende Verbindungen der Formel (I) überführt, oder in Verbindungen der Formel

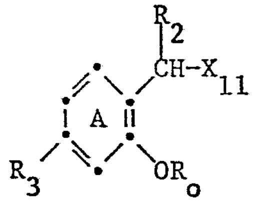

(VI)

oder deren Salzen, worin $X_{11}$ ein oxidativ in $R_1$ überführbarer Rest bedeutet, $X_{11}$ oxidativ in $R_1$ überführt, oder eine Verbindung der Formel

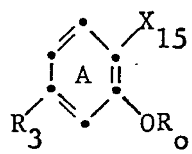

(VIII)

oder ein Salz davon, worin $X_{15}$ ein in die Gruppe der Formel $-CH(R_2)-R_1$ überführbarer Rest bedeutet, $X_{15}$ durch Umlagerung in die Gruppe der Formel $-CH(R_2)-R_1$ überführt, oder in einer Verbindung der Formel

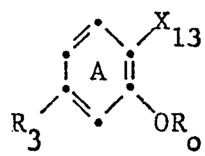

(VII),

worin $X_{13}$ einen in die Gruppe der Formel $-CH(R_2)-R_1$ (VIIa) überführbaren Rest bedeutet, oder einem Salz oder Isomeren davon der Rest $X_{13}$ in die Gruppe der Formel (VIIa) überführt wird und/oder, wenn erwünscht, ein verfahrensgemäss erhältliches Salz in die freie Verbindung oder in ein anderes Salz überführt, eine verfahrensgemäss erhältliche freie Verbindung in ein Salz oder in eine andere freie Verbindung überführt und/oder gewünschtenfalls ein verfahrensgemäss erhältliches Isomerengemisch in seine Komponenten auftrennt.

2. Verfahren nach Anspruch 1 zur Herstellung von Phenol-Derivaten, insbesondere solche der allgemeinen Formel

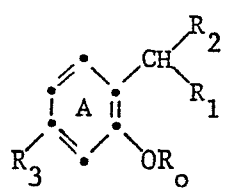

(I),

worin $R_o$ Wasserstoff oder einen Acylrest darstellt, $R_1$ Carboxy, verestertes Carboxy oder amidiertes Carboxy bedeutet, $R_2$ für Wasserstoff oder einen aliphatischen Rest steht, $R_3$ eine durch zwei einwertige Kohlenwasserstoffreste oder durch einen zweiwertigen Kohlenwasserstoffrest disubstituierte Aminogruppe darstellt und der aromatische Ring A zusätzlich substituiert sein kann, und ihre Salze und Isomeren, dadurch gekennzeichnet, dass man Verbindungen der Formel

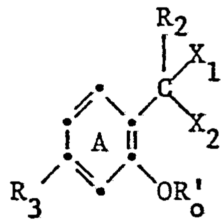

(II),

worin $X_1$ Wasserstoff ist, $X_2$ von $R_1$ verschiedenes, funktionell abgewandeltes Carboxy bedeutet und $R_o'$ die Bedeutung von $R_o$ hat oder worin $X_1$ Wasserstoff ist und $X_2$ gemeinsam mit $R_o'$ die Gruppe >C=O bilden oder worin $X_1$ gemeinsam mit $X_2$ die Gruppe =C=O bilden und $R_o'$ die Bedeutung von $R_o$ hat, oder deren Salze mit Solvolysemitteln behandelt oder in Verbindung der Formel

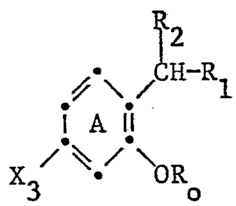

(III)

oder deren Salzen, worin $X_3$ einen in $R_3$ überführbaren Rest darstellt, $X_3$ in $R_3$ überführt oder zur Herstellung von Verbindungen der Formel (I) gemäss Anspruch 1, worin $R_o$ Wasserstoff ist, dadurch gekennzeichnet, dass man in Verbindungen der Formel

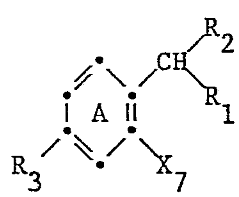

(IV),

worin $X_7$ einen in die Gruppe $-OR_o$ überführbaren Rest darstellt, den Rest $X_7$ in die Gruppe $-OR_o$ überführt oder zur Herstellung von Verbindungen der Formel (I) gemäss Anspruch 1, dadurch gekennzeichnet, dass man Verbindungen der Formel

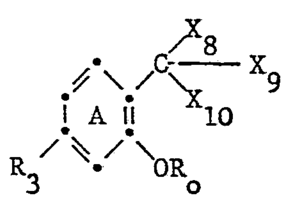

(V)

oder deren Salze, worin $X_8$ und $X_9$ jeweils Carboxy bedeuten, und $X_{10}$ die Bedeutung von $R_2$ hat, worin $X_8$ die Bedeutung von $R_1$ und $X_9$ die von $R_2$ hat und $X_{10}$ Hydroxy, funktionell abgewandeltes Hydroxy oder sekundäres Amino darstellt oder worin $X_8$ die Bedeutung von $R_1$ hat und $X_9$ und $X_{10}$ geminsam die Gruppen $= R_2'$ oder eine tautomere Form hierzu bilden und $R_2'$ für einen zweiwertigen aliphatischen Rest steht, durch Reduktion in entsprechende Verbindungen der Formel (I) überführt oder zur Herstellung von Verbindungen der Formel (I) gemäss Anspruch 1, worin $R_1$ Carboxy oder verestertes Carboxy bedeutet, dadurch gekennzeichnet, dass man in Verbindungen der Formel

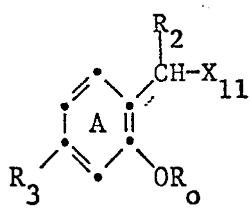

(VI)

oder deren Salzen, worin $X_{11}$ ein oxidativ in $R_1$ überführbarer Rest bedeutet, $X_{11}$ oxidativ in $R_1$ überführt oder zur Herstellung von Verbindungen der Formel (I) gemäss Anspruch 1, dadurch gekennzeichnet, dass in einer Verbindung der Formel

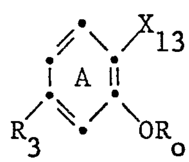

(VII),

worin $X_{13}$ einen in die Gruppe der Formel $-CH(R_2)-R_1$ (VIIa) überführbaren Rest bedeutet, oder einem Salz oder Isomeren davon der Rest $X_{13}$ in die Gruppe der Formel (VIIa) überführt wird oder zur Herstellung von Verbindungen der Formel (I) gemäss Anspruch 1, worin $R_1$ Niederalkoxycarbonyl bedeutet und $R_2$ Wasserstoff oder einen aliphatischen Rest darstellt bzw. worin $R_1$ amidiertes Carboxy bzw. ein entsprechendes Ammoniumcarboxylat und $R_2$ Wasserstoff ist, dadurch gekennzeichnet, dass man eine Verbindung der Formel

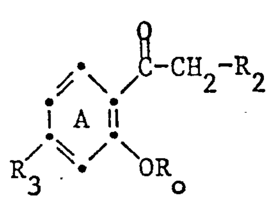

(VIII)

oder ein Salz davon, oxidativ umlagert und/oder, wenn erwünscht, ein verfahrensgemäss erhältliches Salz in die freie Verbindung oder in ein anderes Salz überführt, eine verfahrensgemäss erhältliche freie Verbindung in eine andere freie Verbindung oder in ein Salz überführt und/ oder gewünschtenfalls ein verfahrensgemäss erhältliches Isomerengemisch in seine Komponenten auftrennt.

3. Verfahren nach Anspruch 2, zur Herstellung von Verbindungen der Formel

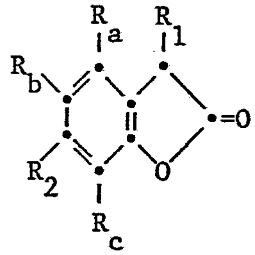

(Ia),

worin $R_1$ Methylen bedeutet, $R_a$, $R_b$ und $R_c$ Wasserstoff oder Niederalkyl bedeuten oder $R_a$ und $R_b$ gemeinsam 3- oder 4-gliedriges Alkylen darstellen, $R_c$ die vorstehende Bedeutung haben und $R_2$ die in Anspruch 1 angegebene Bedeutung hat, und deren Salze und Isomeren, dadurch gekennzeichnet, dass man Verbindungen der Formel

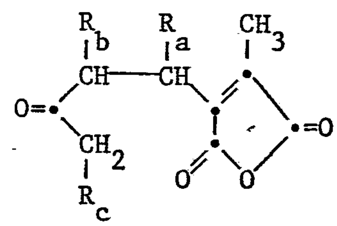

(IIb)

mit Aminen der Formel $R_2$-H (IIc) oder deren Salzen umsetzt.

4. Verfahren nach Anspruch 2, dadurch gekennzeichnet, dass man Verbindungen der Formel (II), worin $X_1$ Wasserstoff ist, $X_2$ von $R_1$ verschiedenes funktionell abgewandeltes Carboxy bedeutet und $R'_o$ die Bedeutung von $R_o$ hat und worin $X_1$ Wasserstoff ist und $X_2$ gemeinsam mit $R'_o$ die Gruppe >C=O bilden, mit einem entsprechenden Solvolysemittel solvolysiert.

5. Verfahren nach Anspruch 1, dadurch gekennzeichnet, dass man eine Verbindung der Formel (III), worin $X_3$ eine Gruppe der Formel $-NH-A_2-X_5$ bedeutet und $A_2$ einen zweiwertigen Kohlenwasserstoffrest darstellt, der auch durch Aza, N-Niederalkylaza, Oxa oder Thia unterbrochen sein kann, und $X_5$ Hydroxy oder reaktionsfähiges verestertes Hydroxy bedeutet, in Gegenwart eines Kondensationsmittels umsetzt, oder in eine Verbindung der Formel (III), worin $X_3$ Amino ist, durch Umsetzung mit einem 2,5-Diniederalkoxy-tetrahydrofuran oder durch Umsetzung mit 2-Buten-1,4-diol oder einem reaktionsfähig veresterten Derivat davon, erforderlichenfalls in Gegenwart eines katalytischen Mittels, zum Pyrrolin-1-yl und Dehydrierung desselben in Gegenwart eines Dehydrierungsmittels den Pyrrolring $R_3$ einführt oder in eine Verbindung der Formel (III), worin $X_5$ für einen durch $R_3$ ersetzbaren Rest steht und der gegebenenfalls in Nachbarposition von $X_3$ mindestens einen Substituenten mit -I- oder M-Effekt aufweist, nucleophile Amine $R_2$-H einführt.

6. Verfahren nach Anspruch 1, dadurch gekennzeichnet, dass man in einer Verbindung der Formel (IV), worin $X_7$ für verethertes Hydroxy steht, dieses durch Etherspaltung in Hydroxy $-OR_o$ überführt.

7. Verfahren nach Anspruch 1, dadurch gekennzeichnet, dass man in einer Verbindung der Formel (V), worin $X_8$ und $X_9$ jeweils Carboxy bedeuten, decarboxyliert, oder in einer Verbindung der Formel (V), worin $X_{10}$ Hydroxy, funktionell abgewandeltes Hydroxy, Alkylthio, Dialkylamino oder im Phenylteil jeweils gegebenenfalls substituiertes Diphenyl-sulfamoyl bedeutet oder worin $X_9$ und $X_{10}$ gemeinsam Oxo, Thioxo oder Hydrazono bedeuten, mit Hilfe eines Reduktionsmittels die entsprechende Gruppe der Formel 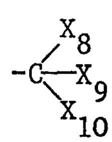 in die Gruppe der Formel $>CR_1(R_2)$ überführt.

8. Verfahren nach Anspruch 1, dadurch gekennzeichnet, dass man Verbindungen der Formel (VI), worin $X_{11}$ Hydroxymethyl, Formyl oder eine Gruppe der Formel $-CH=CH-X_{14}$, worin $X_{14}$ Wasserstoff oder ein aliphati-

scher Rest ist, mit Hilfe eines Oxidationsmittels oxidiert.

9. Verfahren nach Anspruch 1, dadurch gekennzeichnet, dass man Verbindungen der Formel (VIII), worin $X_{15}$ für die Grüppe der Formel $-CO-CH_2-R_2$ steht und $R_2$ Wasserstoff darstellt, analog der Willgerodt-Kindler-Reaktion zu solchen Verbindungen der Formel (I) umlagert, worin $R_1$ amidiertes Carboxy oder ein entsprechendes Thiocarbamoyl oder Ammoniumcarboxylat bedeutet und $R_2$ für Wasserstoff steht.

10. Verfahren nach Anspruch 1, dadurch gekennzeichnet, dass man von einer auf irgendeiner Stufe des Verfahrens als Zwischenstufe erhältlichen Verbindung ausgeht und die fehlenden Schritte durchführt und einen Ausgangsstoff in Form eines Salzes, Isomeren und/oder Racemates bzw. Antipoden verwendet oder insbesondere unter den Reaktionsbedingungen bildet.

11. Verfahren nach einem der Ansprüche 1 und 5-10, dadurch gekennzeichnet, dass man Verbindungen der Formel (I), worin $R_o$ Wasserstoff, einen Niederalkanoyl- oder einen Arylniederalkanoylrest bedeutet, $R_1$ Carboxy, mit einem aliphatischen oder aromatischen Alkohol verestertes Carboxy, Carbamoyl oder mono- oder disubstituiertes Carbamoyl darstellt, $R_2$ einen gesättigten und unsubstituierten aliphatischen Rest bedeutet, $R_3$ eine durch zwei einwertige aliphatische Rest disubstituierte Aminogruppe oder eine durch einen zweiwertigen aliphatischen Rest disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_3$, unsubstituiert sein kann, und ihre Salze, insbesondere pharmazeutisch verwendbare Salze, und Isomeren herstellt.

12. Verfahren nach einem der Ansprüche 1 und 5-10, dadurch gekennzeichnet, dass man Verbindungen der Formel (I),

worin $R_o$ Wasserstoff, Niederalkanoyl oder Phenylniederalkanoyl bedeutet, wobei der Phenylrest ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, oder Phenylniederalkanoyl bedeutet, welches sich von einer Phenylniederalkancarbonsäure der Formel (I) ableitet, wobei die Reste $R_2$ und $R_3$ sowie die Substituenten des Rings A die nachstehend angegebenen Bedeutungen haben und $R_o$ für Wasserstoff oder Niederalkanoyl steht, $R_1$ Carboxy, Niederalkoxycarbonyl, Hydroxyniederalkoxycarbonyl, Niederalkanoyl-oxy-niederalkoxycarbonyl, Phenylniederalkoxycarbonyl, Phenoxycarbonyl, Carbamoyl, N-Mono-, N,N-Diniederalkylcarbamoyl, N-Mono- oder N,N-Di-phenylniederalkylcarbamoyl, N-Mono-, N,N-Diphenylcarbamoyl, N-Niederalkyl-N-phenylniederalkyl-carbamoyl, N-Niederalkyl-N-phenyl-carbamoyl, N-Phenylniederalkyl-N-phenyl-carbamoyl, Niederalkylen-carbamoyl, Niederalkenyl-carbamoyl, durch Monoaza, N'-Niederalkylmonoaza, Monooxa oder Monothia unterbrochenes Niederalkylencarbamoyl, Niederalkenylencarbamoyl darstellt, wobei Phenyl bzw. Phenoxy jeweils ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und wobei Niederalkylen bzw. Niederalkenylen unverzweigt oder verzweigt und/oder ein oder zwei ortho-anellierte Benzosysteme aufweisen können, $R_2$ Wasserstoff oder Niederalkyl bedeutet und $R_3$ einerseits N,N-Di-niederalkyl-amino, N-Cycloniederalkyl-N-niederalkyl-amino, N-Niederalkyl-N-phenylniederalkyl-amino, N,N-Di-cycloniederalkylniederalkyl-amino, N-Cycloniederalkylniederalkyl-N-phenylniederalkyl-amino oder N,N-Di-phenylniederalkyl-amino bedeutet oder $R_3$ andererseits jeweils 5- bis 8-gliedriges Niederalkylenamino, Niederalkenylenamino, durch Monoaza, N'-Niederalkylmonoaza, Monooxa oder Monothia unterbrochenes Niederalkylenamino, durch Monoaza, N'-Niederalkylmonoaza, Monooxa oder Monothia unterbrochenes Niederalkenylenamino oder ein oder zwei ortho-anellierte Benzosysteme aufweisendes Niederalkylen- bzw. Niederalkenylen-amino darstellt, wobei Niederalkylen bzw. Niederalkenylen unverzweigt oder verzweigt sein und 4 bis 14, insbesondere 4 bis 7, C-Atome aufweisen kann und/oder ein oder zwei ortho-anellierte Benzosysteme aufweisen kann und Phenyl oder Benzo jeweils ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und der aromatische Ring A ein- oder mehrfach durch Niederalkyl, Hydroxyniederalkyl, Halogenniederalkyl, Niederalkenyl, 3- oder 4-gliedriges Alkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder, bis auf $R_3$, unsubstituiert sein kann, und ihre Salze, insbesondere pharmazeutisch verwendbare Salze, und Isomeren, herstellt.

13. Verfahren nach einem der Ansprüche 2-4, dadurch gekennzeichnet, dass man Verbindungen der Formel (I), worin $R_o$ Wasserstoff, Niederalkanoyloxy oder im Phenylteil unsubstituiertes bzw. ein- oder mehrfach durch Niederalkyl, Niederalkoxy, Hydroxy, Halogen, Niederalkanoyloxy und/oder Trifluormethyl substituiertes Phenylniederalkanoyloxy bedeutet, $R_1$ Carboxy, mit einem Niederalkanol, einem durch Hydroxy, Niederalkoxy, Niederalkanoyloxy oder im Phenylteil unsubstituiertes oder Niederalkyl, Niederalkoxy, Hydroxy, Halogen, Niederalkanoyloxy und/oder Trifluormethyl enthaltendes Phenyl substituiertes Niederalkanol, mit einem unsubstituierten oder Niederalkyl, Niederalkoxy, Hydroxy, Halogen, Niederalkanoyloxy und/oder Trifluormethyl enthaltenden Phenol verestertes Carboxy, Carbamoyl, durch Niederalkyl, im Phenylteil unsubstituiertes bzw. Niederalkyl, Niederalkoxy, Hydroxy, Halogen, Niederalkanoyloxy und/oder Trifluormethyl aufweisendes Phenylniederalkyl monosubstituiertes Carbamoyl, durch Niederalkyl, durch im Phenylteil unsubstituiertes bzw. Niederalkyl,

Niederalkoxy, Hydroxy, Halogen, Niederalkanoyloxy und/oder Trifluormethyl aufweisendes Phenylniederalkyl, durch Niederalkylen oder durch Monoaza, N-alkyliertes Monoaza, Monooxa bzw. Monothia unterbrochenes Niederalkylen disubstituiertes Carbamoyl darstellt, $R_2$ Wasserstoff oder Niederalkyl bedeutet, $R_3$ eine durch Niederalkyl, durch im Cycloalkylteil 3- bis 7-gliedriges Cycloalkyl-niederalkyl, durch im Phenylteil unsubstituiertes bzw. Niederalkyl, Niederalkoxy, Hydroxy, Halogen, Niederalkanoyloxy und/oder Trifluormethyl enthaltendes Phenylniederalkyl, durch Niederalkylen, durch Niederalkenylen, durch Aza, N-Niederalkylaza, Oxa oder Thia unterbrochenes Niederalkylen oder durch Aza oder N-Niederalkylaza unterbrochenes Niederalkenylen disubstituierte Aminogruppe darstellt und der aromatische Ring A zusätzlich durch Niederalkyl, Niederalkoxy, Hydroxy, Halogen Niederalkanoyloxy, 3- oder 4-gliedriges Alkylen und/oder Trifluormethyl substituiert sein kann, und ihre Salze, insbesondere pharmazeutisch verwendbare Salze, und Isomeren, herstellt.

14. Verfahren nach einem der Ansprüche 1 und 5-10, dadurch gekennzeichnet, dass man Verbindungen der Formel

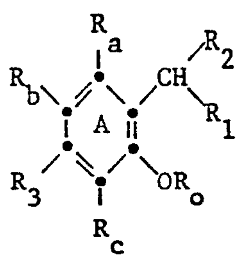

(Ia),

worin $R_o$ Wasserstoff oder Niederalkanoyl bedeutet, oder Phenylniederalkanoyl bedeutet, welches sich von einer Phenylniederalkancarbonsäure der Formel (I) ableitet, wobei die Reste $R_2$, $R_3$, $R_a$, $R_b$ und $R_c$ die nachstehend angegebenen Bedeutungen haben und $R_o$ Wasserstoff ist, $R_1$ Carboxy, Niederalkoxycarbonyl, Niederalkanoyloxyniederalkoxycarbonyl, Carbamoyl, N,N-Di-phenylniederalkylcarbamoyl, Niederalkylencarbamoyl, oder durch Monooxa unterbroches Niederalkylencarbamoyl darstellt, $R_2$ Wasserstoff oder Niederalkyl bedeutet, $R_3$ einerseits N,N-Di-phenylniederalkyl-amino darstellt oder $R_3$ andererseits jeweils 5- bis 8-gliedriges Niederalkylenamino, Niederalkenylenamino, durch Monooxa unterbrochenes Niederalkylenamino oder ein ortho-annelliertes Benzosystem aufweisendes Niederalkylenamino bzw. Niederalkenylenamino bedeutet, und/oder $R_a$, $R_b$ und $R_c$ unabhängig voneinander Wasserstoff, Niederalkyl oder Halogen darstellen, und ihre Salze, insbesondere pharmazeutisch verwendbare Salze, und Isomeren, herstellt.

15. Verfahren nach einem der Ansprüche 2-4, dadurch gekennzeichnet, dass man Verbindungen der Formel

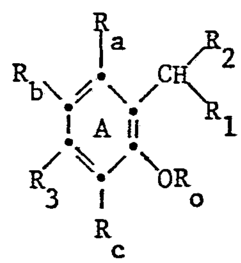

(Ia),

worin $R_o$ Wasserstoff oder Niederalkanoyl bedeutet, $R_1$ Carboxy, Niederalkoxycarbonyl, Niederalkylencarbamoyl oder Oxa-niederalkylen-carbamoyl darstellt, $R_2$ Wasserstoff oder Niederalkyl, bedeutet, $R_3$ Di-niederalkyl-amino, Di-cycloalkylniederalkylamino, Di-phenylniederalkyl-amino, jeweils 5- bis 8-gliedriges Niederalkylenamino, Niederalkenylenamino, Monoaza-niederalkylen-amino, N'-Niederalkylmonoaza-niederalkylenamino, Monooxaniederalkylen-amino, Monothianiederalkylenamino, Monoaza-niederalkenylen-amino oder N'-Niederalkylmonoaza-niederalkenylenamino darstellt und $R_a$, $R_b$, $R_c$ unabhängig voneinander Wasserstoff, Niederalkyl, Niederalkoxy, Hydroxy, Halogen, Niederalkanoyloxy, 3- bzw. 4-gliedriges Alkylen oder Trifluormethyl bedeuten und ihre Salze und Isomeren, herstellt.

16. Verfahren nach einem der Ansprüche 2-4, dadurch gekennzeichnet, dass man Verbindungen der Formel (Ia), worin $R_o$ Wasserstoff oder Niederalkanoyl bedeutet, $R_1$ Carboxy, Niederalkoxycarbonyl, 5- bis 8-gliedriges Niederalkylencarbamoyl, 5- bis 8-gliedriges Monooxaniederalkylencarbamoyl darstellt, $R_2$ Wasserstoff oder Niederalkyl darstellt, $R_3$ Di-niederalkyl-amino, 5- bis 8-gliedriges Niederalkylen-

amino, 5- bis 8-gliedriges Niederalkenylenamino oder 5- bis 8-gliedriges Monooxa-niederalkylenamino bedeutet und $R_a$ sowie $R_c$ Wasserstoff bedeuten und $R_b$ Halogen bedeutet und ihre Salze und Isomeren, herstellt.

17. Verfahren nach einem der Ansprüche 2-4, dadurch gekennzeichnet, dass man Verbindungen der Formel (Ia), worin $R_1$ Niederalkanoyl mit bis und mit 5 C-Atomen darstellt, $R_1$ Niederalkoxycarbonyl mit bis und mit 5 C-Atomen bedeutet, $R_2$ Niederalkyl mit bis und mit 4 C-Atomen darstellt, $R_3$ Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt und ihre Salze und Isomeren, herstellt.

18. Verfahren nach einem der Ansprüche 1 und 5-10, dadurch gekennzeichnet, dass man 2-(5-Chlor-2-hydroxy-4-morpholino-phenyl)-propionsäure oder ein Salz oder Isomeres davon herstellt.

19. Verfahren nach einem der Ansprüche 2-4, dadurch gekennzeichnet, dass man 2-(5-Chlor-2-hydroxy-4-morpholino-phenyl)-propionsäure oder ein Salz oder Isomeres davon herstellt.

20. Verfahren nach einem der Ansprüche 2-4, dadurch gekennzeichnet, dass man 2-[2-Hydroxy-5-methyl-4-(pyrrolidin-1-yl)-phenyl]-propionsäurepyrrolidid oder ein Salz oder Isomeres davon herstellt.

21. Verfahren nach einem der Ansprüche 2-4, dadurch gekennzeichnet, dass man 2-(2-Hydroxy-5-methyl-4-morpholino-phenyl)-propionsäuremorpholid oder ein Salz oder Isomeres davon herstellt.

22. Verfahren nach einem der Ansprüche 2-4, dadurch gekennzeichnet, dass man 2-(5-Chlor-2-hydroxy-4-morpholino-phenyl)-propionsäuremethylester oder ein Salz oder Isomeres davon herstellt.

23. Verfahren nach einem der Ansprüche 2-4, dadurch gekennzeichnet, dass man 2-(5-Chlor-2-hydroxy-4-morpholino-phenyl)-propionsäure-

morpholid oder ein Salz oder Isomeres davon herstellt.

24. Verfahren nach einem der Ansprüche 2-4, dadurch gekennzeichnet, dass man 2-(2-Acetoxy-5-chlor-4-morpholino-phenyl)-propionsäuremethylester oder ein Salz oder Isomeres davon herstellt.

25. Verfahren nach einem der Ansprüche 2-4, dadurch gekennzeichnet, dass man 5-Chlor-2-hydroxy-4-(piperidin-1-yl)-phenylessigsäure oder ein Salz davon herstellt.

26. Verfahren nach einem der Ansprüche 2-4, dadurch gekennzeichnet, dass man 2-(5-Chlor-2-hydroxy-4-(pyrrolidin-1-yl)-phenyl-propionsäure-Natriumsalz oder ein Isomeres davon herstellt.

27. Verfahren nach einem der Ansprüche 2-4, dadurch gekennzeichnet, dass man 2-(5-Chlor-2-hydroxy-4-morpholino-phenyl)-propionsäure-Natriumsalz oder ein Isomeres davon herstellt.

28. Verfahren nach einem der Ansprüche 2-4, dadurch gekennzeichnet, dass man 2-(4-Dibenzylamino-2-hydroxy-5-methyl-phenyl)-propionsäure-dibenzylamid oder ein Salz oder Isomeres davon herstellt.

29. Verfahren nach einem der Ansprüche 2-4, dadurch gekennzeichnet, dass man 2-(4-Dibenzylamino-2-hydroxy-5-methyl-phenyl)-propionsäure oder ein Salz oder Isomeres davon herstellt.

30. Verfahren nach einem der Ansprüche 1 und 5-10, dadurch gekennzeichnet, dass man 2-(5-Chlor-2-hydroxy-4-morpholino-phenyl)-propionsäure oder ein Salz oder Isomeres davon herstellt.

31. Verfahren nach einem der Ansprüche 1 und 5-10, dadurch gekennzeichnet, dass man 2-[2-Hydroxy-5-methyl-4-(pyrrolidin-1-yl)-phenyl-propionsäurepyrrolidid oder ein Salz oder Isomeres davon herstellt.

32. Verfahren nach einem der Ansprüche 1 und 5-10, dadurch gekennzeichnet, dass man 2-(2-Hydroxy-2-methyl-4-morpholino-phenyl)-propionsäuremorpholid oder ein Salz oder Isomeres davon herstellt.

33. Verfahren nach einem der Ansprüche 1 und 5-10, dadurch gekennzeichnet, dass man 2-(5-Chlor-2-hydroxy-4-morpholino-phenyl)-propionsäuremethylester oder ein Salz oder Isomeres davon herstellt.

34. Verfahren nach einem der Ansprüche 1 und 5-10, dadurch gekennzeichnet, dass man 2-(5-Chlor-2-hydroxy-4-morpholino-phenyl)-propionsäuremorpholid oder ein Salz oder Isomeres davon herstellt.

35. Verfahren nach einem der Ansprüche 1 und 5-10, dadurch gekennzeichnet, dass man 2-(2-Acetoxy-5-chlor-4-morpholino-phenyl)-propionsäuremethylester oder ein Salz oder Isomeres davon herstellt.

36. Verfahren nach einem der Ansprüche 1 und 5-10, dadurch gekennzeichnet, dass man 5-Chlor-2-hydroxy-4-(piperidin-1-yl)-phenylessigsäure oder ein Salz davon herstellt.

37. Verfahren nach einem der Ansprüche 1 und 5-10, dadurch gekennzeichnet, dass man 2-(5-Chlor-2-hydroxy-4-(pyrrolidin-1-yl)-phenyl-propionsäure-Natriumsalz oder ein Isomeres davon herstellt.

38. Verfahren nach einem der Ansprüche 1 und 5-10, dadurch gekennzeichnet, dass man 2-(5-Chlor-2-hydroxy-4-morpholino-phenyl)-propionsäure-Natriumsalz oder ein Isomeres davon herstellt.

39. Verfahren nach einem der Ansprüche 1 und 5-10, dadurch gekennzeichnet, dass man 2-(4-Dibenzylamino-2-hydroxy-5-methyl-phenyl)-propionsäuredibenzylamid oder ein Salz oder Isomeres davon herstellt.

40. Verfahren nach einem der Ansprüche 1 und 5-10, dadurch gekennzeichnet, dass man 2-(4-Dibenzylamino-2-hydroxy-5-methyl-phenyl)-propionsäure oder ein Salz oder Isomeres davon herstellt.

41. Verfahren nach einem der Ansprüche 1 und 5-10, dadurch gekennzeichnet, dass man 2-[2-Hydroxy-5-methyl-4-(pyrrol-1-yl)-phenyl]-propionsäure oder ein Salz oder Isomeres davon herstellt.

42. Verfahren nach einem der Ansprüche 1 und 5-10, dadurch gekennzeichnet, dass man 2-[2-Acetoxy-5-methyl-4-(pyrrol-1-yl)-phenyl]-propionsäuremethyl oder ein Salz oder Isomeres davon herstellt.

43. Verfahren nach einem der Ansprüche 1 und 5-10, dadurch gekennzeichnet, dass man 2-[2-Acetoxy-5-methyl-4-(pyrrolidin-1-yl)-phenyl]-propionsäuremethylester oder ein Salz oder Isomeres davon herstellt.

44. Verfahren nach einem der Ansprüche 1 und 5-10, dadurch gekennzeichnet, dass man 2-(2-Acetoxy-5-brom-4-morpholino-phenyl)-propionsäuremethylester oder ein Salz oder Isomeres davon herstellt.

45. Verfahren nach einem der Ansprüche 1 und 5-10, dadurch gekennzeichnet, dass man 2-[2-Hydroxy-5-methyl-2-(pyrrolidin-1-yl)-phenyl]-propionsäurepyrrolidid oder ein Salz oder Isomeres davon herstellt.

46. Verfahren nach einem der Ansprüche 1 und 5-10, dadurch gekennzeichnet, dass man 2-[2-Hydroxy-5-methyl-4-(pyrrol-1-yl)-phenyl]-propionsäuredibenzylamid oder ein Salz oder Isomeres davon herstellt.

47. Verfahren nach einem der Ansprüche 1 und 5-10, dadurch gekennzeichnet, dass man 2-[2-Hydroxy-5-methyl-4-(pyrrol-1-yl)-phenyl]-propionsäuredibenzylamid oder ein Salz oder Isomeres davon herstellt.

48. Das Verfahren der Beispiele 1-27 und die danach erhältlichen neuen Verbindungen.

49. Verfahren zur Herstellung von pharmazeutischen Präparaten, dadurch gekennzeichnet, dass man eine Verbindung gemäss einem der Ansprüche 1 und 11-47 mit üblichen Hilfs- und Trägerstoffen vermischt.